# EUROPEAN PATENT APPLICATION

(11) **EP 4 596 715 A2**
(43) Date of publication of application: **06.08.2025**
(21) Application number: 25170646.1
(22) Date of filing: 08.09.2022
(51) Int. Cl.: C12Q 1/6834

(54) **METHOD FOR DETECTING SENSE AND ANTISENSE STRANDS IN AN OLIGONUCLEOTIDE DUPLEX**

(30) Priority: 09.09.2021 US 202163242208 P
(62) Divisional of application: 22786684.5
(71) Applicant: Meso Scale Technologies, LLC., Rockville, MD 20850 (US)
(72) Inventor: HARKINS, Seth B., Bethesda, 20817 (US); BREAK, Timothy J., Clarksburg, 20871 (US)
(74) Representative: J A Kemp LLP

(57) **Abstract**

Described herein is a method for detecting an oligonucleotide in a sample, and in particular, to a method for detecting sense and antisense strands of an oligonucleotide duplex in a sample.

## Description

### FIELD OF THE INVENTION

The present disclosure relates to a method for detecting or quantifying an oligonucleotide in a sample, and in particular, to a method for detecting or quantifying a sense and an antisense strand of an oligonucleotide duplex.

### BACKGROUND OF THE INVENTION

Nucleic acid-based therapeutics make up a class of promising candidates for drug therapy, particularly for biological targets that conventional therapeutics such as small molecule, protein- or antibody-based therapeutics cannot access. Nucleic acid-based therapeutics include single stranded or double stranded oligonucleotide molecules that inhibit DNA or RNA expression, for example, to reduce or prevent production of an abnormal protein associated with a disease. Several nucleic acid-based therapeutics have been approved by the U.S. Food and Drug Administration and more are being investigated in clinical trials for the treatment of a variety of diseases.

Nucleic acids are large molecules that are highly charged, rapidly degraded and cleared from the body, which can result in poor pharmacological properties. Stoddard et al. (2018) "Editorial: Nucleic Acids Research and Nucleic Acid Therapeutics." Nuc. Acids Res. 46(4):1563-1564. Consequently, pharmacokinetics, tissue targeting, and tissue accumulation are all important considerations when developing nucleic-acid base therapeutics. Highly sensitive and quantitative assay are needed to characterize nucleic acid pharmacokinetics. Thayer et al. (2020) "POE Immunoassay: Plate-based oligonucleotide electrochemiluminescent immunoassay for the quantification of nucleic acids in biological matrices." Scientific Reports. 10(1):10425 (doi.org/10.1038/s41598-020-66829-6).

Although various polymerase-chain reaction (PCR) based, size-exclusion chromatography (SEC), and liquid chromatography-mass spectrometry (LC-MS) methods exist for characterizing nucleic acid therapeutics, these methods are limited by assay sensitivity and time-consuming extraction steps. *Id.* The unique biophysical properties of oligonucleotide therapeutics can lead to atypical absorption, distribution, metabolism and elimination (ADME) processes, as well as pharmacokinetics-pharmacodynamics (PKPD) uncoupling. The difficulty in understanding these relationships can lead to inefficiencies throughout the drug development process, including increased animal usage, and may lead to an increased risk in human trials. *Id.* Quantification of both strands of an oligonucleotide therapeutic can be important to understanding the stability and metabolic pathways of the oligonucleotide therapeutic and to developing models to elucidate the pharmacology of the therapeutic. *Id.*

As such, there remains a need for sensitive assays for the detection or quantification of oligonucleotide therapeutics, for example, from a sample obtained from a patient.

### SUMMARY OF THE INVENTION

Described herein is a method of detecting or quantifying a sense and an antisense strand of an oligonucleotide duplex in a sample. In one aspect, the method includes:
(a) contacting the sample with a composition that includes a set of probes, wherein the set of probes includes:
   (i) a sense probe that includes a first single stranded oligonucleotide tag that is complementary to at least a portion of a first capture oligonucleotide immobilized on a support surface, a sense binding portion capable of hybridizing to a nucleotide sequence of the sense strand of the oligonucleotide duplex, and a first label; and
   (ii) an antisense probe that includes a second single stranded oligonucleotide tag that is complementary to at least a portion of a second capture oligonucleotide immobilized on the support surface, an antisense binding portion capable of hybridizing to a nucleotide sequence of the antisense strand of the oligonucleotide duplex, and a second label,

   wherein the sense binding portion of the sense probe has a sense binding length that is shorter than a sense strand length of the sense strand, and
   wherein the antisense binding portion of the antisense strand has an antisense binding length that is shorter than an antisense strand length of the antisense strand; and
(b) incubating the probes with the sample to form a hybridization mixture that contains hybridization complexes comprising:
   (i) a sense complex comprising the sense probe hybridized with the sense strand of the oligonucleotide duplex; and
   (ii) an antisense complex comprising the antisense probe hybridized with the antisense strand of the oligonucleotide duplex;
(c) contacting the support surface with the hybridization mixture under conditions in which the first and second oligonucleotide tags of the hybridization complexes hybridize to the first and second capture oligonucleotides immobilized on the support surface and contacting the hybridization mixture with a single-strand specific nuclease; and
(d) detecting or quantifying the sense and antisense strands of the oligonucleotide duplex based on the presence label immobilized on the support surface.

In one aspect, (c) includes:
(i) contacting the support surface with the hybridization mixture under conditions in which the first and second oligonucleotide tags of the hybridization complexes hybridize to the first and second capture oligonucleotides on the support surface to immobilize the hybridization complexes on the support surface; and
(ii) contacting the immobilized hybridization complexes with a single-strand specific nuclease.

In one aspect, (c) includes:
(i) contacting the hybridization mixture with a single-strand specific nuclease to form a reaction mixture; and
(ii) contacting the support surface with the reaction mixture of (i) under conditions in which the first and second oligonucleotide tags of the sense and antisense probes hybridize to the first and second capture oligonucleotides immobilized on the support surface.

In one aspect, the oligonucleotide tag of the antisense probe hybridizes to a capture oligonucleotide immobilized on the support surface. In one aspect, the oligonucleotide tag of the antisense probe that is part of an antisense complex hybridizes to a capture oligonucleotide immobilized on the support surface. In one aspect, the oligonucleotide tag of the antisense probe is not part of an antisense complex. In one aspect, the oligonucleotide tag of the antisense probe is part of a hybridization complex. In one aspect, the hybridization complex does not include an antisense strand of the oligonucleotide duplex. In one aspect, the hybridization complex is a probe-probe complex. In one aspect, the probe-probe complex includes a single stranded overhang.

In one aspect, the oligonucleotide tag of the sense probe hybridizes to a capture oligonucleotide immobilized on the support surface. In one aspect, the oligonucleotide tag of the sense probe that is part of a sense complex hybridizes to a capture oligonucleotide immobilized on the support surface. In one aspect, the oligonucleotide tag of the sense probe is not part of a sense complex. In one aspect, the oligonucleotide tag of the sense probe is part of a hybridization complex. In one aspect, the hybridization complex does not include a sense strand of the oligonucleotide duplex. In one aspect, the hybridization complex is a probe-probe complex. In one aspect, the probe-probe complex includes a single stranded overhang.

In one aspect, the sense and antisense strands of the oligonucleotide duplex each include, individually, from about 8 to about 50 nucleotides. In one aspect, the sense and antisense strands of the oligonucleotide duplex each include, individually, from about 16 to about 30 nucleotides.

In one aspect, the sense strand of the oligonucleotide duplex includes DNA. In one aspect, the sense strand of the oligonucleotide duplex includes RNA. In one aspect, the antisense strand of the oligonucleotide duplex includes DNA. In one aspect, the antisense strand of the oligonucleotide duplex includes RNA.

In one aspect, the oligonucleotide duplex includes a DNA/DNA duplex. In one aspect, the oligonucleotide duplex includes a RNA/RNA duplex. In one aspect, the oligonucleotide duplex includes a DNA/RNA heteroduplex.

In one aspect, the sense strand, antisense strand or both the sense and antisense strands of the oligonucleotide duplex individually include one or more modified nucleic acids. In one aspect, the sense strand, the antisense strand or both the sense and antisense strands of the oligonucleotide duplex individually include a 5'- or 3'- conjugate. In one aspect, the conjugate includes polyethylene glycol (PEG), N-acetylgalactosamine (GalNAc), a cell penetrating peptide (CPP), α-tocopherol, an aptamer, an antibody, cholesterol, squalene, a fatty acid, or a nucleolipid.

In one aspect, the sense and antisense strand of the oligonucleotide duplex includes a nucleic acid sequence of a microorganism. In one aspect, the sense and antisense strand of the oligonucleotide duplex includes a nucleic acid sequence of a microorganism that is a component of the human microbiome. In one aspect, the microorganism is a bacteria, fungi, protozoa or a virus. In one aspect, the microorganism is a bacteria. In one aspect, the sense strand and the antisense strand of the oligonucleotide duplex comprise 16S rRNA or rDNA from bacteria.

In one aspect, the sense binding length of the sense probe is shorter than the sense strand length of the sense strand by at least 1 nucleotide. In one aspect, the sense binding length is about 10 to about 16 nucleotides in length. In one aspect, the sense binding portion of the sense probe has a 5' end that aligns with a 3' end of the sense strand of the oligonucleotide duplex.

In one aspect, the antisense binding length of the antisense probe is shorter than the antisense strand length of the antisense strand by at least 1 nucleotide. In one aspect, the antisense binding length is about 10 to about 16 nucleotides in length. In one aspect, the antisense binding portion of the antisense probe has a 5' end that aligns with a 3' end of the antisense strand of the oligonucleotide duplex.

In one aspect, the first oligonucleotide tag has a first oligonucleotide tag length and the first capture oligonucleotide has a first capture oligonucleotide length, and the first oligonucleotide tag length is the same as the first capture oligonucleotide length. In one aspect, the first oligonucleotide tag has a first oligonucleotide tag length and the first capture oligonucleotide has a first capture oligonucleotide length, and the first oligonucleotide tag length is the shorter than the first capture oligonucleotide length.

In one aspect, the second oligonucleotide tag has a second oligonucleotide tag length and the second capture oligonucleotide has a second capture oligonucleotide length, and the second oligonucleotide tag length is the same as the second capture oligonucleotide length. In one aspect, the second oligonucleotide tag has a second oligonucleotide tag length and the second capture oligonucleotide has a second capture oligonucleotide length, and the second oligonucleotide tag length is the shorter than the second capture oligonucleotide length.

In one aspect, the sense probe includes DNA. In one aspect, the sense binding portion of the sense probe includes DNA. In one aspect, the first oligonucleotide tag of the sense probe includes DNA. In one aspect, the sense binding portion and the oligonucleotide tag of the sense probe include DNA. In one aspect, the sense binding portion of the sense probe includes DNA and the oligonucleotide tag of the sense probe includes RNA.

In one aspect, the antisense probe includes DNA. In one aspect, the antisense binding portion of the antisense probe includes DNA. In one aspect, the first oligonucleotide tag of the antisense probe includes DNA. In one aspect, the antisense binding portion and the oligonucleotide tag of the antisense probe include DNA. In one aspect, the antisense binding portion of the antisense probe includes DNA and the oligonucleotide tag of the antisense probe includes RNA.

In one aspect, the sense probe includes RNA. In one aspect, the sense binding portion of the sense probe includes RNA. In one aspect, the first oligonucleotide tag of the sense probe includes RNA. In one aspect, the sense binding portion and the oligonucleotide tag of the sense probe include RNA. In one aspect, the sense binding portion of the sense probe includes RNA and the oligonucleotide tag of the sense probe includes DNA.

In one aspect, the antisense probe includes RNA. In one aspect, the antisense binding portion of the antisense probe includes RNA. In one aspect, the first oligonucleotide tag of the antisense probe includes RNA. In one aspect, the antisense binding portion and the oligonucleotide tag of the antisense probe include RNA. In one aspect, the antisense binding portion of the antisense probe includes RNA and the oligonucleotide tag of the antisense probe includes DNA.

In one aspect, the sense probe, the antisense probe or both include one or more modified nucleic acids. In one aspect, one or more modified nucleotides include a locked nucleic acid (LNA). In one aspect, one or more modified nucleotides are selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof.

In one aspect, the single-strand specific nuclease includes a single-strand specific DNase. In one aspect, the single-strand specific DNase is S1 nuclease, P1 nuclease or Mung Bean nuclease. In one aspect, the single-strand specific nuclease includes a single-strand specific RNase. In one aspect, the single-strand specific RNase is RNase A, RNase H, RNase I, RNase III, RNase L, RNase P, RNase PhyM, RNase T1, RNase T2, RNase U2, RNase V, PNPase, RNase PH, RNase R, RNase D, RNase T, RNaseONE, oligoribonuclease, exoribonuclease I, or exoribonuclease II.

In one aspect, (a)-(c) are performed concurrently. In one aspect, (a)-(c) are performed sequentially.

In one aspect, the hybridization conditions in (b) include:
(i) incubating the probes with the sample a first temperature to denature the sense and antisense strands of the oligonucleotide duplex; and
(ii) incubating the probes with the denatured sense and antisense strands of the oligonucleotide duplex at a second temperature to allow the sense and antisense probes to hybridize to the sense and antisense strands.

In one aspect, hybridization further includes incubating the sense and antisense complexes at a hold temperature of about 2°C to about 8°C.

In one aspect, the hybridization conditions in (b) include:
(i) incubating the probes with the sample a first temperature from about 60°C to about 95°C for about 1 minute to about 15 minutes;
(ii) incubating the probes with the sample at a second temperature from about 10°C to about 65°C for about 30 seconds to about 5 minutes; and
(iii) incubating the probes with the sample at a hold temperature from about 2°C to about 8°C.

In one aspect, the hybridization conditions in (b) include:
(i) incubating the probes with the sample a first temperature of about 95°C for about 2 minutes;
(ii) incubating the probes with the sample at a second temperature of about 65°C for about 1 min; and
(iii) incubating the probes with the sample at a hold temperature of about 4°C.

In one aspect, the hybridization conditions include a first temperature transition rate between steps (i) and (ii) of about 1 °C/s to about 2 °C/s. In one aspect, the hybridization conditions include first temperature transition rate between steps (i) and (ii) of about 1.8°C/s. In one aspect, the hybridization conditions include a second temperature transition rate between steps (ii) and (iii) about 0.05°C/s to about 1 C/s. In one aspect, the hybridization conditions include a second temperature transition rate between steps (ii) and (iii) of about 0.1°C/s.

In one aspect, the probes are incubated with the sample in a buffer that includes diluent 54 or N- PLEX hybridization Buffer 1 or 2.

In one aspect, the sample includes a plurality of oligonucleotide duplexes and the composition in (a) includes a plurality of sets of probes, wherein each set of probes hybridizes with a unique sense or antisense strand of a unique oligonucleotide duplex.

In one aspect, (c) includes incubating the support surface with the sense and antisense complexes for about 15 minutes to about 12 hours at a temperature of about 20 °C to about 40 °C. In one aspect, (c) includes incubating the support surface with the sense and antisense complexes for about 1 hour to about 2 hours at a temperature of about 20 °C to about 40 °C. In one aspect, the support surface is incubated with the sense and antisense complexes while shaking. In one aspect, (c) includes incubating the support surface with the sense and antisense complexes for about 1 hour at a temperature of about 37°C, while shaking at about 705 rpm.

In one aspect, the composition in (a) includes about 20 pM to about 10 nM sense probe. In one aspect, the composition in (a) includes about 20 pM to about 10 nM antisense probe.

In one aspect, the sample includes a biological sample. In one aspect, the sample includes an untreated biological sample. In one aspect, the sample includes a pretreated biological sample. In one aspect, the sample includes a purified sample. In one aspect, the sample is purified by precipitation, centrifugation, or column chromatography. In one aspect, the sample includes an extracted sample. In one aspect, the sample includes a naturally occurring RNase. In one aspect, the method includes combining the sample with an RNase inhibitor before (a). In one aspect, the sample includes cell-free DNA.

In one aspect, the biological sample includes a fluid obtained from an organism. In one aspect, the biological sample includes whole blood, plasma, serum, urine, feces, breast milk, saliva, or amniotic fluid. In one aspect, the sample includes an environmental sample. In one aspect, the sample includes a manufacturing process sample.

In one aspect, the method has a limit of detection of less than about 200 pg/mL.

In one aspect, the support surface includes one or more electrodes. In one aspect, one or more electrodes include a carbon electrode. In one aspect, one or more electrodes include carbon ink electrodes. In one aspect, one or more electrodes are included in a multi-well plate. In one aspect, each well of the multi-well plate includes an electrode.

In one aspect, the label includes a member of a binding pair. In one aspect, the label includes biotin.

In one aspect, the label includes an electrochemiluminescent (ECL) label. In one aspect, the method includes a step of generating an assay signal by contacting the electrodes with an electrochemiluminescence read buffer that includes an electrochemiluminescence co-reactant, and applying an electrical potential to the electrodes. In one aspect, the co-reactant is selected from a tertiary amine, tripropylamine, N-butyldiethanolamine, and combinations thereof.

In one aspect, a composition is provided that includes a set of probes. In one aspect, the set of probes includes:
(a) a sense probe that includes a first single stranded oligonucleotide tag that is complementary to at least a portion of a first capture oligonucleotide, a sense binding portion capable of hybridizing to a nucleotide sequence of a sense strand of an oligonucleotide duplex, and a first label; and
(b) an antisense probe that includes a second single stranded oligonucleotide tag that is complementary to at least a portion of a second capture oligonucleotide, an antisense binding portion capable of hybridizing to a nucleotide sequence of an antisense strand of the oligonucleotide duplex, and a second label,
wherein the sense binding portion of the sense probe has a sense binding length that is shorter than a sense strand length of the sense strand, and wherein the antisense binding portion of the antisense strand has an antisense binding length that is shorter than an antisense strand length of the antisense strand.

In one aspect, a composition is provided that includes:
(a) an oligonucleotide duplex that includes a sense strand and an antisense strand; and
(b) a set of probes that includes:
   (i) a sense probe that includes a first single stranded oligonucleotide tag that is complementary to at least a portion of a first capture oligonucleotide, a sense binding portion capable of hybridizing to a nucleotide sequence of a sense strand of an oligonucleotide duplex, and a first label; and
   (ii) an antisense probe that includes a second single stranded oligonucleotide tag that is complementary to at least a portion of a second capture oligonucleotide, an antisense binding portion capable of hybridizing to a nucleotide sequence of an antisense strand of the oligonucleotide duplex, and a second label,
wherein the sense binding portion of the sense probe has a sense binding length that is shorter than a sense strand length of the sense strand, and wherein the antisense binding portion of the antisense strand has an antisense binding length that is shorter than an antisense strand length of the antisense strand.

In one aspect, a composition is provided that includes one or more hybridization complexes. In one aspect, the hybridization complexes include:
(a) a sense complex that includes a sense probe hybridized to a sense strand of an oligonucleotide duplex, wherein the sense probe includes a first single stranded oligonucleotide tag that is complementary to at least a portion of a first capture oligonucleotide, a sense binding portion capable of hybridizing to a nucleotide sequence of the sense strand of the oligonucleotide duplex, and a first label, wherein the sense binding portion of the sense probe has a sense binding length that is shorter than a sense strand length of the sense strand;
(b) an antisense complex that includes an antisense probe hybridized to an antisense strand of the oligonucleotide duplex, wherein the antisense probe includes a second single stranded oligonucleotide tag that is complementary to at least a portion of a second capture oligonucleotide, an antisense binding portion capable of hybridizing to a nucleotide sequence of the antisense strand of the oligonucleotide duplex, and a second label, wherein the antisense binding portion of the antisense strand has an antisense binding length that is shorter than an antisense strand length of the antisense strand,
and combinations thereof.

In one aspect, the sense and antisense strands of the oligonucleotide duplex in the composition each include, individually, from about 8 to about 50 nucleotides. In one aspect, the sense and antisense strands of the oligonucleotide duplex in the composition each include, individually, from about 16 to about 30 nucleotides.

In one aspect, the sense strand of the oligonucleotide duplex in the composition includes DNA. In one aspect, the sense strand of the oligonucleotide duplex includes RNA. In one aspect, the antisense strand of the oligonucleotide duplex in the composition includes DNA. In one aspect, the antisense strand of the oligonucleotide duplex includes RNA. In one aspect, the oligonucleotide duplex includes a DNA/DNA duplex. In one aspect, the oligonucleotide duplex includes a RNA/RNA duplex. In one aspect, the oligonucleotide duplex includes a DNA/RNA heteroduplex.

In one aspect, the sense strand, antisense strand or both the sense and antisense strands of the oligonucleotide duplex in the composition individually include one or more modified nucleic acids. In one aspect, the sense strand, the antisense strand or both the sense and antisense strands of the oligonucleotide duplex in the composition individually include a 5'- or 3'- conjugate. In one aspect, the conjugate includes polyethylene glycol (PEG), N-acetylgalactosamine (GalNAc), a cell penetrating peptide (CPP), *α*-tocopherol, an aptamer, an antibody, cholesterol, squalene, a fatty acid, or a nucleolipid.

In one aspect, the sense binding length of the sense probe in the composition is shorter than the sense strand length of the sense strand by at least 1 nucleotide. In one aspect, the sense binding length of the sense probe in the composition is about 10 to about 16 nucleotides in length. In one aspect, the sense binding portion of the sense probe in the composition has a 5' end that aligns with a 3' end of the sense strand of the oligonucleotide duplex.

In one aspect, the antisense binding length of the antisense probe in the composition is shorter than the antisense strand length of the antisense strand by at least 1 nucleotide. In one aspect, the antisense binding length of the antisense probe in the composition is about 10 to about 16 nucleotides in length. In one aspect, the antisense binding portion of the antisense probe in the composition has a 5' end that aligns with a 3' end of the antisense strand of the oligonucleotide duplex.

In one aspect, the sense probe in the composition includes DNA. In one aspect, the sense binding portion of the sense probe includes DNA. In one aspect, the first oligonucleotide tag of the sense probe includes DNA. In one aspect, the sense binding portion and the oligonucleotide tag of the sense probe include DNA. In one aspect, the sense binding portion of the sense probe includes DNA and the oligonucleotide tag of the sense probe includes RNA.

In one aspect, the antisense probe in the composition includes DNA. In one aspect, the antisense binding portion of the antisense probe includes DNA. In one aspect, the first oligonucleotide tag of the antisense probe includes DNA. In one aspect, the antisense binding portion and the oligonucleotide tag of the antisense probe include DNA. In one aspect, the antisense binding portion of the antisense probe includes DNA and the oligonucleotide tag of the antisense probe includes RNA.

In one aspect, the sense probe in the composition includes RNA. In one aspect, the sense binding portion of the sense probe includes RNA. In one aspect, the first oligonucleotide tag of the sense probe includes RNA. In one aspect, the sense binding portion and the oligonucleotide tag of the sense probe include RNA. In one aspect, the sense binding portion of the sense probe includes RNA and the oligonucleotide tag of the sense probe includes DNA.

In one aspect, the antisense probe in the composition includes RNA. In one aspect, the antisense binding portion of the antisense probe includes RNA. In one aspect, the first oligonucleotide tag of the antisense probe includes RNA. In one aspect, the antisense binding portion and the oligonucleotide tag of the antisense probe include RNA. In one aspect, the antisense binding portion of the antisense probe includes RNA and the oligonucleotide tag of the antisense probe includes DNA.

In one aspect, the sense probe, the antisense probe or both probes in the composition include one or more modified nucleic acids. In one aspect, one or more modified nucleotides include a locked nucleic acid (LNA). In one aspect, one or more modified nucleotides are selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof.

In one aspect, the label in the composition includes a member of a binding pair. In one aspect, the label includes biotin. In one aspect, the label in the composition includes an electrochemiluminescent label.

In one aspect, a kit is provided for carrying out the method described herein.

### BRIEF DESCRIPTION OF THE FIGURES

FIG. 1A is a schematic representation of an antisense binding complex in which an antisense binding probe described herein is hybridized to an antisense oligonucleotide sequence.
FIG. 1B is a schematic representation of a sense binding complex in which a sense binding probe described herein is hybridized to a sense oligonucleotide sequence.
FIG. 2A is a schematic representation of an antisense binding complex formed between an antisense binding strand of an oligonucleotide duplex and a "short" antisense binding probe.
FIG. 2B is a schematic representation of a sense binding complex formed between a sense binding strand of an oligonucleotide duplex and a "short" sense binding probe.
FIG. 2C is a schematic representation of showing a "non-productive" binding complex formed between a "short" sense binding probe and a "short" antisense binding probe.
FIG. 3A is a schematic representation of an antisense binding complex formed between an antisense binding strand of an oligonucleotide duplex and a "full-length" antisense binding probe.
FIG. 3B is a schematic representation of a sense binding complex formed between a sense binding strand of an oligonucleotide duplex and a "full-length" sense binding probe.
FIG.3C is a schematic representation of showing a "non-productive" binding complex formed between a "full-length" sense binding probe and a "full-length" antisense binding probe.
FIG.4A is a schematic of an oligonucleotide tag of a probe hybridized to a capture oligonucleotide immobilized on a support surface, wherein the probe is part of an antisense or sense complex with a single-stranded overhang.
FIG.4B is a schematic of an oligonucleotide tag of a probe hybridized to a capture oligonucleotide immobilized on a support surface, wherein the probe is part of an antisense or sense complex that does not have a single-stranded overhang.
FIG. 4C is a schematic of an oligonucleotide tag of a probe hybridized to a capture oligonucleotide immobilized on a support surface, wherein the probe is not part of an antisense or sense complex.
FIG. 4D is a schematic of an oligonucleotide tag of a probe hybridized to a capture oligonucleotide immobilized on as support surface, wherein the probe is part of a probe-probe complex that includes a single-stranded overhang.
FIG. 4E is a schematic of an oligonucleotide tag of a probe hybridized to a capture oligonucleotide immobilized on as support surface, wherein the probe is part of a probe-probe complex that does not include a single-stranded overhang.
FIG. 5A is a graph showing the ECL signal curve for 1x and 4x concentrations of an antisense (AS) probe hybridized in Diluent 54.
FIG. 5B is a graph showing the ECL signal curve for 1x and 4x concentrations of an antisense (AS) probe hybridized in Hybridization Buffers.
FIG. 6A is a graph showing the ECL signal curve for 1x and 4x concentrations of a sense (SS) probe hybridized in Diluent 54.
FIG. 6B is a graph showing the ECL signal curve for 1x and 4x concentrations of a sense (SS) probe hybridized in Hybridization Buffers.
FIG. 7A is a graph showing the ECL signal curve for a 16-mer (FL) and 12-mer antisense (AS) probe hybridized in Diluent 54.
FIG. 7B is a graph showing the ECL signal curve a 16-mer (FL) and 12-mer antisense (AS) probe hybridized in Hybridization Buffers.
FIG. 8A is a graph showing the ECL signal curve for a 16-mer (FL) probe hybridized to the antisense (AS) strand alone or in a heteroduplex.
FIG. 8B is a graph showing the ECL signal curve a 12-mer probe hybridized to the antisense (AS) strand alone or in a heteroduplex.
FIG. 9A is a graph showing the ECL signal curve for a 16-mer (FL) probe hybridized to the sense (SS) strand alone or in a heteroduplex.
FIG. 9B is a graph showing the ECL signal curve for a 12-mer probe hybridized to the sense (SS) strand alone or in a heteroduplex.
FIG. 10A is a graph showing the ECL signal curve for a 12-mer probe hybridized to the antisense (AS) strand alone or in a heteroduplex.
FIG. 10B is a graph showing the ECL signal curve for a 12-mer probe hybridized to the sense (SS) strand alone or in a heteroduplex.
FIG. 11A is a graph showing the ECL signal curve for a 16-mer (FL) probe hybridized to the antisense (AS) strand under long or short hybridization conditions.
FIG. 11B is a graph showing the ECL signal curve for a 12-mer probe hybridized to the antisense (AS) strand under long or short hybridization conditions.
FIG. 12A is a graph showing the ECL signal curve for an individual sense (SS) strand in Diluent 54 and in plasma.
FIG. 12B is a graph showing the ECL signal curve for the sense (SS) strand in a heteroduplex in Diluent 54 and in plasma.
FIG. 13A is a graph showing the ECL signal curve for an individual sense (SS) strand in Diluent 54 or brain lysate.
FIG. 13B is a graph showing the ECL signal curve for the sense (SS) strand of a heteroduplex in Diluent 54 or brain lysate.
FIG. 14A is a graph showing the ECL signal curve for an individual antisense (AS) strand with or without LNA.
FIG. 14B is a graph showing the ECL signal curve for the antisense (AS) strand of a heteroduplex with or without LNA.
FIG. 15A is a graph showing the ECL signal curve for an antisense (AS) LNA probe at varying concentrations.
FIG. 15B is a graph showing the ECL signal curve for a sense (SS) LNA probe at varying concentrations.
FIG. 16A is a graph showing the ECL signal curve for multiplex detection of an individual antisense (AS) strand with unmodified or LNA modified 12-mer probe.
FIG. 16B is a graph showing the ECL signal curve for multiplex detection of an antisense (AS) strand of a heteroduplex with unmodified or LNA modified 12-mer probe.
FIG. 17A is a graph showing the ECL signal curve for multiplex detection of an individual sense (SS) strand with unmodified or LNA modified 12-mer probe.
FIG. 17B is a graph showing the ECL signal curve for multiplex detection of a sense (SS) strand of a heteroduplex with unmodified or LNA modified 12-mer probe.
FIG. 18A is a graph showing the ECL signal curve for an individual antisense (AS) strand detected using a FL, 14-mer, 13-mer and 12-mer probe.
FIG. 18B is a graph showing the ECL signal curve for an individual sense (SS) strand detected using a FL, 14-mer, 13-mer and 12-mer probe.
FIG. 19A is a graph showing the ECL signal curve for an antisense (AS) strand of a heteroduplex detected using a FL, 14-mer, 13-mer and 12-mer probe.
FIG. 19B is a graph showing the ECL signal curve for a sense (SS) strand of a heteroduplex detected using a FL, 14-mer, 13-mer and 12-mer probe.
FIG. 20A is a graph showing the ECL signal curve for an antisense (AS) strand in a heteroduplex when detected using a combination of antisense (AS) and sense (SS) probe lengths.
FIG. 20B is a graph showing the ECL signal curve for a sense (SS) strand in a heteroduplex when detected using a combination of antisense (AS) and sense (SS) probe lengths.

### DETAILED DESCRIPTION OF THE INVENTION

### A. Definitions

Unless otherwise defined, scientific and technical terms used herein shall have the meanings that are commonly understood by those of ordinary skill in the art. Further, unless otherwise required by context, singular terms shall include pluralities and plural terms shall include the singular, for example, "a" or "an", include pluralities, e.g., "one or more" or "at least one" and the term "or" can mean "and/or", unless stated otherwise. The terms "including," "includes" and "included", are not limiting. Ranges provided herein, of any type, include all values within a particular range described and values about an endpoint for a particular range. As used herein, ranges expressed using the word "between" are inclusive of the range endpoints. Thus, for example, a range of between 50°C and 70°C includes 50°C to 70°C, i.e., it includes the endpoints of 50°C and 70°C.

As used herein, the term "about" is used to modify, for example, the quantity of an ingredient in a composition, concentration, volume, process temperature, process time, yield, flow rate, pressure, and ranges thereof, employed in describing the invention. The term "about" refers to variation in the numerical quantity that can occur, for example, through typical measuring and handling procedures used for making compounds, compositions, concentrates or formulations; through inadvertent error in these procedures; through differences in the manufacture, source, or purity of starting materials or ingredients used to carry out the methods, and other similar considerations. The term "about" also encompasses amounts that differ due to aging of a formulation with a particular initial concentration or mixture, and amounts that differ due to mixing or processing a formulation with a particular initial concentration or mixture. Where modified by the term "about," the claims appended hereto include such equivalents.

Generally, nomenclatures used in connection with, and techniques of, cell and tissue culture, molecular biology, and protein and oligo- or polynucleotide chemistry and hybridization described herein are those well-known and commonly used in the art. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

The term "nucleotide" refers to a monomeric unit that includes a nucleobase, a sugar, and one or more internucleotidic linkages. As used herein, the term nucleotide includes naturally occurring nucleotides and modified nucleotides. Naturally occurring nucleotides include guanine, (G), adenine, (A), cytosine, (C), thymine, (T), and uracil (U) as well as naturally occurring base analogs. In deoxyribonucleic acid (DNA), the sugar is deoxyribose. In ribonucleic acid (RNA), the sugar is ribose. The term "modified nucleotide" refers to a nucleotide that includes a modification at a nucleobase, a sugar or an internucleotidic linkage, wherein the modified nucleotide remains capable of base-pairing to a complementary naturally occurring or modified nucleotide. The term "polynucleotide" refers to polymer of two or more nucleotides covalently linked to each other by an internucleosidic linkage.

As used herein the term "oligonucleotide" refers to a short polymer that includes two or more nucleotides, generally from about 5 to about 100 nucleotides covalently linked by internucleosidic linkages. In one aspect, the oligonucleotide is a polymer that is from about 5, 6, 7, 8, 9, 10, 11, 12, 13, 14, 15, 20, 25 or 30 nucleotides and up to about 30, 35, 40, 45, 50 or 100 nucleotides in length, or from about 8 to about 50 nucleotides in length, about 10 to about 40 nucleotides in length, about 12 to about 30 nucleotides in length, or about 18 to about 30 nucleotides in length. As used herein, the term oligonucleotide can refer to a single-stranded oligonucleotide or a double stranded oligonucleotide, or the individual oligonucleotide strands of a double-stranded oligonucleotide. In one aspect, the term "oligonucleotide" refers to a double-stranded oligonucleotide therapeutic.

An "oligonucleotide therapeutic" is an oligonucleotide that includes at least one strand that is at least partially complementary to and can hybridize to a target nucleic acid. In one aspect, the oligonucleotide therapeutic includes a sense strand and an antisense strand. In one aspect, the oligonucleotide therapeutic can hybridize to the target nucleic acid and modulate expression or an amount of the target nucleic acid. The term "modulates" can include increasing or decreasing expression or an amount of the target nucleic acid. The term "expression" refers to the process by which information in a gene is used to produce a protein and includes, but is not limited to, transcription, splicing, post-transcriptional modification, and translation. In one aspect, the oligonucleotide therapeutic increases expression or an amount of a target nucleic acid. In one aspect, the oligonucleotide therapeutic decreases expression or an amount of a target nucleic acid. In one aspect, the oligonucleotide is chemically synthesized and purified or isolated. In one aspect, the oligonucleotide is made by solid phase chemical synthesis. Methods for making oligonucleotides, including, for example, sense and antisense oligonucleotides, probes, tags or capture oligonucleotides as described herein, are known.

As used herein, "base-pairing" refers to specific hydrogen bonding between purines and pyrimidines that leads to the formation of a double-stranded oligonucleotide. In DNA, adenine (A) pairs with thymine (T), and guanine (G) pairs with cytosine (C). In RNA, adenine (A) pairs with uracil (U), and guanine (G) pairs with cytosine (C). While not limited to any particular mechanism, the most common mechanism of base-pairing involves hydrogen bonding, which may be Watson-Crick, Hoogsteen or reversed Hoogsteen hydrogen bonding, between complementary nucleobases.

The term "chimeric" refers to a compound having at least two chemically distinct regions. In embodiments, each region has a plurality of subunits. As used herein, the term "chimeric probe" includes linked single-stranded DNA and/or RNA derived from two or more biological sources.

"Complementary" refers to nucleic acid molecules or oligonucleotides that interact by the formation of hydrogen bonds, for example, according to the Watson-Crick, Hoogsteen or reversed Hoogsteen hydrogen bonding base-pairing models. Hybridization can occur between two complementary DNA molecules (DNA-DNA hybridization), two RNA molecules (RNA-RNA hybridization), or between complementary DNA and RNA molecules (DNA-RNA hybridization). When used in connection with nucleotides, the term "complementary" refers to a pair of nucleotides, for example, that includes a purine and a pyrimidine, which are capable of base-pairing with each other. The complementary pair of nucleotides can include a pair of naturally occurring nucleotides, a pair of modified nucleotides or a pair that includes a naturally occurring nucleotide and a modified nucleotide. When used in connection with an oligonucleotide, the term "complementary" means that nucleotides of one oligonucleotide or a portion thereof are capable of hydrogen bonding nucleotides of another oligonucleotide or portion thereof other when the complementary nucleotides are aligned. Hybridization can occur between a short nucleotide sequence that is complementary to a portion of a longer nucleotide sequence. Hybridization can occur between sequences that do not have 100% "sequence complementarity" (i.e., sequences where less than 100% of the nucleotides align based on a base-pairing model such as the Watson-Crick, Hoogsteen or reversed Hoogsteen hydrogen bonding base-pairing models), although sequences having less sequence complementarity are less stable and less likely hybridize than sequences having greater sequence complementarity. In one aspect, the nucleotides of the complementary sequences have 100% sequence complementarity (i.e., each nucleotide of one oligonucleotides sequence or region can hydrogen bond with each nucleotide of a second oligonucleotide strand or region) based on the Watson-Crick model. In another aspect, the nucleotides of the complementary sequences have at least about 90%, about 95%, about 96%, about 97%, about 98% or about 99% sequence complementarity based on the Watson-Crick model. In one aspect, "substantial complementarity" refers to sequences that are partially complementary and are able to hybridize under physiologically relevant conditions. In one aspect, "substantial complementarity" refers to sequences that are partially complementary and are able to hybridize under stringent hybridization conditions. In one aspect, complementarity refers to the complementarity between two oligonucleotides of a double-stranded oligonucleotide therapeutic. In one aspect, complementarity refers to the complementarity between a single stranded oligonucleotide therapeutic and a single-stranded oligonucleotide probe. In one aspect, complementarity refers to the complementarity between a single stranded oligonucleotide tag and a single stranded capture oligonucleotide. In one aspect, complementarity refers to the complementarity between a single stranded oligonucleotide and a chimeric probe.

Whether or not two complementary sequences hybridize can depend on the stringency of the hybridization conditions, which can vary depending on conditions such as temperature, solvent, ionic strength and other parameters. The stringency of the hybridization conditions can be selected to provide selective formation or maintenance of a desired hybridization product of two complementary nucleic acid sequences, in the presence of other potentially cross-reacting or interfering sequences. Stringent conditions are sequence-dependent - typically longer complementary sequences specifically hybridize at higher temperatures than shorter complementary sequences. Generally, stringent hybridization conditions are between about 5°C to about 10°C lower than the thermal melting point (Tₘ) (i.e., the temperature at which 50% of the sequences hybridize to a substantially complementary sequence) for a specific nucleotide sequence at a defined ionic strength, concentration of chemical denaturants, pH and concentration of the hybridization partners. Generally, nucleotide sequences having a higher percentage of G and C bases hybridize under more stringent conditions than nucleotide sequences having a lower percentage of G and C bases. Generally, stringency can be increased by increasing temperature, increasing pH, decreasing ionic strength, or increasing the concentration of chemical nucleic acid denaturants (such as formamide, dimethylformamide, dimethylsulfoxide, ethylene glycol, propylene glycol and ethylene carbonate). Stringent hybridization conditions typically include salt concentrations of less than about 1 M, about 500 mM, or about 200 mM; hybridization temperatures above about 20°C, about 30°C, about 40°C, about 60°C or about 80°C; and chemical denaturant concentrations above about 10%, about 20%, about 30%, about 40% or about 50%. Because many factors can affect the stringency of hybridization, the combination of parameters may be more significant than the absolute value of any parameter alone.

In one aspect, complementarity refers to the complementarity between an oligonucleotide and a target nucleic acid sequence. In one aspect, the target nucleic acid includes DNA or RNA. In one aspect, the target RNA includes mRNA, pre-mRNA, non-coding RNA, pri-microRNA, pre-microRNA, mature microRNA, or promoter-directed RNA. In one aspect, the target nucleic acid is a cellular gene or mRNA transcribed from the gene whose expression is associated with a particular disorder or disease. In one aspect, the target nucleic acid is a nucleic acid molecule from an infectious agent. In one aspect, the target nucleic acid is a viral or bacterial nucleic acid.

As used herein, "hybridize," "hybridizing" or "hybridization" refers to base-pairing between two complementary oligonucleotides. In one aspect, a single-stranded oligonucleotide strand of a double-stranded oligonucleotide therapeutic can hybridize to a target nucleic acid. In one aspect, two single-stranded oligonucleotide strands of a double-stranded oligonucleotide hybridize to each other. In one aspect, an oligonucleotide probe hybridizes to a single-stranded oligonucleotide strand of a double-stranded oligonucleotide. In one aspect, a single-stranded oligonucleotide tag hybridizes to a single-stranded capture oligonucleotide. "Specifically hybridize" refers to hybridization between two complementary oligonucleotides that occurs with greater affinity and without significant cross-hybridization with other oligonucleotides in a sample. In one aspect, the complementary oligonucleotides specifically hybridize under physiologically relevant conditions, such as the conditions found in the cytoplasm of a cell. In another aspect, the complementary oligonucleotides specifically hybridize under stringent hybridization conditions.

As used herein, "oligonucleotide duplex" refers to a double-stranded oligonucleotide that is formed by hybridization of two single-stranded oligonucleotides that are at least partially complementary to one another and hybridize to one another via base-pairing between complementary nucleobases. In one aspect, at least one strand of the oligonucleotide duplex includes DNA. In one aspect, at least one strand of the oligonucleotide duplex includes RNA. In one aspect, both strands of the oligonucleotide duplex include DNA. In one aspect, both strands of the oligonucleotide duplex include RNA. In one aspect, the double-stranded oligonucleotide is a heteroduplex that includes one strand that is DNA and one strand that is RNA. In one aspect, the sugar-phosphate backbones of the two oligonucleotide strands of the oligonucleotide duplex are oriented in opposite directions (i.e., one strand runs 5' to 3' and the other 3' to 5'), which is referred to as "antiparallel". In one aspect, the two oligonucleotide strands of the oligonucleotide duplex are the same length as one another such that the oligonucleotide duplex is double-stranded over its entire length, i.e., the oligonucleotide duplex has blunt ends. In one aspect, the two oligonucleotide strands of the oligonucleotide duplex are the same length as one another but align in such a manner that the oligonucleotide duplex is not double-stranded over its entire length, i.e., the oligonucleotide duplex has a single stranded 3' overhang or a single stranded 5' overhang at both ends of the duplex. In one aspect, the two oligonucleotide strands of the oligonucleotide duplex are a different length from each other such that the oligonucleotide duplex is not double-stranded over its entire length, i.e., the oligonucleotide duplex has a single stranded 3' overhang or a single stranded 5' overhang at one or both ends of the oligonucleotide duplex. In one aspect, the single stranded overhang is from about 1 to about 5, about 1 to about 4, about 1 to about 3, or about 1 to about 2 nucleotides. In one aspect, the oligonucleotide duplex has one blunt end and one end that includes a single stranded overhang. When used in connection with an oligonucleotide, the term "length" refers to the number of nucleotide residues in the polymeric backbone of a single-stranded oligonucleotide.

As used herein, the term "overhang" refers to a double-stranded oligonucleotide in which at least one end of one strand is longer than the corresponding end of the other strand. In one aspect, the single-stranded overhang is located at the 3'-terminus of one or both strands of the double stranded oligonucleotide. In one aspect, the single-stranded overhang is located at the 5'-terminus of one or both strands of the double stranded oligonucleotide. In one aspect, the single-stranded overhang includes from about 1 to about 5 nucleotides, from about 1 to about 4 nucleotides, from about 1 to about 3 nucleotides, or from about 1 to about 2 nucleotides. In one aspect, one end of the double-stranded oligonucleotide is blunt and the other end includes a 3' or a 5' overhang. In one aspect, both ends of the double-stranded oligonucleotide include a single stranded overhang.

The term "antisense" refers to an oligonucleotide with a nucleic acid sequence that is inverted relative to the orientation necessary for transcription of a target nucleic acid, such that the antisense oligonucleotide and can hybridize to the target nucleic acid, for example, through Watson-Crick base-pairing. The "sense strand" of the oligonucleotide duplex is complementary to the antisense strand and is therefore "sense" to at least part of the target nucleic acid. In one aspect, the antisense and sense strands of the oligonucleotide duplex are at least about 90%, about 95%, about 96%, about 97%, about 98%, about 99% or about 100% complementary to one another.

A single stranded oligonucleotide has "direction" or "directionality" because adjacent nucleotides are joined by an internucleosidic linkage, such as a phosphodiester bond between their 5' and 3' carbons atoms, such that the terminal 5' and 3' carbons are exposed at either end of the oligonucleotide, which can be referred to as the 5'- (phosphoryl) and 3'- (hydroxyl) ends of the molecule.

The term "identical" means that an oligonucleotide sequences include identical nucleic acid bases at the same positions over a comparison window. The term "% sequence identity" can be determined by comparing two aligned sequences over a window of comparison, determining the number of positions at which the identical nucleic acid base occurs in both sequences to yield the number of matched positions, dividing the number of matched positions by the total number of positions in the comparison window, and multiplying the result by 100 to yield the percentage of sequence identity. The comparison window can include a full-length sequence or may be a subpart of a larger sequence. Various methods and algorithms are known for determining the percent identity between two or sequences, including, but not limited MEGALIGN (DNASTAR, Inc. Madison, Wis.), FASTA, BLAST, or ENTREZ.

In one aspect, a nucleotide of an oligonucleotide described herein includes a structural analog with a non-naturally occurring chemical structures that can also participate in hybridization reactions. In one example, a nucleotide or nucleic acid may include a chemical modification that links it to a label or provides a reactive functional group that can be linked to a label, for example, through the use of amine or thiol-modified nucleotide bases, phosphates or sugars. The term "reactive functional group" refers to an atom or associated group of atoms that can undergo a further chemical reaction, for example, to form a covalent bond with another functional group. Examples of reactive functional groups include, but are not limited to, amino, thiol, hydroxy, and carbonyl groups. In one aspect, the reactive functional group includes a thiol group. Labels that can be linked to nucleotides or nucleic acids through these chemical modifications include, but are not limited to, detectable moieties such as biotin, haptens, fluorophores, and electrochemiluminescent (ECL) labels.

The term "modified oligonucleotide" refers to an oligonucleotide that includes at least one nucleoside modification, for example, a sugar modification or a nucleobase modification; or an internucleoside linkage modification. In one aspect, the modified oligonucleotide includes one or more modifications that include, but are not limited to, phosphodiester (PO); phosphorothioate (PS); 2'O-methyl (2'OMe); 2'O-methoxyethyl (MOE); phosphorothioate constrained ethyl (cEt); peptide nucleic acid (PNA); phosphoroamidate morpholino (PMO); locked nucleic acid (LNA); 2'-deoxy-2'-fluoro (2'-F); or a combination thereof. "Locked nucleic acid nucleoside" or "LNA" refers a nucleoside that includes a bicyclic sugar moiety with a 4'-CH₂-O-2'bridge. "Phosphorothioate" refers to an internucleotide linkage in which one of the non-bridging oxygens is replaced by sulfur.

The term "conjugate" refers to an atom or group of atoms that is directly or indirectly attached to an oligonucleotide. In one aspect, the conjugate is connected to the oligonucleotide through a stable linker or a cleavable linker. In one aspect, the conjugate modifies one or more properties of the oligonucleotide to which it is attached, including, but not limited to pharmacodynamics, pharmacokinetic, binding, absorption, cellular distribution, cellular uptake, charge or clearance properties. Examples of conjugates include, but are not limited to, polyethylene glycol (PEG), N-acetylegalactosamine (GalNAc), cell penetrating peptides (CPP), vitamin E (also known as α- tocopherol), aptamers, antibodies, cholesterol or cholesterol derivatives, squalene, fatty acids, nucleolipids, and spherical nucleic acids.

The term "nuclease" refers to an enzyme, for example, a hydrolase, that can cleave the backbone of an oligonucleotide polymer. In one aspect, the nuclease is a phosphodiesterase that cleaves a phosphodiester linkage in the backbone of an oligonucleotide. "Ribonuclease" or "RNase" refers to an enzyme that preferentially cleave ribonucleic acid (RNA). "Deoxyribonuclease" or "DNase" refers to an enzyme that preferentially cleaves deoxyribonucleic acid (DNA). In one aspect, the nuclease is a "single-strand specific nuclease" that preferentially cleaves single-stranded oligonucleotides or single-stranded regions of a double-stranded oligonucleotide. A single-strand specific RNase is an enzyme that preferentially cleaves single-stranded RNA. A single-strand specific DNase is an enzyme that preferentially cleaves single-stranded DNA.

"Target nucleic acid" refers to a nucleic acid of interest with a known sequence to which an oligonucleotide is designed to hybridize. In one aspect, the target nucleic acid is a nucleic acid with a known sequence to which an oligonucleotide therapeutic is designed to hybridize. In one aspect, the target nucleic acid is a sequence found in the DNA or RNA of a prokaryotic or eukaroytic organism. In one aspect, the target nucleic acid includes miRNA, therapeutic RNA, mRNA, an RNA virus, or a combination thereof. In one aspect, hybridization of the oligonucleotide to the target nucleic acid in a cell alters activity of a gene expressed by the cell. In one aspect, hybridization of the oligonucleotide to the target nucleic acid increases activity of a gene. In one aspect, hybridization of the antisense oligonucleotide to the target nucleic acid decreases activity of a gene.

In one aspect, the target nucleic acid includes 16S ribosomal DNA (16S rDNA) or 16 ribosomal RNA (16S rRNA). 16s rRNA is the ribosomal RNA component of the small subunit of ribosomes of prokaryotes responsible for the essential process of converting genetic messages to functional cell components via the translation of mRNA to proteins. The gene 16s rDNA encodes the 16S rRNA sequence. The 16S rRNA gene is conserved in bacteria, and contain hypervariable regions that can provide species-specific signature sequences and is widely used in identification of bacteria and phylogenetic, identification, classification and quantitation studies.

The term "subject" or "patient" refers to an organism to which an oligonucleotide composition is administered for experimental, diagnostic, prophylactic or therapeutic purposes and includes, but is not limited to animals, for example, mammals such as mice, rats, rabbits, non-human primates, and humans; insects; worms; and plants. In one aspect, a subject may be suffering from or susceptible to a disease or disorder.

As used herein, the term "human microbiome" refers to the collection of all microbes, such as bacteria, fungi, viruses, and their genes, that naturally live on and within the human body. In one aspect, the microbes of the human microbiome live on or within human organs, tissues and bodily fluids including the skin, mammary glands, nasal passages, seminal fluid, uterus, ovarian follicles, lung, saliva, oral mucosa, conjunctiva, biliary tract, and gastrointestinal tract. In one aspect, the human microbiome consists of microbes that are commensal and co-exist without harming humans. In one aspect, the human microbiome consists of microbes that are helpful to the human body. In once aspect, the human microbiome consists of microbes that are harmful to the human body. In one aspect, the human microbiome consists of groups of microbes that are helpful and harmful to the human body. In one aspect, the human microbiome consists of microbes that are symbiotic wherein both the human body and microbiota benefit.

"Probe" refers to a reagent that includes a single stranded oligonucleotide sequence that is capable of hybridizing to a single-strand of an oligonucleotide duplex. In one aspect, the probe includes a single stranded oligonucleotide sequence that is capable of hybridizing to a sense or an antisense strand of an oligonucleotide duplex. A "sense probe" is an oligonucleotide that includes a single stranded oligonucleotide sequence that is capable of hybridizing to a sense strand of an oligonucleotide duplex. An "antisense probe" is an oligonucleotide that includes a single stranded oligonucleotide sequence that is capable of hybridizing to an antisense strand of an oligonucleotide duplex. In one aspect, the probe includes a single stranded oligonucleotide sequence that is complementary or substantially complementary to a sense or an antisense strand of an oligonucleotide duplex. In one aspect, the probe includes an oligonucleotide tag (which can be referred to as a targeting sequence) that is complementary to sequence of a capture oligonucleotide. Probes can include DNA or RNA or a combination of DNA and RNA sequences and can include one or more modified nucleotides or modified internucleotidic linkages. Probes can be prepared by any suitable method known in the art, including, but not limited to, chemical or enzymatic synthesis.

"Linker" refers to one or more atoms that join one chemical moiety to another chemical moiety. In one aspect, a linker joins a reactive functional group or label to an oligonucleotide. The linker can be a nucleotide or non-nucleotide compound that includes one or more atoms, for example, from about 2, about 3, about 4, about 5, about 6, about 7, about 8, about 9 or about 10 atoms to about 11, about 12, about 13, about 14, about 15, about 16, about 17, about 18, about 19, or about 20 atoms and can include atoms such as carbon, oxygen, sulfur, nitrogen and phosphorus and combinations thereof. Examples of linkers include low molecular weight groups such as amide, ester, carbonate and ether groups, as well as higher molecular weight linking groups such as polyethylene glycol (PEG) and alkyl chains. Linkers may include one or more atoms, units, or molecules.

"Label" refers to a chemical group or moiety that has a detectable physical property or is capable of causing a chemical group or moiety to exhibit a detectable physical property, including, for example, an enzyme that catalyzes conversion of a substrate into a detectable product. A label can be detected by spectroscopic, photochemical, biochemical, immunochemical, electrical, optical, chemical, or other methods. Examples of labels include, but are not limited to, radioisotopes, enzymes, substrates, fluorescent molecules, chemiluminescent moieties, electrochemiluminescent moieties, magnetic particles, and bioluminescent moieties. In another aspect, the label is a compound that is a member of a binding pair, in which a first member of the binding pair (which can be referred to as a "primary binding reagent") is attached to a substrate, for example, an oligonucleotide, and the other member of the binding pair (which can be referred to as a "secondary binding reagent") has a detectable physical property or is attached to a moiety with a detectable physical property. Non-limiting examples of binding pairs include biotin and streptavidin, or avidin; complementary oligonucleotides; hapten and hapten binding partner; and antibody/antigen binding pairs.

As used herein, "concurrently" when used in connection with method steps described herein, refers to a method in which the steps are performed substantially at the same time, i.e., in which the execution of at least a portion of one method step overlaps in time with the execution of a portion of another method step. "Concurrently" does not require exact simultaneous activity, i.e., it is not required that all method steps begin or end at the same time. In one aspect, "concurrently" can mean that all reagents necessary for the method steps are combined in the same reaction mixture such that the reactions occur in the same reaction volume or during the same incubation period.

As used herein, "sequentially" when used in connection with method steps described herein, refers to a method in which the steps are performed at different points in time, for example, in which separate events occur in the practice of the method. In one aspect, the sequential steps are performed during separate incubation periods. In one aspect, the sequential steps are performed in different reaction mixtures. In one aspect, the sequential method steps are performed at a different time. In one aspect, the sequential method steps are performed at a different time, but in the same rection cell or on the same surface.

"Capture oligonucleotide" refers to an oligonucleotide reagent that can be immobilized on a support surface and is designed to hybridize to (and, therefore, to capture on the surface) a complementary oligonucleotide tag. In one aspect, the capture oligonucleotide is a single stranded sequence that can selectively hybridize, for example, under stringent hybridization conditions, with a single stranded oligonucleotide tag present on an oligonucleotide probe. Capture oligonucleotides may be provided in solid form (e.g., lyophilized), in solution, or immobilized to a support surface, e.g., on particles (e.g., microparticles, beads) or in arrays.

"Detection" can refer to detecting or quantifying the presence of a substance, such as an oligonucleotide, based on the presence or absence of a label. In one aspect, "detecting" refers to a process in which the presence or absence of a substance, such as an oligonucleotide is determined. In one aspect, "quantifying" refers to a process when an amount of a substance, such as an oligonucleotide, is determined.

"Corresponding" can be used to refer to the relationship between a capture oligonucleotide and an oligonucleotide tag, wherein the oligonucleotide tag is designed to specifically bind to a particular capture oligonucleotide sequence under stringent hybridization conditions. In one aspect, an oligonucleotide tag specifically binds to its corresponding capture oligonucleotide and does not bind or cross-react with other capture oligonucleotides under stringent conditions. In one aspect, an oligonucleotide tag specifically binds to its corresponding capture oligonucleotide and does not bind or cross-react with other capture oligonucleotides in an array under stringent conditions. In one aspect, the oligonucleotide tag is a single stranded oligonucleotide that has a sequence that is complementary to at least part of a sequence of its "corresponding" capture oligonucleotide. In one aspect, the nucleotides of the "corresponding" oligonucleotide tag and capture oligonucleotide sequences have 100% sequence complementarity based on the Watson-Crick model. In another aspect, the nucleotides of the corresponding sequences have at least about 90%, 95%, 96%, 97%, 98% or 99% sequence complementarity based on the Watson-Crick model.

"Corresponding" can be used to refer to the relationship between a sense binding portion of a sense probe, or an antisense binding portion of an antisense probe and a sense strand or antisense strand, respectively, of an oligonucleotide duplex. In one aspect, a sense binding portion of a sense probe specifically binds to its corresponding sense strand of an oligonucleotide duplex and does not bind or cross-react with the sense strand of other oligonucleotide duplexes in the sample, with the antisense strand of the oligonucleotide duplex, or with an oligonucleotide tag in the sample. In one aspect, an antisense binding portion of an antisense probe specifically binds to its corresponding antisense strand of an oligonucleotide duplex and does not bind or cross-react with the antisense strand of other oligonucleotide duplexes in the sample, with the sense strand of the oligonucleotide duplex, or with an oligonucleotide in the sample. In one aspect, the nucleotides of the "corresponding" sense binding portion or antisense binding portion and sense oligonucleotide or antisense oligonucleotide, respectively, have 100% sequence complementarity based on the Watson-Crick model. In another aspect, the nucleotides of the "corresponding" sense binding portion or antisense binding portion and sense oligonucleotide or antisense oligonucleotide, respectively have at least about 90%, 95%, 96%, 97%, 98% or 99% sequence complementarity based on the Watson-Crick model.

"Cross-react" or "cross-reactive" refers to the ability of an oligonucleotide sequence to hybridize to more than one other oligonucleotide sequence in a sample. In one aspect, the term "cross-react" refers to the ability of a first oligonucleotide sequence to hybridize to a second oligonucleotide sequence in a sample, wherein the second oligonucleotide sequence is not complementary or substantially complementary to the first oligonucleotide sequence. In one aspect, the term "cross-react" or "cross-reactive" refers to the ability of a capture oligonucleotide to hybridize to more than one oligonucleotide tag or more than one tagged target nucleotide sequence in a sample. In one aspect, the cross-reactive capture oligonucleotide hybridizes to one or more oligonucleotide tags in a sample under stringent capture hybridization conditions. "Non-cross-reactive" or "non-cross-reacting" refers to a first oligonucleotide sequence that hybridizes only to a particular oligonucleotide sequence in a sample, for example, the ability of a first oligonucleotide sequence to hybridize only to its corresponding complementary sequence in a sample. In one aspect, the term "non-cross-reactive" refers to the ability of a capture oligonucleotide to hybridize only to one oligonucleotide tag in a sample that include more than one oligonucleotide tag or more than one tagged target nucleotide sequences. In one aspect, the non-cross-reactive capture oligonucleotide hybridizes only to one oligonucleotide tag in a sample under stringent hybridization conditions. In one aspect, non-cross-reactive means that the ratio at which the first oligonucleotide binds to a sequence other than its complementary sequence in a sample is less than 0.05% under stringent hybridization conditions. In one aspect, stringent capture hybridization conditions include a temperature of between 27 °C and 47 °C, a formamide concentration between 21% and 41%, a salt concentration between 300 mM and 500 mM and a pH between 7.5 and 8.5. In one aspect, stringent capture hybridization conditions include a temperature of about 37 °C, a formamide concentration of about 31%, a salt concentration of about 400 mM and a pH of 8.0.

"Array" refers to one or more support surfaces having more than one spatially distinct (i.e., not overlapping) addressable locations, referred to herein as binding domains or array elements. In one aspect, each addressable location includes an assay reagent, including, for example, a capture oligonucleotide.

A "support surface" refers to a surface material onto which, various substances, for example, one or more capture oligonucleotides can be immobilized. A "support surface" can be planar or non-planar. In one aspect, the support surface includes a flat surface. In one aspect, the support surface is a plate with a plurality of wells, i.e., a "multi-well plate." Multi-well plates can include any number of wells of any size or shape, arranged in any pattern or configuration. In another aspect, the support surface has a curved surface. In one aspect, the support surface is provided by one or more particles, beads or microspheres. The terms particles, beads or microspheres can be used interchangeably unless otherwise indicated. In one aspect, the support surface includes color coded particles, beads or microspheres. In one aspect, the support surface includes an assay module, such as an assay plate, slide, cartridge, bead, or chip. In one aspect, the support surface includes assay flow cells or assay fluidics.

In one aspect, the support surface includes a plurality of addressable locations (which may be referred to as "spots"), for example, as is typical in "gene chip" devices. In another aspect, the array includes a plurality of support surfaces that each have one addressable location, as in "bead array" approaches where each bead in a suspension of beads represents an addressable location (which, for example, may be addressed using flow cytometric or microscopic detection techniques). In another aspect, the array includes a plurality of support surfaces that each have one or more, or two or more addressable locations per surface. The addressable locations on a support surface can be arranged in uniform rows and columns or can form other patterns. The number of addressable locations on the array can vary, for example from less than about 10 to more than about 50, about 100, about 200, about 500, or about 1000. "Multiplexing" refers to the simultaneous analysis of more than one assay target in a single assay.

In the context of analytes measured in an assay, or a reagents used in an assay, the term "plurality" means more than one structurally or functionally different analyte or reagent (e.g., reagent A and reagent B), rather than just more than one copy of the analyte or reagent (e.g., reagent A and another copy of reagent A). For example, the term "plurality of detection reagents" means that more than one structurally or functionally different detection reagent is present in an assay, for example, the different detection reagents each specifically bind a different target analyte and does not describe a situation where there are multiple copies of one reagent. However, use of the term "plurality" in this context does not preclude the possibility that multiple copies are present of any of the plurality of analytes or reagents. For example, a plurality of immobilized targeting reagent complements could refer to immobilized targeting reagent complements that include one or more copies of targeting reagent complement A and one or more copies of targeting reagent complement B. When referring to a plurality of analytes or reagents, the terms "first," "second," "third," etc. or "additional" can be used to distinguish between the unique analytes or reagents. For example, a "first" detection reagent binds to a "first" target analyte and a "second" detection reagent binds to a "second" target analyte or a different portion of the target analyte.

"Unique" is a relative term that depends on the other components present in a composition or mixture. For example, when used in connection with a nucleotide sequence, for example the nucleotide sequence of an analyte binding portion of a probe, the term "unique" means that the nucleotide sequence of one analyte binding portion is different from the nucleotide sequence of the analyte binding portion of the other probes in the composition or mixture. Similarly, when used in connection with a targeting reagent or oligonucleotide tag, the term "unique" means that the nucleotide sequence of the targeting reagent or oligonucleotide tag is different from the nucleotide sequence of other targeting reagents or tag in the composition or mixture. The term "unique" does not preclude the possibility that multiple copies of a "unique" analyte or reagent may be present in an assay or sample.

"Carbon-based" refers to a material that contains elemental carbon (C) as a principal component. Examples of carbon-containing or carbon-based materials include, but are not limited to, carbon, carbon black, graphitic carbon, glassy carbon, carbon nanotubes, carbon fibrils, graphite, carbon fibers and mixtures thereof. Carbon-based materials can include elemental carbon, including, for example, graphite, carbon black or carbon nanotubes. In one aspect, carbon-based materials include conducting carbon-polymer composites, conducting polymers, or conducting particles dispersed in a matrix, for example, carbon inks, carbon pastes, or metal inks. Conducting carbon particles include, for example, carbon fibrils, carbon black, or graphitic carbon, dispersed in a matrix, for example, a polymer matrix such as ethylene vinyl acetate (EVA), polystyrene, polyethylene, polyvinyl alcohol, polyvinyl acetate, polyvinyl chloride or acrylonitrile butadiene styrene (ABS). Such polymer matrices can also include copolymers with more than one type of component monomer which may include monomers selected from vinyl acetate, ethylene, vinyl alcohol, vinyl chloride, acrylonitrile, butadiene, styrene or other monomers.

### B. Overview

Provided herein is a method for detecting or quantifying an oligonucleotide duplex in a sample. In one aspect, a method is provided for detecting or quantifying a first and a second strand of an oligonucleotide duplex in a sample. In one aspect, a method is provided for detecting, or quantifying a sense and an antisense strand of an oligonucleotide duplex in a sample. In one aspect, the method is used for detecting, or quantifying a sense and an antisense strand of a double-stranded oligonucleotide therapeutic.

The method described herein provides a robust and sensitive method for characterizing oligonucleotide therapeutics in a variety of complex biological samples including, for example, biological fluids, including, but not limited to, plasma, serum, whole blood, urine, feces, breast milk, saliva, and amniotic fluid; and tissues or tissue homogenates, including, but not limited to, organs or organ homogenates, such as brain, liver, spleen, heart, lung, and kidney or other tissues, for example, muscle, skin, or bone marrow. In one aspect, the sample is an environmental sample. In one aspect, the sample is a manufacturing process sample.

In one aspect, the method can be used to characterize, for example, pharmacokinetics, biodistribution and cell uptake of a therapeutic oligonucleotide, including, but not limited to, pharmacokinetics (PK), pharmacodynamics (PD), clearance, half-life, peak concentration, exposure-response relationships, biodistribution, tissue targeting, tissue accumulation, tissue bioavailability, or combinations thereof. In one aspect, the method can be used to detect or quantify both strands of an oligonucleotide duplex, for example, to assess the stability of the duplex as well as the pharmacokinetics, biodistribution and cell uptake of the individual strands of an oligonucleotide duplex.

In one aspect, the method or kit is used to identify, detect or quantify one or more nucleotide sequences or variants of a microorganism. In one aspect, the method or kit is used to identify, detect or quantify one or more nucleotide sequences or variants of bacteria, fungi, protozoa or a virus. In one aspect, the method or kit is used to identify, detect or quantify one or more nucleotide sequences of bacteria, fungi, protozoa or a virus that is a component of the human microbiome. In one aspect of the method or kit is used to identify, detect or quantify one or more nucleotide sequences or variants of 16S rRNA or rDNA from bacteria.

In one aspect, the bacteria is *Achromobacter, Acidaminococcus, Acinetobacter, Actinomycetales, Aerococcus, Anaerococcus, Aggregatibacter, Aeromonas, Alcaligenes, Anaerobiospirillum, Atopobium, Bacillus, Bacillota, Bacteroides, Bacterionema, Bartonella, Bifidobacterium, Bordetella, Borrelia, Brucella, Burkholderia, Buchnera, Butyriviberio, Campylobacter, Capnocytophaga, Cardiobacterium, Chlamydia, Chlamydophila, Collinsella, Citrobacter, Clostridium, Corynebacterium, Cutibacterium, Dialister, Demodex, Eggerthella, Eikenella, Enterococcus, Enterobacter, Escherichia, Eubacterium, Faecalibacterium, Finegoldia, Firmicutes, Flavobacterium, Francisella, Fusobacterium, Gardnerella, Gordonia, Haemophilus, Helicobacter, Kingella, Klebsiella, Lactobacillus, Legionella, Leptospira, Leptotrichia, Listeria, Megasphaera, Methanobrevibacter, Microbacterium, Micrococcus, Mobiluncus, Morganella, Moraxella, Mycobacterium, Mycoplasma, Neisseria, Peptococcus, Peptoniphilus, Peptostreptococcus, Plesiomonas, Porphyromonas, Prevotella, Propionibacterium, Proteus, Providencia, Pseudomonas, Pseudomonadota, Rickettsia, Roseburia, Rothia, Ruminococcus, Sarcina, Salmonella, Selenomonas, Shigella, Slackia, Sneathia, Spirochaeta, Staphylococcus, Streptobacillus, Streptococcus, Streptomyces, Tannerella, Treponema, Trichophyton, Ureaplasma, Veillonella, Vibrio, Wolinella* or *Yersinia* bacteria.

In one aspect, a method is provided for detecting or quantifying a sense and an antisense strand of an oligonucleotide duplex in a sample. In one aspect, the method includes contacting the sample with a composition that includes a set of probes that includes a sense probe and an antisense probe. In one aspect, the sense probe includes a single stranded oligonucleotide tag that is complementary to at least a portion of a capture oligonucleotide immobilized on a support surface, a sense binding portion capable of hybridizing to a nucleotide sequence of the sense strand of the oligonucleotide duplex, and a label. In one aspect, the antisense probe includes a single stranded oligonucleotide tag that is complementary to at least a portion of a second capture oligonucleotide immobilized on the support surface, an antisense binding portion capable of hybridizing to a nucleotide sequence of the antisense strand of the oligonucleotide duplex, and a label. In one aspect, the sense binding portion of the sense probe has a sense binding length that is shorter than a sense strand length of the sense strand. In one aspect, the antisense binding portion of the antisense strand has an antisense binding length that is shorter than an antisense strand length of the antisense strand.

In one aspect, the method further includes a step of incubating the probes with the sample to form a hybridization mixture. In one aspect, the hybridization mixture includes "productive" or "desirable" hybridization complexes in which the sense probe hybridizes with the sense strand of the oligonucleotide duplex to form a sense complex and the antisense probe hybridizes with the antisense strand of the oligonucleotide duplex to form an antisense complex. In one aspect, the hybridization mixture includes one or more "non-productive" or undesirable hybridization complexes. One example of an unproductive hybridization complex is when the sense probe fails to hybridize to the sense strand of the oligonucleotide or when the antisense probe fails to hybridize to the antisense strand of the oligonucleotide. Another example of an unproductive hybridization complex is when the sense probe and antisense probes hybridize to each other to form a probe-probe complex.

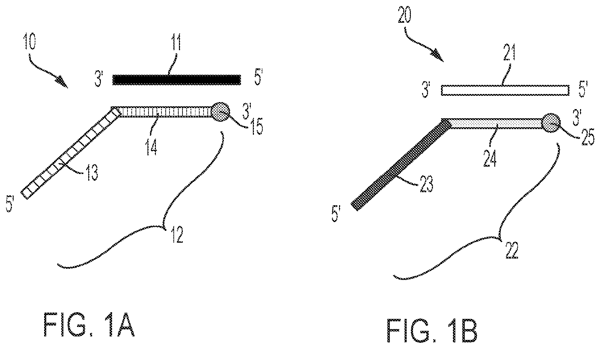

FIG. 1A is a schematic of an antisense complex 10 that includes an antisense strand 11 of an oligonucleotide duplex and an antisense probe 12, wherein the antisense probe 12 includes an oligonucleotide tag 13, an antisense binding portion 14 and a label 15. FIG. 1B is a schematic of a sense complex 20 that includes a sense strand 21 from an oligonucleotide duplex and a sense probe 22, wherein the sense probe 22 includes an oligonucleotide tag 23, a sense binding portion 24 and a label 25.

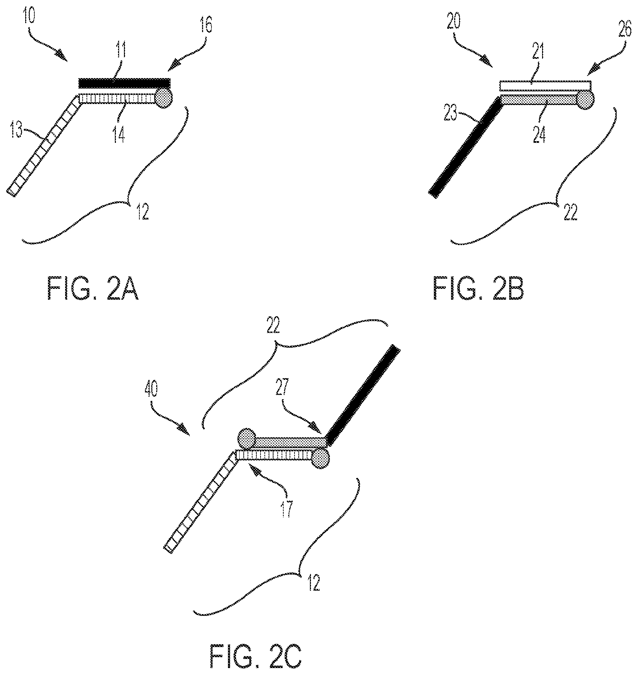

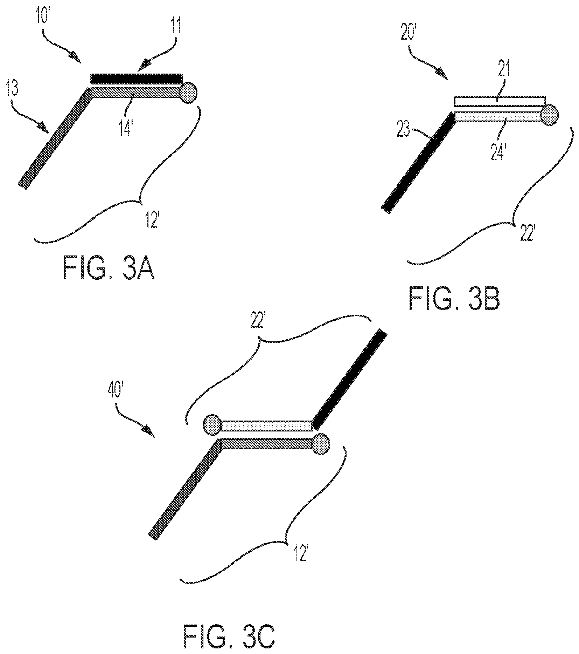

One difficulty arising when trying to detect a sense and an antisense strand of an oligonucleotide duplex is the potential unproductive binding due to probe-probe hybridization. The hybridization complexes that are possible in a hybridization mixture that contain sense and antisense strands of an oligonucleotide duplex and sense and antisense probes are shown in FIG. 2A-2C (using a probe with a short binding portion) and FIG. 3A-3C (using a probe with a full-length binding portion).

FIG. 2A is a schematic of an antisense complex 10 that includes an antisense strand 11 of an oligonucleotide duplex and an antisense probe 12, wherein the antisense probe 12 includes an oligonucleotide tag 13, a "short" antisense binding portion 14 and a label 15. FIG. 2B is a schematic of a sense complex 20 that includes a sense strand 21 from an oligonucleotide duplex and a sense probe 22, wherein the sense probe 22 includes an oligonucleotide tag 23, an "short" sense binding portion 24, and a label 25. As used herein, the term "short" binding portion means that the binding portion of the antisense and sense probes have a length that is shorter than (i.e., includes at least one less nucleotide) the antisense and sense strands of the oligonucleotide duplex, respectively, such that there is a single-stranded overhang 16 in the antisense complex 10 and a single-stranded overhang 26 in the sense complex 22.

In one aspect, a terminal portion of the antisense 11 strand of the antisense complex 10 is single-stranded. In one aspect, a 3' terminal portion of the antisense 11 strand of the antisense complex 10 is single-stranded. In one aspect, a 5' terminal portion of the antisense 11 strand of the antisense complex 10 is single-stranded. In one aspect, a terminal portion of the sense 21 strand of the sense complex 20 is single-stranded. In one aspect, a 3' terminal portion of the sense 21 strand of the sense complex 20 is single-stranded. In one aspect, a 5' terminal portion of the sense 21 strand of the sense complex 20 is single-stranded. In one aspect, the single-stranded overhang is from about 1 to about 10 nucleotides in length, about 1 to about 5 nucleotides in length, about 1 to about 3 nucleotides in length, or about 1 to about 2 nucleotides in length. In one aspect, the single-stranded overhand is about 1, about 2, about 3, about 4, about 5, about 6, about 7, about 8, about 9, or about 10 nucleotides in length.

FIG. 2C is a schematic of a probe-probe binding complex 40 in which the "short" antisense binding portion 14 of the antisense probe 12 hybridizes to the "short" sense binding portion 24 of the sense probe 22. In this situation, when the antisense probe 10 has a "short" antisense binding portion 14 and the sense probe 20 has a "short" sense binding portion 24, there is a single-strand overhang 17 and 27 exposed in the probe-probe complex 40. In one aspect, the 5' end of the antisense binding portion 14 of the antisense probe 12 in the probe-probe complex 40 is single-stranded. In one aspect, the 5' end of the sense binding portion 24 of the sense probe 22 in the probe-probe complex 40 is single-stranded. In one aspect, the 5' end of the antisense binding portion 14 of the antisense probe 12 and the 5' end of the sense binding portion 24 of the sense probe 22 in the probe-probe complex 40 are each single-stranded. In one aspect, the 3' end of the antisense binding portion 14 of the antisense probe 12 in the probe-probe complex 40 is single-stranded. In one aspect, the 3' end of the sense binding portion 24 of the sense probe 22 in the probe-probe complex 40 is single-stranded. In one aspect, the 3' end of the antisense binding portion 14 of the antisense probe 12 and the 3' end of the sense binding portion 24 of the sense probe 22 in the probe-probe complex 40 are each single-stranded. In one aspect, the single-stranded overhang is from about 1 to about 10 nucleotides in length, about 1 to about 5 nucleotides in length, about 1 to about 3 nucleotides in length, or about 1 to about 2 nucleotides in length. In one aspect, the single-stranded overhand is about 1, about 2, about 3, about 4, about 5, about 6, about 7, about 8, about 9, or about 10 nucleotides in length.

FIG. 3A is a schematic of an antisense complex 10' that includes an antisense strand 11 of an oligonucleotide duplex and an antisense probe 12', wherein the antisense probe 12' includes an oligonucleotide tag 13, a "full-length" antisense binding portion 14' and a label 15. FIG. 3B is a schematic of a sense complex 20' that includes a sense strand 21 from an oligonucleotide duplex and a sense probe 22', wherein the sense probe 22' includes an oligonucleotide tag 23, an "full-length" sense binding portion 24' and a label 25. As used herein, the term "full-length" binding portion means that the binding portion of the antisense and sense probes are the same length (i.e., include the same number of nucleotide bases) as the antisense and sense strand of the oligonucleotide duplex, respectively, such that there is no single-stranded overhang in the antisense 10' or sense complex 20'. FIG. 3C is a schematic of a probe-probe binding complex 40' in which the "full-length" antisense binding portion 14' of the antisense probe 12' is hybridized to the "full-length" sense portion 24' of the sense probe 22', in which there is no single-stranded overhang.

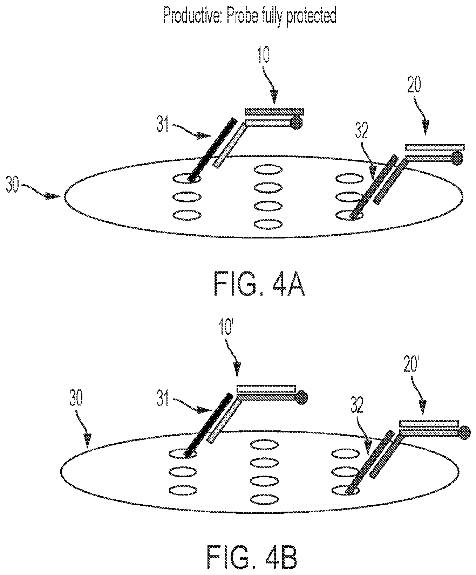

In one aspect, the support surface is contacted with the hybridization mixture under conditions in which the oligonucleotide tag of the sense or antisense probe hybridizes to a capture oligonucleotide immobilized on the support surface. In one aspect, the oligonucleotide tag of the antisense probe that is part of an antisense complex hybridizes to a capture oligonucleotide immobilized on the support surface. In one aspect, the oligonucleotide tag of the sense probe that is part of a sense complex hybridizes to a capture oligonucleotide immobilized on the support surface. In FIG. 4A, the antisense binding portion of the antisense probe is "short" such that the antisense strand portion of the antisense complex includes is a single-stranded overhang and the sense binding portion of the sense probe is "short" such that the sense strand portion of the sense complex includes a single-stranded overhang. In FIG. 4B, the antisense binding portion of the antisense probe is "full-length" such that the antisense strand portion of the antisense complex does not include a single-stranded overhang and the sense binding portion of the sense probe is "full-length" such that the sense strand portion of the sense complex does not include a single-stranded overhang. Situations in which an antisense or sense complex is hybridized to a support surface via the oligonucleotide tag of the antisense or sense probe, respectively, is referred to herein as "productive."

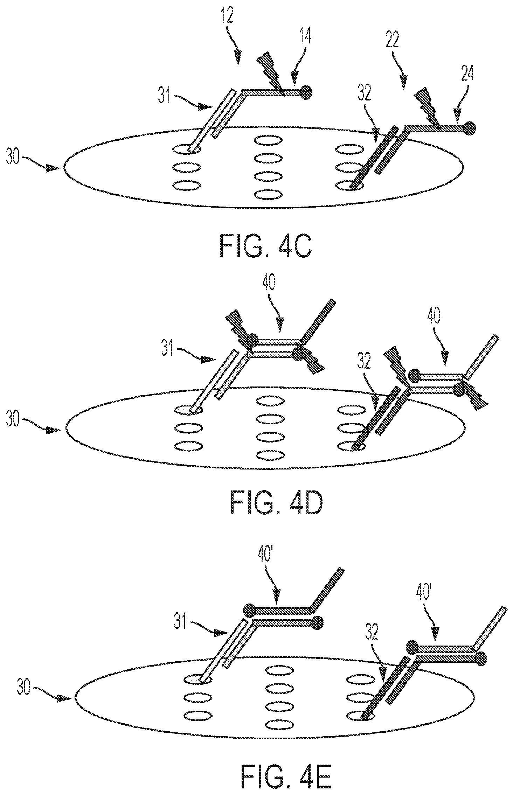

In one aspect, the oligonucleotide tag of the antisense or sense probe is not part of an antisense or sense complex, respectively. Situations in which a hybridization complex that is not an antisense complex or a sense complex is hybridized to a support surface via the oligonucleotide tag of the antisense probe is referred to herein as "unproductive." In one aspect, shown in FIG. 4C, the oligonucleotide tag of the antisense probe 12 or sense probe 22 binds only the antisense probe 12 or sense probe 22 to the support surface 30. In this situation, the antisense binding portion 14 and sense binding portion 24 of the antisense probe 12 or sense probe 22, respectively, remain single-stranded.

In one aspect, the oligonucleotide tag of the antisense or sense probe is part of a probe-probe complex and the oligonucleotide tag of the antisense probe 12 or sense probe 22 immobilizes a probe-probe complex 40' onto the support surface 30. In one aspect, shown in FIG. 4D, the antisense-binding portion and sense-binding portion of the probes are "short" such that there is a single-strand overhang in the binding portions of the probe-probe complex 40. In one aspect, shown in FIG. 4E, the antisense-binding portion and sense-binding portion of the probes are "full-length" such that there is no single-strand overhang in the binding portions of the probe-probe complex 40'.

In one aspect, the sample includes a plurality of oligonucleotide duplexes and the composition includes a plurality of sets of probes, wherein each set of probes hybridizes with a unique sense or antisense strand of a unique oligonucleotide duplex.

In one aspect, the method includes step-down hybridization conditions, in which the probes hybridize to their respective sense or antisense strands during incremental reductions in annealing temperature. In one aspect, the hybridization conditions include a denaturing step in which the sample is incubated a first temperature to denature the sense and antisense strands of the oligonucleotide duplex. In one aspect, the hybridization conditions include an annealing step in which the probes are incubated with the denatured sense and antisense strands of the oligonucleotide duplex at a second temperature to allow the sense and antisense probes to hybridize to the sense and antisense strands. In one aspect, the method includes incubating the sense and antisense complexes at a hold temperature of about 2°C to about 8°C.

In one aspect, the denaturing step includes incubating the sample at a first temperature from about 60°C to about 95°C to denature the sense and antisense strands of the oligonucleotide duplex. In one aspect, the denaturing step includes incubating the sample at a first temperature of at least about 60°C, about 65°C, about 70°C, about 75°C, or about 80°C and up to about 85°C, about 90°C, about 95°C, or about 100°C. In one aspect, the denaturing step includes incubating the sample at a first temperature of about 60°C, about 65°C, about 70°C, about 75°C, about 80°C, about 85°C, about 90°C, about 95°C, or about 100°C. In one aspect, the hybridization conditions include incubating the sample at a first temperature of about 80°C to about 95°C to denature the sense and antisense strands of the oligonucleotide duplex. In one aspect, the hybridization conditions include incubating the sample at a first temperature of about 90°C to about 95°C to denature the sense and antisense strands of the oligonucleotide duplex. In one aspect, the hybridization conditions include incubating the sample at a first temperature of about 95°C to denature the sense and antisense strands of the oligonucleotide duplex. In one aspect, the sample is incubated for about 1 minute to about 15 minutes. In one aspect, the sample is incubated for at least about 30 seconds, about 1 minute, about 2 minutes, about 3 minutes, about 4 minute or about 5 minutes and up to about 10 minutes or about 15 minutes. In one aspect, the sample is incubated for about 30 seconds, about 1 minute, about 2 minutes, about 3 minutes, about 4 minutes, about 5 minutes, about 10 minutes or about 15 minutes. In one aspect, the sample is incubated from about 1 minute to about 5 minutes. In one aspect, the sample is incubated from about 1 minute to about 2 minutes. In one aspect, the sample is incubated for about 2 minutes. In one aspect, the denaturing step includes incubating the probes with the sample a first temperature from about 60°C to about 95°C for about 1 minute to about 15 minutes to denature the sense and antisense strands of the oligonucleotide duplex. In one aspect, the hybridization conditions include incubating the probes with the sample a first temperature of about 80°C to about 95°C for about 1 minute to about 5 minutes to denature the sense and antisense strands of the oligonucleotide duplex. In one aspect, the hybridization conditions include incubating the probes with the sample a first temperature of about 95°C for about 2 minutes to denature the sense and antisense strands of the oligonucleotide duplex.

In one aspect, the annealing step includes incubating the probes with the sample at a second temperature from about 10°C to about 65°C to allow the sense and antisense probes to hybridize to the sense and antisense strands. In one aspect, the annealing step includes incubating the probes with the sample at a second temperature of at least about 10°C, about 15°C, about 20°C, about 25°C, about 30°C, about 35°C, or about 40°C and up to about 45°C, about 50°C, about 55°C, about 60°C, or about 65°C to allow the sense and antisense probes to hybridize to the sense and antisense strands. In one aspect, the annealing step includes incubating the probes with the sample at a second temperature of about 10°C, about 15°C, about 20°C, about 25°C, about 30°C, about 35°C, about 40°C, about 45°C, about 50°C, about 55°C, about 60°C, or about 65°C to allow the sense and antisense probes to hybridize to the sense and antisense strands. In one aspect, the hybridization conditions include incubating the probes with the sample at a second temperature from about 40°C and about 65°C to allow the sense and antisense probes to hybridize to the sense and antisense strands. In one aspect, the hybridization conditions include incubating the probes with the sample at a second temperature of 60°C to about 65°C to allow the sense and antisense probes to hybridize to the sense and antisense strands. In one aspect, the hybridization conditions include incubating the probes with the sample at a second temperature of about 65° to allow the sense and antisense probes to hybridize to the sense and antisense strands. In one aspect, the annealing step includes incubating the probes with the sample at a second temperature for about 30 seconds to about 5 minutes to allow the sense and antisense probes to hybridize to the sense and antisense strands. In one aspect, the annealing step includes incubating the probes with the sample at a second temperature for at least about 30 seconds, about 60 seconds, about 90 seconds and up to about 2 minutes, about 3 minutes, about 4 minutes or about 5 minutes to allow the sense and antisense probes to hybridize to the sense and antisense strands. In one aspect, the hybridization conditions include incubating the probes with the sample at a second temperature for about 1 minute to about 2 minutes to allow the sense and antisense probes to hybridize to the sense and antisense strands. In one aspect, the hybridization conditions include incubating the probes with the sample at a second temperature to allow the sense and antisense probes to hybridize to the sense and antisense strands. In one aspect, the annealing step includes incubating the probes with the sample at a second temperature from about 10°C to about 65°C for about 30 seconds to about 5 minutes to allow the sense and antisense probes to hybridize to the sense and antisense strands. In one aspect, the hybridization conditions include incubating the probes with the sample at a second temperature from about 40°C and about 65°C for about 1 minute to about 2 minutes to allow the sense and antisense probes to hybridize to the sense and antisense strands. In one aspect, the hybridization conditions include incubating the probes with the sample at a second temperature of about 65°C for about 1 min to allow the sense and antisense probes to hybridize to the sense and antisense strands. In one aspect, the hybridization conditions include incubating the probes with the sample at a hold temperature of about 2°C to about 8°C. In one aspect, the hybridization conditions include incubating the probes with the sample at a hold temperature of about 4°C.

In one aspect, the temperature transition rate between the annealing step and the hold is from about 0.05°C/s to about 0.5°C/s. In one aspect, the temperature transition rate between the annealing step and the hold is about 0.1°C/s.

In one aspect, the probes are incubated with the sample in a buffer that includes diluent 54 or N-PLEX hybridization Buffer 1 or 2.

In one aspect, the hybridization mixture containing the hybridization complexes is contacted with a single-strand specific nuclease. In one aspect, the support surface is first contacted with the hybridization mixture under conditions in which the first and second oligonucleotide tags of the sense and antisense probes hybridize to the first and second capture oligonucleotides on the support surface to immobilize the hybridization complexes on the support surface; and then the support surface is contacted with a single-strand specific nuclease. In one aspect, the hybridization mixture containing the hybridization complexes is contacted with a single-strand specific nuclease to form a reaction mixture; and then the support surface is contacted with the reaction mixture under conditions in which the oligonucleotide tags of the sense and antisense probes hybridize to the capture oligonucleotides immobilized on the support surface.

In one aspect, the single-strand specific nuclease includes a single-strand specific DNase. In one aspect, the single-strand specific DNase is S1 nuclease, P1 nuclease or Mung Bean nuclease. In one aspect, the single-strand specific nuclease includes a single-strand specific RNase. In one aspect, the single-strand specific RNase is RNase A, RNase H, RNase I, RNase III, RNase L, RNase P, RNase PhyM, RNase T1, RNase T2, RNase U2, RNase V, PNPase, RNase PH, RNase R, RNase D, RNase T, RNaseONE, oligoribonuclease, exoribonuclease I, or exoribonuclease II.

In one aspect, shown in FIG. 4C, the single-stranded nuclease cleaves unbound single-stranded probe in the hybridization complexes, detaching the label from the probe. Advantageously, this removes the label from the probe and reduces background caused by immobilization of the unbound probes to the support surface.

In one aspect, shown in FIG. 4D, the single-stranded nuclease cleaves the single-stranded overhang the probe-probe complex formed using "short" probes, removing label from the probe-probe complex and thereby reducing background levels.

In one aspect, shown in FIG. 4E, the probe-probe complex is formed from FL probes such that there is no single stranded overhang. In this situation, the labels remain immobilized on the support surface, resulting in high background levels.

In one aspect, one or more of the method steps of contacting the sample with the composition that includes the set of probes, incubating the probes with the sample to form hybridization complexes that include a sense complex and an antisense complex, contacting a support surface with the hybridization mixture that includes the hybridization complexes and contacting the support surface with a single-strand specific nuclease are performed concurrently. In one aspect, the method steps of contacting the sample with the composition that includes the set of probes, incubating the probes with the sample to form hybridization complexes that includes a sense complex and an antisense complex, contacting a support surface with the hybridization mixture that includes the hybridization complexes and contacting the hybridization complexes with a single-strand specific nuclease are all performed concurrently.

In one aspect, one or more of the method steps of contacting the sample with the composition that includes the set of probes, incubating the probes with the sample to form a hybridization mixture that includes hybridization complexes that include a sense complex and an antisense complex, contacting a support surface with the hybridization mixture and contacting the hybridization complexes with a single-strand specific nuclease are performed sequentially. In one aspect, the method steps of contacting the sample with the composition that includes the set of probes, incubating the probes with the sample to form a hybridization mixture that includes hybridization complexes that includes a sense complex and an antisense complex, contacting a support surface with the hybridization mixture and contacting the hybridization complexes with a single-strand specific nuclease are each performed sequentially.

In one aspect, the support surface is incubated with the hybridization complexes for about 15 minutes to about 12 hours. In one aspect, the support surface is incubated with the hybridization complexes for at least about 15 minutes, about 30 minutes, about 1 hour, about 2 hours, about 3 hours, about 4 hours, about 5 hours, or about 6 hours and up to about 6 hours or about 12 hours. In one aspect, the support surface is incubated with the hybridization complexes for about 30 minutes to about 3 hours. In one aspect, the support surface is incubated with the hybridization complexes for about 1 hour to about 2 hours.

In one aspect, the support surface is incubated with the hybridization complexes at a temperature of about 20 °C to about 40 °C. In one aspect, the support surface is incubated with the hybridization complexes at a temperature of at least about 20°C, about 25°C, or about 30°C, and up to about 25°C, or about 40 °C. In one aspect, the support surface is incubated with the hybridization complexes at a temperature of about 20 °C to about 40 °C. In one aspect, the support surface is incubated with the hybridization complexes at a temperature of about 30 °C to about 40 °C. In one aspect, the support surface is incubated with the hybridization complexes at a temperature of about 35 °C to about 40 °C. In one aspect, the support surface is incubated with the hybridization complexes at a temperature of about 35 °C, about 36 °C, about 37 °C, about 38 °C, about 39 °C, or about 40 °C. In one aspect, the support surface is incubated with the sense and antisense complexes at a temperature of about 37°C,

In one aspect, the support surface is incubated with the hybridization complexes while shaking. while shaking at about 700 rpm to about 900 rpm. In one aspect, the support surface is incubated with the hybridization complexes while shaking. while shaking at from about 700 rpm, 705 rpm, 710 rpm, 725 rpm, 750 rpm and up to about 800 rpm, about 850 rpm or about 900 rpm. In one aspect, the support surface is incubated with the hybridization complexes while shaking. at about 705 rpm.

In one aspect, the support surface is incubated with the hybridization complexes for about 15 minutes to about 12 hours at a temperature of about 20 °C to about 40 °C. In one aspect, the support surface is incubated with the hybridization complexes for about 1 hour to about 2 hours at a temperature of about 20 °C to about 40 °C. In one aspect, the support surface is incubated with the sense and antisense complexes while shaking. In one aspect, the support surface is incubated with the sense and antisense complexes for about 1 hour at a temperature of about 37°C, while shaking at about 705 rpm.

In one aspect, the sample includes a plurality of oligonucleotide duplexes and the probe composition includes a plurality of sets of probes, in which each set of probes hybridizes with a unique sense or antisense strand of a unique oligonucleotide duplex. In one aspect, the probe composition includes about 20 pM to about 10 nM sense probe. In one aspect, the probe composition includes from about 20 pM, about 50 pM, about 100 pM, about 150 pM, about 200 pM, or about 250 pM and up to about 0.5 nM, about 1 nM, about 5 nM, or about 10 nM sense probe. In one aspect, the probe composition includes about 100 pM to about 500 pM sense probe. In one aspect, the probe composition includes about 20 pM to about 200 pM sense probe. In one aspect, the probe composition includes about 20 pM to about 100 pM sense probe. In one aspect, the probe composition includes from about 20 pM, about 50 pM, about 100 pM, about 150 pM, about 200 pM or about 250 pM, and up to about 0.5 nM, about 1 nM, about 5 nM, or about 10 nM antisense probe. In one aspect, the probe composition includes about 100 pM to about 500 pM antisense probe. In one aspect, the probe composition includes about 20 pM to about 200 pM antisense probe.

In one aspect, the method includes detecting or quantifying the sense and antisense strands of the oligonucleotide duplex based on the presence the label immobilized on the support surface. In one aspect, the method has a lower limit of detection (LLOD) of less than about 200 pg/mL.

### C. Oligonucleotide Duplex

In one aspect, the method described herein is used to detect an oligonucleotide duplex in a sample. In one aspect, the method described herein is used to detect a first and second strand of an oligonucleotide duplex. In one aspect, the oligonucleotide duplex is a double-stranded oligonucleotide therapeutic. In one aspect, the oligonucleotide therapeutic includes a sense and an antisense strand.

In one aspect, both strands of the oligonucleotide duplex include DNA. In one aspect, the oligonucleotide duplex is a DNA/DNA duplex. In one aspect, both strands of the oligonucleotide duplex include RNA. In one aspect the oligonucleotide duplex is an RNA/RNA duplex. In one aspect, one strand of the oligonucleotide duplex includes DNA and one strand of the oligonucleotide duplex includes RNA. In one aspect, the oligonucleotide duplex is a DNA/RNA heteroduplex. In one aspect, the sense strand of the oligonucleotide duplex includes DNA. In one aspect, the sense strand of the oligonucleotide duplex includes RNA. In one aspect, the antisense strand of the oligonucleotide duplex includes DNA. In one aspect, the antisense strand of the oligonucleotide duplex includes RNA.

In one aspect, one or both strands of the oligonucleotide duplex include one or more modified nucleotides. In one aspect, one of the strands of the oligonucleotide duplex includes DNA and one or more modified nucleotides. In one aspect, one of the strands of the oligonucleotide duplex includes RNA and one or more modified nucleotides. In one aspect, the antisense strand of the oligonucleotide duplex includes DNA and one or more modified nucleotides. In one aspect, the antisense strand of the oligonucleotide duplex includes RNA and one or more modified nucleotides. In one aspect, the sense strand of the oligonucleotide duplex includes DNA and one or more modified nucleotides. In one aspect, the sense strand of the oligonucleotide duplex includes RNA and one or more modified nucleotides. In one aspect, the modified nucleotide includes a modification at a nucleobase, a sugar or an internucleotidic linkage. In one aspect, the sense strand, antisense strand or both the sense and antisense strands of the oligonucleotide duplex individually include one or more modified nucleic acids. In one aspect, the sense strand, the antisense strand or both the sense and antisense strands of the oligonucleotide duplex individually include a 5'- or 3'- bioconjugate. In one aspect, the bioconjugate includes polyethylene glycol (PEG), N-acetylgalactosamine (GalNAc), a cell penetrating peptide (CPP), *α*-tocopherol, an aptamer, an antibody, cholesterol, squalene, a fatty acid, or a nucleolipid.

In one aspect, each strand of the oligonucleotide duplex independently includes from about 5 to about 100 nucleotides. In one aspect, each strand of the oligonucleotide duplex independently includes from about 5, about 6, about 7, about 8, about 9, about 10, about 11, about 12, about 13, about 14, about 15, about 20, about 25 or about 30 nucleotides and up to about 30, about 35, about 40, about 45, about 50 or about 100 nucleotides in length. In one aspect, each strand of the oligonucleotide duplex includes from about 8 to about 50 nucleotides, about 10 to about 40 nucleotides, about 10 to about 30 nucleotides, about 12 to about 30 nucleotides, about 16 to about 30, or about 18 to about 30 nucleotides.

### D. Oligonucleotide Probes

In one aspect, a sense probe is provided that is capable of hybridizing to a sense strand of an oligonucleotide duplex. In one aspect, an antisense probe is provided that is capable of hybridizing to an antisense strand of an oligonucleotide duplex. In one aspect, an antisense probe is provided that includes an oligonucleotide tag, an antisense-binding portion and a label. In one aspect, a sense probe is provided that includes an oligonucleotide tag, a sense-binding portion and a label.

### 1. Oligonucleotide Tag

In one aspect, the sense and antisense probes include an oligonucleotide tag having a nucleic acid sequence that hybridizes to an oligonucleotide sequence of a capture oligonucleotide. In one aspect, the oligonucleotide tag includes a single-stranded oligonucleotide that is complementary to at least a portion of the nucleotide sequence of a single stranded capture oligonucleotide. In one aspect, the oligonucleotide tag hybridizes to its corresponding capture oligonucleotide.

In one aspect, the oligonucleotide tag does not cross-react with or hybridize to capture oligonucleotides that are not its corresponding capture oligonucleotide. In one aspect, the oligonucleotide tag does not cross-react with or hybridize to capture oligonucleotides that are not its corresponding capture oligonucleotide under stringent hybridization conditions. In one aspect, the oligonucleotide tag does not hybridize to the sense or antisense strand of the oligonucleotide duplex. In one aspect, the oligonucleotide tag of the sense probe does not hybridize to the antisense binding portion of the antisense probe, or vice versa. In one aspect, the oligonucleotide tag of the sense probe does not hybridize to the antisense binding portion of the antisense probe under physiologically relevant or stringent conditions. In one aspect, the oligonucleotide tag of the antisense probe does not hybridize to the sense binding portion of the sense probe under physiologically relevant or stringent conditions. In one aspect, the oligonucleotide tag does not hybridize to the sense or antisense strand of the oligonucleotide duplex under physiologically relevant or stringent conditions.

In one aspect, the oligonucleotide tag and its corresponding capture oligonucleotide have 100% "sequence complementarity" based on the Watson-Crick model. In one aspect, the oligonucleotide tag and the capture oligonucleotide have sequences that have at least about 90%, about 95%, about 96%, about 97%, about 98% or about 99% sequence complementarity based on the Watson-Crick model.

In one aspect, the oligonucleotide tag is recombinantly produced. In one aspect, the oligonucleotide tag is chemically synthesized. In one aspect, the oligonucleotide tags are not naturally occurring sequences. In one aspect, the oligonucleotide tag includes a single stranded DNA sequence. In one aspect, the oligonucleotide tag includes a single stranded RNA sequence. In one aspect, the oligonucleotide tag of the sense probe includes RNA. In one aspect, the oligonucleotide tag of the antisense probe includes RNA. In one aspect, the oligonucleotide tag of the sense probe includes DNA. In one aspect, the oligonucleotide tag of the antisense probe includes DNA.

In one aspect, the oligonucleotide tag of the antisense probe includes one or more modified nucleic acids. In one aspect, the oligonucleotide tag of the antisense probe includes one or more modified nucleotides selected from phosphodiester (PO); phosphorothioate (PS); 2'O-methyl (2' OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof. In one aspect, the oligonucleotide tag of the antisense probe includes a locked nucleic acid (LNA).

In one aspect, the oligonucleotide tag of the sense probe includes one or more modified nucleic acids. In one aspect, the oligonucleotide tag of the sense probe includes one or more modified nucleotides selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof In one aspect, the oligonucleotide tag of the sense probe includes a locked nucleic acid (LNA).

In one aspect, the oligonucleotide tag includes one or more modified nucleotides.

In one aspect, the oligonucleotide tag is attached to the 5'-end of the antisense probe. In one aspect, the oligonucleotide tag is attached to the 3'-end of the antisense probe. In one aspect, the oligonucleotide tag is attached to the 5'-end of the sense probe. In another aspect, the oligonucleotide tag is attached to the 3'-end of the sense probe. In one aspect, the oligonucleotide tag is not complementary to and does not hybridize with the sense or antisense strand of the oligonucleotide duplex.

In one aspect, the oligonucleotide tag has a nucleotide sequence that is at least about 15, about 16, about 17, about 18, about 19, about 20, about 21, about 22, about 23, about 24 or about 25 and up to about 25, about 26, about 27, about 28, about 29, about 30, about 31, about 32, about 33, about 34, about 35, about 36, about 37, about 38, about 39 or about 40, or from about 15 and about 40, or about 20 and about 30 nucleotides in length. In one aspect, the oligonucleotide tag includes a nucleotide sequence that is at least about 1, about 2, about 3, about 4, about 5, about 6, about 7, about 8, about 9, or about 10 and up to about 11, about 12, about 13, about 14, about 15, about 16, about 17, about 18, about 19, or about 20, or from about 1 to about 20, from about 10 to about 15, or from about 12 to about 13 nucleotides shorter than the complementary capture oligonucleotide sequence. In one aspect, the tag has a nucleotide sequence that is at least about 24, about 30 or about 36 nucleotides in length. In one aspect, the oligonucleotide tag has a length that is the same as the length of the corresponding capture oligonucleotide. In one aspect, the oligonucleotide tag has a length that is the shorter than the length of the corresponding capture oligonucleotide.

### 2. Binding portion

In one aspect, the antisense probe includes an antisense binding portion. In one aspect, the antisense binding portion of the antisense probe has a nucleic acid sequence that is complementary to a nucleic acid sequence of an antisense strand of an oligonucleotide duplex. In one aspect, the antisense binding portion of the antisense probe is capable of hybridizing to an antisense strand of an oligonucleotide duplex. In one aspect, the antisense binding portion and the antisense strand of the oligonucleotide duplex have 100% "sequence complementarity" based on the Watson-Crick model. In one aspect, the antisense binding portion and the antisense strand of the oligonucleotide duplex have sequences that have at least about 90%, about 95%, about 96%, about 97%, about 98% or about 99% sequence complementarity based on the Watson-Crick model.

In one aspect, the sense probe includes a sense binding portion. In one aspect, the sense binding portion of the sense probe has a nucleic acid sequence that is complementary to a nucleic acid sequence of a sense strand of an oligonucleotide duplex. In one aspect, the sense binding portion of the sense probe is capable of hybridizing to a sense strand of an oligonucleotide duplex. In one aspect, the sense binding portion and the sense strand of the oligonucleotide duplex have 100% "sequence complementarity" based on the Watson-Crick model. In one aspect, the sense binding portion and the sense strand of the oligonucleotide duplex have sequences that have at least about 90%, about 95%, about 96%, about 97%, about 98% or about 99% sequence complementarity based on the Watson-Crick model.

In one aspect, the antisense binding length of the antisense probe is shorter than the antisense strand length of the antisense strand by at least 1 nucleotide. In one aspect, the antisense binding portion has a length that is from about 1 to about 10 nucleotides shorter than the antisense strand of the oligonucleotide duplex. In one aspect, the antisense binding portion has a length that is from about 1 to about 5 nucleotides shorter than the antisense strand of the oligonucleotide duplex. In one aspect, the antisense binding portion has a length of about 10 to about 25 nucleotides, or about 10 to about 20 nucleotides, or about 10 to about 16 nucleotides in length. In one aspect, the antisense binding portion of the antisense probe has a length this is from about 50% to about 99% of the length of the antisense strand of the oligonucleotide duplex. In one aspect, the antisense binding portion of the antisense probe has a length this is from about 75% to about 95% of the length of the antisense strand of the oligonucleotide duplex.

In one aspect, the sense binding length of the sense probe is shorter than the sense strand length of the sense strand by at least 1 nucleotide. In one aspect, the sense binding portion has a length that is from about 1 to about 10 nucleotides shorter than the sense strand of the oligonucleotide duplex. In one aspect, the sense binding portion has a length that is from about 1 to about 5 nucleotides shorter than the sense strand of the oligonucleotide duplex. In one aspect, the sense binding portion has a length of about 10 to about 25 nucleotides, or about 10 to about 20 nucleotides, or about 10 to about 16 nucleotides in length. In one aspect, the sense binding portion of the sense probe has a length this is from about 50% to about 99% of the length of the sense strand of the oligonucleotide duplex. In one aspect, the sense binding portion of the sense probe has a length this is from about 75% to about 95% of the length of the sense strand of the oligonucleotide duplex.

In one aspect, the sense binding portion of the sense probe has a 5' end that aligns with a 3' end of the sense strand of the oligonucleotide duplex. In one aspect, the sense binding portion of the sense probe has a 3' end that aligns with a 5' end of the sense strand of the oligonucleotide duplex. In one aspect, the antisense binding portion of the antisense probe has a 5' end that aligns with a 3' end of the antisense strand of the oligonucleotide duplex. In one aspect, the antisense binding portion of the antisense probe has a 3' end that aligns with a 5' end of the antisense strand of the oligonucleotide duplex.

In one aspect, the antisense binding portion of the antisense probe includes DNA. In one aspect, the antisense binding portion of the antisense probe includes RNA. In one aspect, the sense binding portion of the sense probe includes DNA. In one aspect, the sense binding portion of the sense probe includes RNA.

In one aspect, the antisense binding portion and the oligonucleotide tag of the antisense probe include DNA. In one aspect, the antisense binding portion and the oligonucleotide tag of the antisense probe include RNA. In one aspect, the antisense binding portion of the antisense probe includes DNA and the oligonucleotide tag of the antisense probe includes RNA. In one aspect, the antisense binding portion of the antisense probe includes RNA and the oligonucleotide tag of the antisense probe includes DNA.

In one aspect, the sense binding portion and the oligonucleotide tag of the sense probe include DNA. In one aspect, the sense binding portion and the oligonucleotide tag of the sense probe include RNA. In one aspect, the sense binding portion of the sense probe includes DNA and the oligonucleotide tag of the sense probe includes RNA. In one aspect, the sense binding portion of the sense probe includes RNA and the oligonucleotide tag of the sense probe includes DNA.

In one aspect, the antisense probe is a chimeric probe that includes an antisense binding portion that includes DNA and an oligonucleotide tag that includes RNA, wherein the antisense binding portion the antisense probe has an antisense binding length that is shorter than the antisense strand length of the antisense strand of the oligonucleotide duplex by at least 1 nucleotide. In one aspect, the antisense probe is a chimeric probe that includes an antisense binding portion that includes RNA and an oligonucleotide tag that includes DNA, wherein the antisense binding portion the antisense probe has an antisense binding length that is shorter than the antisense strand length of the antisense strand of the oligonucleotide duplex by at least 1 nucleotide. In one aspect, the sense probe is a chimeric probe that includes a sense binding portion that includes DNA and an oligonucleotide tag that includes RNA, wherein the sense binding portion the sense probe has a sense binding length that is shorter than the sense strand length of the sense strand of the oligonucleotide duplex by at least 1 nucleotide. In one aspect, the sense probe is a chimeric probe that includes a sense binding portion that includes RNA and an oligonucleotide tag that includes DNA, wherein the sense binding portion the sense probe has a sense binding length that is shorter than the sense strand length of the sense strand of the oligonucleotide duplex by at least 1 nucleotide.

In one aspect, the binding portion of the antisense probe includes one or more modified nucleic acids. In one aspect, the binding portion of the antisense probe includes one or more modified nucleotides selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof. In one aspect, the binding portion of the antisense probe includes a locked nucleic acid (LNA).

In one aspect, the binding portion of the sense probe includes one or more modified nucleic acids. In one aspect, the binding portion of the sense probe includes one or more modified nucleotides selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof. In one aspect, the binding portion of the sense probe includes a locked nucleic acid (LNA).

### 3. Label

In one aspect, the probe includes a label. In one aspect, the label is attached directly to the probe. In another aspect, the label is attached to the probe through a linker. In one aspect, the label is attached to the 5' end of the probe. In one aspect, the label is attached to the 3' end of the probe. In one aspect, the label is a primary label. In one aspect, the primary label has a detectable physical property. Examples or primary labels include, but are not limited to, radioisotopes, enzymes, substrates, fluorescent molecules, chemiluminescent moieties, electrochemiluminescent (ECL) moieties, magnetic particles, and bioluminescent moieties. In one aspect, the primary label is an electrochemiluminescence (ECL) label. In one aspect, the ECL label is an organometallic complex that includes a transition metal, for example, ruthenium. In one aspect, the primary label is a MSD SULFO-TAG label (Meso Scale Diagnostics, LLC, Rockville, MD, U.S.A.).

In one aspect, the label is a compound that is a member of a binding pair, in which a first member of the binding pair (which can be referred to as a "primary binding reagent") is attached to a substrate, for example, an oligonucleotide, and the other member of the binding pair (which can be referred to as a "secondary binding reagent") has a detectable physical property or is attached to a moiety that has a detectable physical property. Non-limiting examples of binding pairs include biotin and streptavidin, or avidin; complementary oligonucleotides; hapten and hapten binding partner; and antibody-antigen binding pairs. In one aspect, the label is a primary binding agent that includes biotin. In one aspect, the secondary binding reagent includes streptavidin. In one aspect, the secondary binding reagent includes a MSD SULFO-TAG label (Meso Scale Diagnostics, LLC, Rockville, MD, U.S.A.).

### E. Samples

In one aspect, the oligonucleotide duplex is in a sample. In one aspect, the sample is a biological sample obtained or derived from a source of interest. In one aspect, a sample is an organism or is obtained from an organism. In one aspect, a sample is a plant or is obtained from a plant. In one aspect, the sample is an animal or is obtained from an animal. In one aspect, the sample is obtained from a mammal. In one aspect, the sample is obtained from a human. In one aspect, the source of interest includes a bioreactor. In one aspect, the sample is a manufacturing process sample. In one aspect, the sample is an environmental sample. In one aspect, the environmental sample includes a water sample, including, for example, an aquifer sample, a ground water sample, or a waste water sample. In one aspect, the environmental sample includes a soil sample. In one aspect, the environmental sample includes a soil microorganism sample. In one aspect, the sample includes cell-free DNA.

In one aspect, the sample includes a biological sample. In one aspect, the sample includes an untreated biological sample. In one aspect, the sample includes a pretreated biological sample. In one aspect, the sample is pretreated, for example, to remove one or more components or to add one or more agents. In one aspect, the sample includes a purified sample. Methods of purifying oligonucleotides from a biological sample are known and include, for example, precipitation, centrifugation, and column chromatography. In one aspect, column chromatography includes high performance liquid chromatography (HPLC), for example, reverse phase high performance liquid chromatography (RP-HPLC), anion exchange high pressure liquid chromatography (AEX HPLC) or polyacrylamide gel electrophoresis (PAGE). In one aspect, the sample is an extracted sample. In one aspect, the sample is filtered, for example, using a semi-permeable membrane. In one aspect, the sample includes oligonucleotides extracted from a sample or obtained by subjecting a sample to techniques such as amplification or reverse transcription of mRNA.

In one aspect, the sample includes one or more target oligonucleotide sequences. In one aspect, the sample includes one or more amplified target oligonucleotide sequences. In one aspect, the sample includes one or more amplified target oligonucleotide sequence obtained by methods including, but not limited to, polymerase chain reaction (PCR) or rolling circle amplification (RCA).

In one aspect, the biological sample includes biological tissue or fluid, including, for example, body fluids, secretions, excretions, cells, tissues or organs or homogenates thereof. In one aspect, the biological fluid includes plasma, serum, whole blood, lymph, urine, feces, breast milk, sputum, saliva, ascites, cerebrospinal fluid, peritoneal fluid, pleural fluid and amniotic fluid. In one aspect, the biological tissue includes tissue or tissue homogenates, including, but not limited to, organs or organ homogenates, such as brain, liver, spleen, heart, lung, and kidney or other tissues, for example, muscle, skin, or bone marrow. In one aspect, the biological sample includes tissue or fine needle biopsy samples, cell-containing body fluids, free floating nucleic acids, gynecological fluids, skin swabs, vaginal swabs, oral swabs, nasal swabs, washings or lavages such as a ductal lavages or bronchoalveolar lavages, aspirates, scrapings; surgical specimens.

In one aspect, the biological sample includes a sample isolated from a portion of the human body. In one aspect, biological sample includes a sample isolated from a nasal passage, oral cavity, skin, ear, mucus membrane, gastrointestinal tract, urogenital tract, respiratory tract, eye or a combination thereof. In one aspect, the biological sample includes a sample obtained from a portion of the human body using methods known in the art, including, but not limited to swabbing, puncture sampling, and serum sampling.

In one aspect, the sample includes a naturally occurring RNase. For samples that include a naturally occurring RNase, it may be desirable to contact the sample with an RNase inhibitor before the sample is contacted with the sense and antisense probes.

### F. Capture oligonucleotide

In one aspect, the method or kit includes one or more capture oligonucleotides that are or can be immobilized in discrete binding domains on a support surface. In one aspect, the capture oligonucleotides are not naturally occurring sequences. In another aspect, the capture oligonucleotides are recombinantly produced. In one aspect, the capture oligonucleotides are chemically synthesized.

In one aspect, the capture oligonucleotides are single stranded capture oligonucleotides having nucleotide sequences that are complementary to a nucleotide sequence of a single stranded oligonucleotide tag. In one aspect, the oligonucleotide tag is attached to a sense or an antisense probe.

In one aspect, the method or kit includes a unique capture oligonucleotide the sense and antisense strand of an oligonucleotide duplex of interest. In one aspect, the capture oligonucleotides are immobilized on a support surface. In one aspect, the capture oligonucleotides are immobilized in an array. In one aspect, the array includes two or more capture oligonucleotides. In one aspect, the array includes from about 2 to about 150 or more capture oligonucleotides.

In one aspect, one or more capture oligonucleotides include single stranded nucleic acid sequences, including for example, nucleic acid sequences including deoxyribonucleic acids (DNA), ribonucleic acids (RNA), or structural analogs that include non-naturally occurring chemical structures that can also participate in hybridization reactions.

In one aspect, the capture oligonucleotides used in a particular array have similar binding energies or melting temperatures (Tm), for example, within at least about 0.5°C, about 1°C, about 2°C, about 3°C, about 4°C, or about 5°C of each other, wherein the melting temperature (Tₘ) of an oligonucleotide refers to the temperature at which 50% of the oligonucleotides is hybridized with its complement and 50% is free in solution. Tₘ can be determined using known methods, for example, by measuring the absorbance change of the oligonucleotide with its complement as a function of temperature. In one aspect, the capture oligonucleotide has a melting temperature (Tₘ) at 50mM NaCl of between about 50°C and about 70°C, about 55°C and about 65°C, or at least about 50°C, about 55°C, or about 60°C and up to about 60°C, about 65°C, or about 70°C. In one aspect, the capture oligonucleotide has a GC content between about 40% and about 60%, or about 40% and about 50%.

In one aspect, the capture oligonucleotide is between about 20 and about 100, about 30 and about 50, or about 35 and about 40 nucleotides in length, for example, at least about 20, about 21, about 22, about 23, about 24, about 25, about 26, about 27, about 28, about 29, about 30, about 31, about 32, about 33, about 34, about 35, or about 36 and up to about 36, about 37, about 38, about 39, about 40, about 41, about 42, about 43, about 44, about 45, about 46, about 47, about 48, about 49, about 50, about 75 or about 100 nucleotides in length. In one aspect, the capture oligonucleotide includes at least 20, about 24, about 30 or about 36 nucleotides. In one aspect, one or more capture oligonucleotides in an array are not identical in length to the nucleic acid sequence of its complementary oligonucleotide tag. In one aspect, the capture oligonucleotide has a sequence that is longer than the sequence of its complementary single stranded oligonucleotide tag, for example, by up to about 5, about 10, about 15, about 20 or about 25 bases.

In one aspect, one or more capture oligonucleotides are covalently or non-covalently immobilized to a support surface. In one aspect, one or more capture oligonucleotides are covalently or non-covalently immobilized to one or more binding domains on a support surface. In one aspect, the capture oligonucleotide is adsorbed to the support surface via electrostatic interactions, for example, between a negatively charged phosphate group on the oligonucleotide and a positive charge on the support surface. In one aspect, one or more capture oligonucleotides are immobilized to the support surface through the binding of a first binding partner attached (directly or through a linker moiety) to the capture oligonucleotide to a second binding partner that is immobilized on the surface. In one aspect, one or more capture oligonucleotides are covalently immobilized to the support surface. In one aspect, one or more capture oligonucleotides are directly immobilized to the support surface. In another aspect, the capture oligonucleotide is immobilized to the support surface through a linker.

Capture oligonucleotides are disclosed in International Application Publication No. WO 2020/227016, entitled "KITS FOR DETECTING ONE OR MORE TARGET NUCLEIC ACID ANALYTES IN A SAMPLE AND METHOD OF MAKING AND USING THE SAME" (Meso Scale Technologies, LLC., Rockville, MD, USA), the disclosure of which is incorporated by reference in its entirety.

### G. Support surface

In one aspect, one or more capture oligonucleotides are immobilized on a support surface. The capture oligonucleotides can be immobilized on a variety of support surfaces, including support surfaces used in conventional binding assays. In one aspect, the support surface has a flat surface. In another aspect, the support surface has a curved surface. In one aspect, the support surface includes an assay module, such as an assay plate, slide, cartridge, bead, or chip. In one aspect, the support surface includes color coded microspheres. See, for example, Yang et al. (2001) BADGE, BeadsArray for the Detection of Gene Expression, a High-Throughput Diagnostic Bioassay. Genome Res. 11(11):1888-1898. In one aspect, the support surface includes one or more beads on which one or more capture oligonucleotides are immobilized.

Support surfaces can be made from a variety of suitable materials including polymers, such as polystyrene and polypropylene, ceramics, glass, composite materials, including, for example, carbon-polymer composites such as carbon-based inks. In one aspect, the support surface is a carbon-based support surface.

In one aspect, the support surface is provided by one or more particles or "beads". In one aspect, the beads can have a diameter up to about 1 cm (or about 10,000 µm), about 5,000 µm, about 1,000 µm, about 500 µm or about 100 µm. In one aspect, beads have a diameter from about 10 nm and about 100 µm, from about 100 nm and about 10 µm or from about 0.5 µm and about 5 µm. In one aspect, the beads are paramagnetic, providing the ability to capture the beads through the use of a magnetic field. In one aspect, the support surface is provided by streptavidin or avidin-coated magnetic beads and biotin-labeled capture oligonucleotides are immobilized on the beads.

In one aspect, the support surface is a plate with a plurality of wells, i.e., a "multi-well plate." Multi-well plates can include any number of wells of any size or shape, arranged in any pattern or configuration. In one aspect, the multi-well plate includes from about 1 to about 10,000 wells. In one aspect, the multi-well assay plates use industry standard formats for the number, size, shape and configuration of the plate and wells. Examples of standard formats include 96-, 384-, 1536- and 9600-well plates, with the wells configured in two-dimensional arrays. Other multi-well formats include single well, two well, six well and twenty-four well and 6144 well plates. In one aspect, the support surface includes a 96 well-plate.

In one aspect, the support surface includes a two-dimensional patterned array in which capture oligonucleotides are printed at known locations, referred to as binding domains. In one aspect, the support surface includes a patterned array of discrete, non-overlapping, addressable binding domains to which capture oligonucleotides are immobilized, wherein the sequence of the capture oligonucleotide in each binding domain is known and can be correlated with an appropriate target analyte. In one aspect, all capture oligonucleotides in a particular binding domain have the same sequence and the capture oligonucleotides in one binding domain have a sequence different from capture oligonucleotides in other binding domains. In one aspect, multiple binding domains are arrayed in orderly rows and columns on a support surface and the precise location and sequence of each binding domain is recorded in a computer database. In one aspect, the array is arranged in a symmetrical grid pattern. In other aspects, the array is arranged another pattern, including, but not limited to, radially distributed lines, spiral lines, or ordered clusters. In another aspect, each binding domain is positioned on a surface of one or more microparticles or beads wherein the microparticles or beads are coded to allow for discrimination between different binding domains.

In one aspect, the support surface is a multi-well plate that includes one or more discrete addressable binding domains within each well that correspond to one or more capture oligonucleotides. In one aspect, the support surface includes at least one binding domain for detecting a wild-type nucleotide sequence and separate binding domain for detecting a mutant nucleotide sequence. In one aspect, each well includes at least about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24 or about 25 binding domains. In one aspect, each well includes at least about 7, about 10, about 16, or about 25 binding domains.

In one aspect, the support surface is a multi-well plate that includes at least 24, 96, or 384 wells and each well includes array of up to 10 binding domains in which different capture oligonucleotides are immobilized in discrete binding domains. In a more particular aspect, the support surface is a 96 well plate in which each well includes an array having up to 10 binding domains. In one aspect, each well of a 96-well plate includes up to 10 binding domains, having up to 10 distinct capture oligonucleotides immobilized thereon. In one aspect, each well includes the same patterned array with the same capture oligonucleotides. In another aspect, different wells may include a different patterned array of capture oligonucleotides.

### H. Nuclease protection assay (NPA)

In one aspect, a method is provided for detecting or quantifying a sense and an antisense strand of an oligonucleotide duplex in a sample using a nuclease protection assay. In one aspect, the sample is contacted with a set of probes, wherein the set of probes includes a sense probe and an antisense probe. In one aspect, the sense probe includes a single stranded oligonucleotide tag that is complementary to at least a portion of a capture oligonucleotide immobilized on a support surface, a sense binding portion capable of hybridizing to a nucleotide sequence of the sense strand of the oligonucleotide duplex, and a label. In one aspect, the antisense probe includes a single stranded oligonucleotide tag that is complementary to at least a portion of a capture oligonucleotide immobilized on the support surface, an antisense binding portion capable of hybridizing to a nucleotide sequence of the antisense strand of the oligonucleotide duplex, and a label. In one aspect, the method includes incubating the probes with the sample to form a hybridization mixture that contains hybridization complexes. In one aspect, the method includes contacting a support surface with the hybridization mixture under conditions in which the oligonucleotide tags of the hybridization complexes hybridize to the capture oligonucleotides immobilized on the support surface. In one aspect, the method includes detecting or quantifying the sense and antisense strands of the oligonucleotide duplex based on the presence of label immobilized on the support surface. In one aspect, the method includes detecting or quantifying the sense and antisense strands of the oligonucleotide duplex based on the presence of labelled sense and antisense hybridization complexes immobilized on the support surface.

In one aspect, the sense binding portion of the sense probe has a sense binding length that is shorter than a sense strand length of the sense strand. In one aspect, the sense binding portion of the sense probe has a sense binding length that is shorter than a sense strand length of the sense strand by at least 1 nucleotide. In one aspect, the antisense binding portion of the antisense strand has an antisense binding length that is shorter than an antisense strand length of the antisense strand. In one aspect, the antisense binding portion of the antisense strand has an antisense binding length that is shorter than an antisense strand length of the antisense strand by at least 1 nucleotide.

In one aspect, the sense probe includes DNA. In one aspect, the sense binding portion of the sense probe includes DNA. In one aspect, the oligonucleotide tag of the sense probe includes DNA. In one aspect, the sense binding portion and the oligonucleotide tag of the sense probe include DNA.

In one aspect, the sense probe includes RNA. In one aspect, the sense binding portion of the sense probe includes RNA. In one aspect, the oligonucleotide tag of the sense probe includes RNA. In one aspect, the sense binding portion and the oligonucleotide tag of the sense probe include RNA.

In one aspect, the sense binding portion of the sense probe includes DNA and the oligonucleotide tag of the sense probe includes RNA. In one aspect, the sense binding portion of the sense probe includes RNA and the oligonucleotide tag of the sense probe includes DNA.

In one aspect, the antisense probe includes DNA. In one aspect, the antisense binding portion of the antisense probe includes DNA. In one aspect, the oligonucleotide tag of the antisense probe includes DNA. In one aspect, the antisense binding portion and the oligonucleotide tag of the antisense probe include DNA.

In one aspect, the antisense probe includes RNA. In one aspect, the antisense binding portion of the antisense probe includes RNA. In one aspect, the oligonucleotide tag of the antisense probe includes RNA. In one aspect, the antisense binding portion and the oligonucleotide tag of the antisense probe include RNA.

In one aspect, the antisense binding portion of the antisense probe includes DNA and the oligonucleotide tag of the antisense probe includes RNA. In one aspect, the antisense binding portion of the antisense probe includes RNA and the oligonucleotide tag of the antisense probe includes DNA.

In one aspect, the antisense probe is a chimeric probe that includes an antisense binding portion that includes DNA and an oligonucleotide tag that includes RNA, wherein the antisense binding portion the antisense probe has an antisense binding length that is shorter than the antisense strand length of the antisense strand of the oligonucleotide duplex by at least 1 nucleotide. In one aspect, the antisense probe is a chimeric probe that includes an antisense binding portion that includes RNA and an oligonucleotide tag that includes DNA, wherein the antisense binding portion the antisense probe has an antisense binding length that is shorter than the antisense strand length of the antisense strand of the oligonucleotide duplex by at least 1 nucleotide. In one aspect, the sense probe is a chimeric probe that includes a sense binding portion that includes DNA and an oligonucleotide tag that includes RNA, wherein the sense binding portion the sense probe has a sense binding length that is shorter than the sense strand length of the sense strand of the oligonucleotide duplex by at least 1 nucleotide. In one aspect, the sense probe is a chimeric probe that includes a sense binding portion that includes RNA and an oligonucleotide tag that includes DNA, wherein the sense binding portion the sense probe has a sense binding length that is shorter than the sense strand length of the sense strand of the oligonucleotide duplex by at least 1 nucleotide.

In one aspect, the method includes incubating the set of probes with the sample to form a hybridization mixture. In one aspect, the hybridization mixture contains hybridization complexes that include a sense complex and an antisense complex. In one aspect, the hybridization mixture includes a sense complex that includes the sense probe hybridized with the sense strand of the oligonucleotide duplex; and an antisense complex that includes the antisense probe hybridized with the antisense strand of the oligonucleotide duplex.

In one aspect, the hybridization mixture further contains one or more of the following unproductive hybridization complexes: a sense probe that is not hybridized with a sense strand of the oligonucleotide duplex; an antisense probe that is not hybridized to the antisense strand of the oligonucleotide duplex; or a probe-probe complex in which the sense probe and antisense probes hybridize to each other. In one aspect, the unproductive hybridization complex includes a single strand overhang. In one aspect, the unproductive hybridization complex includes a single strand oligonucleotide sequence. In one aspect, the unproductive hybridization complex includes a single strand RNA sequence. In one aspect, the unproductive hybridization complex includes a single strand DNA sequence.

In one aspect, the single-strand overhang or single strand oligonucleotide sequence in an unproductive hybridization complex is digested with a single-strand specific nuclease. In one aspect, the single-strand overhang or single strand oligonucleotide sequence is a DNA sequence that is digested with a single-strand specific DNase. In one aspect, the single-strand overhang or single strand oligonucleotide sequence is an RNA sequence that is digested with a single-strand specific RNase.

In one aspect, digestion of the single-stranded overhang or single strand oligonucleotide sequence in an unproductive hybridization complex is performed while the hybridization complexes are in solution (i.e., before they hybridization complexes are immobilized to the support surface via the oligonucleotide tags of the sense or antisense probes). In one aspect, digestion of the single-stranded overhang or single strand oligonucleotide sequence in an unproductive hybridization complex is performed after the hybridization complexes are immobilized on a support surface via the oligonucleotide tags of the sense or antisense probes. In one aspect, the method includes a wash step after the single stranded overhang or single stranded oligonucleotide sequences are digested with a single-strand specific RNase. In one aspect, the method includes a wash step after the single stranded overhang or single stranded oligonucleotide sequences are digested with a single-strand specific RNase and immobilized on the support surface. In one aspect, the sense and antisense strands of the oligonucleotide duplex are detected or quantified based on the presence of label immobilized on the support surface. In one aspect, the sense and antisense strands of the oligonucleotide duplex are detected or quantified based on the presence of labeled sense or antisense complexes immobilized on the support surface.

### I. Electrodes

In one aspect, a sense or an antisense strand of an oligonucleotide duplex is detected or quantified using electrochemiluminescence (ECL). Multiplexed measurement of analytes using electrochemiluminescence is described in U.S. Pat. Nos. 7,842,246 and 6,977,722, the disclosures of which are incorporated herein by reference in their entireties.

In one aspect, the support surface includes one or more electrodes. In one aspect, the support surface includes one or more working electrodes and one or more counter electrodes. In one aspect, the support surface includes one or more binding domains formed on one or more electrodes for use in electrochemical or electrochemiluminescence assays.

In one aspect, the binding domains are formed by collecting beads coated with capture oligonucleotides onto the electrode surface. In one aspect, the beads are paramagnetic and the beads are collected on the electrode through the use of a magnetic field.

In one aspect, the electrodes are provided within an assay module that provides assay containers, assay flow cells, assay fluidics or other components useful for carrying out an assay. Examples of assay modules for carrying out electrochemiluminescence assays include, for example, multiarray case, assay plates case, cartridge case, and the like. In one aspect, the electrodes are provided within an assay module that provides assay containers, assay flow cells, assay fluidics or other components useful for carrying out an assay. Examples of assay modules for carrying out electrochemiluminescence assays can be found in U.S. Patent Nos. 6,673,533, 7,842,246, 9,731,297, and 8,298834. In one aspect, the support surface is multi-well plate that includes at least one electrode. In one aspect, each well of a multi-well assay plate includes at least one electrode. In one aspect, at least one well of the multi-well assay plate includes a working electrode. In another aspect, at least one well of the multi-well assay plate includes a working electrode and a counter electrode. In another aspect, each well of the multi-well assay plate includes a working electrode and a counter electrode. In one aspect, the working electrode is adjacent, but not in electrical contact with the counter electrode.

In one aspect, the electrodes are constructed from a conductive material, including, for example, a metal such as gold, silver, platinum, nickel, steel, iridium, copper, aluminum, a conductive alloy, or combinations thereof. In another aspect, the electrodes include semiconducting materials such as silicon and germanium or semi-conducting films such as indium tin oxide (ITO) and antimony tin oxide (ATO). In another aspect, the electrodes include oxide coated metals, such as aluminum oxide coated aluminum. In one aspect, the electrode includes a carbon-based material. In one aspect the electrodes include mixtures of materials containing conducting composites, inks, pastes, polymer blends, and metal/non-metal composites, including for example, mixtures of conductive or semi-conductive materials with non-conductive materials. In one aspect, the electrodes include carbon-based materials such as carbon, glassy carbon, carbon black, graphitic carbon, carbon nanotubes, carbon fibrils, graphite, carbon fibers and mixtures thereof. In one aspect, the electrodes include conducting carbon-polymer composites, conducting polymers, or conducting particles dispersed in a matrix, for example, carbon inks, carbon pastes, or metal inks. In one aspect, the working electrode is made of a carbon-polymer composite that includes, for example, conducting carbon particles, such as carbon fibrils, carbon black, or graphitic carbon, dispersed in a matrix, for example, a polymer matrix such as ethylene vinyl acetate (EVA), polystyrene, polyethylene, polyvinyal acetate, polyvinyl chloride, polyvinyl alcohol , acrylonitrile butadiene styrene (ABS), or copolymers of one or more of these polymers.

In one aspect, the working electrode is made of a continuous conducting sheet or a film of one or more conducting materials, which may be extruded, pressed or molded. In another aspect, the working electrode is made of a conducting material deposited or patterned on a substrate, for example, by printing, painting, coating, spin-coating, evaporation, chemical vapor deposition, electrolytic deposition, electroless deposition, photolithography or other electronics microfabrication techniques. Inone aspect, the working electrode includes a conductive carbon ink printed on a polymeric support, for example, by ink-jet printing, laser printing, or screenprinting. Carbon inks are known and include materials produced by Acheson Colloids Co. (e.g., Acheson 440B, 423ss, PF407A, PF407C, PM-003A, 30D071, 435A, Electrodag 505SS, and Aquadag^{™}), E. I. Du Pont de Nemours and Co. (e.g., Dupont 7105, 7101, 7102, 7103, 7144, 7082, 7861D, and CB050), Conductive Compounds Inc (e.g., C-100), and Ercon Inc. (e.g., G-451).

In one aspect, the working electrode is a continuous film. In another aspect, the working electrode includes one or more discrete regions or a pattern of discrete regions. Alternately, the working electrode may include a plurality of connected regions. One or more regions of exposed electrode surface on a working electrode can be defined by a patterned insulating layer covering the working electrode, for example, by screen printing a patterned dielectric ink layer over a working electrode, or by adhering a die-cut insulating film. The exposed regions may define the array elements of arrays of reagents printed on the working electrode and may take on array shapes and patterns as described above. In one aspect, the insulating layer defines a series of circular regions (or "spots") of exposed working electrode surface.

A counter electrode may have one or more of the properties described above generally for working electrodes. In one aspect, the working and counter electrodes are constructed from the same material. In another aspect, the working and counter electrodes are not constructed from the same material, for example, the working electrode may be a carbon electrode and the counter electrode may be a metal electrode.

In one aspect, one or more capture oligonucleotides are immobilized on one or more electrodes by passive adsorption. In another aspect, one or more capture oligonucleotides are covalently immobilized on the electrodes. In one aspect the electrodes are derivatized or modified, for example, to immobilize reagents such as capture oligonucleotides on the surface of the electrodes. In one aspect, the electrode is modified by chemical or mechanical treatment to improve the immobilization of reagents, for example, to introduce functional groups for immobilization of reagents or to enhance its adsorptive properties. Examples of functional groups that can be introduced include, but are not limited, to carboxylic acid (COOH), hydroxy (OH), amino (NH₂), activated carboxyls (e.g., N-hydroxy succinimide (NHS)-esters), poly-(ethylene glycols), thiols, alkyl ((CH₂)ₙ) groups, or combinations thereof). In one aspect, one or more reagents, for example, capture oligonucleotides, are immobilize by either covalent or noncovalent means to a carbon-containing electrode, for example, carbon black, fibrils, or carbon dispersed in another material. It has been found that capture oligonucleotides having thiol groups can bind covalently to carbon-containing electrodes, for example to screen-printed carbon ink electrodes, without having to first deposit an additional thiol-reactive layer such as a protein layer or a chemical cross-linking layer. In one aspect, methods are provided for direct attachment of capture oligonucleotides having thiol groups, such as thiol-modified oligonucleotides, to electrodes which provide simple, robust, efficient and reproducible processes for forming capture surfaces and arrays on electrodes. In one aspect, one or more capture oligonucleotides having thiol groups are directly immobilized on carbon-containing electrodes, such as screen-printed carbon ink electrodes, through reaction of the thiols with the electrode, without first adding a thiol-reactive layer to the electrode.

In one aspect the electrode is treated with a plasma, for example, a low temperature plasma, such as a glow-discharge plasma, to alter the physical properties, chemical composition, or surface-chemical properties of the electrode, for example, to aid in the immobilization of reagents such as a capture oligonucleotide, or to reduce contaminants, improve adhesion to other materials, alter the wettability of the surface, facilitate deposition of materials, create patterns, or improve uniformity. Examples of useful plasmas include oxygen, nitrogen, argon, ammonia, hydrogen, fluorocarbons, water and combinations thereof. In one aspect, oxygen plasma is used to treat an electrode with carbon particles in a carbon-polymer composite material. In another aspect, oxygen is used to introduce carboxylic acids or other oxidized carbon functionality into carbon or organic materials (for example, activated esters or acyl chlorides) to facilitate coupling of reagents. In another aspect, ammonia-containing plasmas may be used to introduce amino groups for use in coupling assay reagents. In one aspect, the electrode is not pretreated to aid in the immobilization of one or more capture oligonucleotides.

In one aspect, the support surface includes an assay module such as a multi-well plate having one or more working or counter electrodes in each well. In one aspect, the multi-well plate includes a plurality of working or counter electrodes in each well. In one aspect, the working or counter electrodes of the multi-well plate include carbon, for example, screen-printed layers of carbon inks. In one aspect, one or more capture oligonucleotides are immobilized on the screen-printed carbon ink through a thiol moiety on the capture oligonucleotide. In one aspect, the working electrode is used to induce an ECL signal from an ECL label. In one aspect, the ECL signal is emitted from ruthenium-tris-bipyridine in the presence of a co-reactant such as a tertiary alkyl amine, for example, tripropyl amine or butyldiethanolamine.

In one aspect, the electrode contains binding domains as described above that are defined by dielectric ink (i.e., electrically insulating ink). The electrode is a working electrode with a dielectric printed over it in a pattern that defines the binding domains described above. In one aspect, the binding domains are roughly circular areas of exposed working electrode (or "spots"). The electrodes are in 96-well plates formed by adhering an injection molded 96-well plate top to a mylar sheet that defines the bottom of the wells. The top surface of the mylar sheet has screen printed carbon ink electrodes printed on it such that each well includes a carbon ink working electrode roughly in the center of the well and two carbon ink counter electrodes roughly towards two edges of the well. The electrodes printed on the bottom of the mylar sheet, connected through conductive through-holes to the top of the sheet, provide contacts for applying electrical voltage to the working and counter electrodes.

### J. Detection

In one aspect, the presence of one or more oligonucleotides sequences is detected or quantified based on the detection of a label immobilized on the support surface. In one aspect, the presence of one or more oligonucleotide sequences is detected or quantified based on the detection of a label on a hybridization complex immobilized on the support surface. In one aspect, the presence of one or more target oligonucleotide sequences is detected or quantified based on the detection of a label on a sense complex immobilized on the support surface. In one aspect, the presence of one or more target oligonucleotide sequences is detected or quantified based on the detection of a label on an antisense complex immobilized on the support surface. In one aspect, an oligonucleotide sequence is detected or quantified in an array.

In one aspect, the presence of the hybridization complex, for example, the antisense or sense complex is detected by monitoring light emission from a label, including, but not limited to, fluorescence, time-resolved fluorescence, fluorescence resonance energy transfer (FRET), fluorescence polarization (FP), luminescence, chemiluminescence, bioluminescence, phosphorescence, light scattering or electrode induced luminescence. In another aspect, the label includes enzymes or other chemically reactive species with a chemical activity that leads to a measurable signal such as light scattering, absorbance, fluorescence, etc. Examples of enzyme labels include, but are not limited to, horseradish peroxidase or alkaline phosphatase. In one aspect, the label is a detectable hapten, including, but not limited to, biotin, fluorescein or digoxigenin. In one aspect, the label includes biotin.

In one aspect, the hybridization complex, for example, the antisense complex or sense complex is immobilized on one or more binding domains located on the support surface. In one aspect, one or more binding domains are located on one or more electrodes and detecting or quantifying includes applying a voltage waveform to one or more electrodes to stimulate the labels on the captured reaction products to produce an electrochemical or luminescent signal. In one aspect, detecting or quantifying includes measuring an ECL signal and correlating the signal with the presence or an amount of sense or antisense oligonucleotide in a sample. In one aspect, the intensity of the emitted light is proportional to the amount of sense or antisense oligonucleotide in the sample such that the emitted light can provide a quantitative determination of the amount of sense or antisense oligonucleotide in the sample.

In one aspect, the support surface is contacted with a detection mixture after the hybridization complexes are immobilized thereon. In one aspect, the support surface is contacted with a detection mixture after the sense or antisense complexes are immobilized thereon. In one aspect, the support surface is contacted with a detection mixture after the single-strand overhang in any unproductive hybridization complexes is digested with a single-stranded nuclease. In one aspect, digestion of the single-stranded overhang in an unproductive hybridization complex is performed while the hybridization complexes are in solution (i.e., before they hybridization complexes are immobilized to the support surface via the oligonucleotide tags of the sense or antisense probes). In one aspect, digestion of the single-stranded overhang in an unproductive hybridization complex is performed after the hybridization complexes are immobilized on the support surface via the oligonucleotide tags of the sense or antisense probes. In one aspect, the detection mixture includes an ECL label. Examples of ECL labels include: i) organometallic compounds where the metal is from, for example, the noble metals of group VIII, including Ru-containing and Os-containing organometallic compounds such as the tris-bipyridyl-ruthenium (RuBpy) moiety and ii) luminol and related compounds. In one aspect, the detection mixture also includes one or more electrochemiluminescence co-reactants, and one or more additional components such as a pH buffering agent, detergent, preservative, anti-foaming agent, salt, metal ion or metal chelating agent. The term "electrochemiluminescent co-reactant" refers to species that participate with the electrochemiluminescent label to and include, but are not limited to, tertiary amines, such as tripropylamine (TPA), oxalate ion, ascorbic acid and persulfate for RuBpy and hydrogen peroxide for luminol. Methods for measuring electrochemiluminescence are known and instruments for making the measurements are commercially available. For example, multiplexed measurement of analytes using electrochemiluminescence is used in the Meso Scale Diagnostics, LLC, MULTI-ARRAY^{®} and SECTOR^{®} Imager line or products (see, e.g., U.S. Pat. Nos. 7,842,246 and 6,977,722, the disclosures of which are incorporated herein by reference in their entireties).

In one aspect, biotin is covalently attached to the hybridization complex and the detection mixture includes a streptavidin-conjugated label which binds to the immobilized hybridization complex through the avidin moiety. In one aspect, the streptavidin-conjugated label is an electrochemiluminescent (ECL) label. In one aspect, the electrochemiluminescent label is an n-hydroxysuccinimide ester, such as the Sulfo-TAG NHS-Ester (Meso Scale Diagnostics, Rockville, MD, U.S.A.).

### K. Kit

In one aspect, a kit is provided for carrying out the method described herein. "Kit" refers to a set of components that are provided or gathered to be used together, for example, to create a composition, to manufacture a device, or to carry out a method. A kit can include one or more components. The components of a kit may be provided in one package or in multiple packages, each of which can contain one or more of the components. A listed component of a kit, may in turn, also be provided as a single physical part or as multiple parts to be combined for the kit use. For example, an instrument component of a kit may be provided fully assembled or as multiple instrument parts to be assembled prior to use. Similarly, a liquid reagent component of a kit may be provided as a complete liquid formulation in a container, as one or more dry reagents and one or more liquid diluents to be combined to provide the complete liquid formulation, or as two or more liquid solutions to be combined to provide the complete liquid formulation. As is known in the art, kit components for assays are often shipped and stored separately due to having different storage needs, e.g., storage temperatures of 4°C versus -70°C.

In one aspect, the kit includes a support surface. In one aspect, the kit includes capture oligonucleotides that can be immobilized on the support surface. In one aspect, the kit includes capture oligonucleotides immobilized on a support surface. In one aspect, the kit includes capture oligonucleotides immobilized on a support surface in an array. In one aspect, the kit includes one or more capture oligonucleotides immobilized to one or more discrete binding domains with a known location within an array. In one aspect, the kit includes two or more capture oligonucleotides immobilized on a bead array.

In one aspect, the kit includes a carbon-based support surface. In one aspect, the support surface includes at least one electrode. In one aspect, the electrode is a carbon-based electrode. In one aspect, the support surface includes one or more carbon ink electrodes. In one aspect, the support surface includes at least one working electrode and at least one counter electrode.

In one aspect, the kit includes a support surface that includes a multi-well assay plate. In one aspect, one or more wells of the multi-well plate include one or more electrodes. In one aspect, the support surface includes a multi-well plate wherein one or more wells include one or more working electrodes and one or more counter electrodes. In one aspect, the support surface includes one or more reference electrodes.

In one aspect, the kit includes a standard format multi-well plate, which are known in the art and can include, but are not limited to, 24, 96, and 384 well plates. In one aspect, the kit includes one or more 96 well plates. In one aspect, the kit includes one multi-well plate. In another aspect, the kit includes 10 multi-well plates. In another aspect, the kit includes from 10 and 100 multi-well plates.

In one aspect, the kit includes a support surface having one or more electrodes on which one or more arrays of capture oligonucleotides are printed. In one aspect, the kit includes one or more multi-well plates on which one or more arrays of capture oligonucleotides have been printed. In another aspect, the kit includes one or more multi-well plates and one or more vials that include one or more capture oligonucleotides, wherein the capture oligonucleotides can be printed onto the multi-well plates.

In one aspect, the kit includes one or more capture oligonucleotides immobilized to one or more binding domains on the support surface. In one aspect, the kit includes one or more capture oligonucleotides immobilized on one or more binding domains within a well of a multi-well plate. In one aspect, the kit includes one or more capture oligonucleotides immobilized on one or more binding domains on an electrode. In one aspect, the kit includes one or more capture oligonucleotides immobilized on one or more binding domains on an electrode within one or more wells of a multi-well plate.

In another aspect, the kit includes one or more oligonucleotide tags. In one aspect, the kit includes one or more oligonucleotide tags provided in containers, wherein the oligonucleotide tags in a container have the same sequence and each container contains oligonucleotide tags having a sequence different from (and not complementary to) the sequence of the oligonucleotide tags in the other containers. In one aspect, the kit includes, in separate containers, at least about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24 or about 25 and up to about 64 unique oligonucleotide tags. In one aspect, the kit includes a set of up to 10 unique oligonucleotide tags.

In one aspect, the kit includes one or more multi-well plates in which up to 10 capture oligonucleotides are immobilized in one or more binding domains within a well of a multi-well plate, wherein each binding domain includes a capture oligonucleotide having a sequence that is different than the sequences of the capture oligonucleotides in the other binding domains within the well. In one aspect, the kit includes a support surface having at least about 1, about 2, about 3, about 4, about 5, about 6, about 7, about 8, about 9, about 10, about 15, about 20, or about 25 distinct capture oligonucleotides immobilized in at least about 1, about 2, about 3, about 4, about 5, about 6, about 7, about 8, about 9, about 10, about 15, about 20, or about 25 unique binding domains. In one aspect, the kit includes a multi-well plate having at least about 1, about 2, about 3, about 4, about 5, about 6, about 7, about 8, about 9, about 10, about 15, about 20, or about 25 distinct capture oligonucleotides immobilized in at least about 1, about 2, about 3, about 4, about 5, about 6, about 7, about 8, about 9, about 10, about 15, about 20, or about 25 unique binding domains in one or more wells. In one aspect, the kit includes one or more multi-well plates in which each well includes up to about 10 capture oligonucleotides immobilized in an array. In one aspect, the multi-well plate can be configured to create from about 1 and about 10 detection assays within each well of the multi-well plate.

In one aspect, the kit includes a single-stranded nuclease. In one aspect, the kit includes a single-stranded nuclease tags provided in a container. In one aspect, the single-strand specific nuclease includes a single-strand specific DNase. In one aspect, the single-strand specific DNase is S1 nuclease, P1 nuclease or Mung Bean nuclease. In one aspect, the single-strand specific nuclease includes a single-strand specific RNase. In one aspect, the single-strand specific RNase is RNase A, RNase H, RNase I, RNase III, RNase L, RNase P, RNase PhyM, RNase T1, RNase T2, RNase U2, RNase V, PNPase, RNase PH, RNase R, RNase D, RNase T, RNaseONE, oligoribonuclease, exoribonuclease I, or exoribonuclease II.

In one aspect, a kit is provided for conducting a luminescence assay, for example, an electrochemiluminescence assay to detect or quantify one or more target nucleotide sequences in a sample. In one aspect, the kit includes one or more assay components useful in carrying out an electrochemiluminescence assay.

In one aspect, the kit includes hybridization buffer that can be used to provide the appropriate conditions (e.g., stringent conditions) for hybridization of oligonucleotide tags to their corresponding complementary capture oligonucleotides sequences. In one aspect, the hybridization buffer includes Diluent 54 (Meso Scale Diagnostics, LLC, Rockville, MD, U.S.A.). In one aspect, the hybridization buffer includes Hybridization Buffer 1 or Hybridization Buffer 2 (Meso Scale Diagnostics, LLC, Rockville, MD, U.S.A.).

In one aspect, the kit includes one or more containers that include a label. In one aspect, the label is selected from a radioactive, fluorescent, chemiluminescent, electrochemiluminescent, light absorbing, light scattering, electrochemical, magnetic and an enzymatic label. In one aspect, the label includes an electrochemiluminescent label. In one aspect, the label includes an organometallic complex that includes a transition metal. In one aspect, the transition metal includes ruthenium. In one aspect, the label is a MSD SULFO-TAG^{™} label (Meso Scale Diagnostics, LLC, Rockville, MD, U.S.A.).

In one aspect, the label includes a primary binding reagent that is a binding partner of a secondary binding reagent. In one aspect, the secondary binding reagent includes biotin, streptavidin, avidin, or an antibody. In one aspect, the secondary binding reagent includes avidin, streptavidin or an antibody. In one aspect, the label includes a hapten selected from biotin, fluorescein and digoxigenin. In one aspect, the label is a primary binding agent that includes a first oligonucleotide sequence and the secondary binding reagent includes a second oligonucleotide sequence that is complementary to the first oligonucleotide sequence of the primary binding agent.

In one aspect, the kit includes one or more containers that include an electrochemiluminescent label. In a more particular aspect, the kit includes one or more containers containing Ru-containing or Os-containing organometallic compounds such as tris-bipyridyl-ruthenium (RuBpy). In one aspect, the label includes an organometallic complex that includes a transition metal. In one aspect, the transition metal includes ruthenium. In one aspect, the label includes the MSD SULFO-TAG^{™} label (Meso Scale Diagnostics, LLC, Rockville, MD, U.S.A.). In another aspect, the kit includes one or more containers containing luminol or other related compounds.

In one aspect, the kit includes one or more containers with one or more electrochemiluminescent co-reactants. In one aspect, one or more electrochemiluminescent co-reactants are covalently or non-covalently immobilized on the support surface. In one aspect, one or more electrochemiluminescent co-reactants are immobilized on one or more working electrodes of the support surface.

In one aspect, the label included in the kit includes a primary binding reagent and a secondary binding reagent. In one aspect, the secondary binding reagent includes biotin, streptavidin, avidin or an antibody.

In one aspect, the kit includes one or more of the following assay components: one or more capture oligonucleotides; and one or more buffers, for example, a wash buffer, a hybridization buffer, a binding buffer, or a read buffer.

In one aspect, the kit includes one or more assay components such as a label. In one aspect, the label is a luminescent label such as an electrochemiluminescent label. In one aspect, the kit includes at least one electrochemiluminescence co-reactant. In one aspect, the electrochemiluminescent co-reactant includes a tertiary amine, tripropylamine, or N-butyldiethanolamine.

In one aspect, the kit includes one or more other assay components. In one aspect, the kit includes one or more assay including, but not limited to, a diluent, blocking agents, stabilizing agents, detergents, salts, pH buffers, and preservatives. In one aspect, the kit includes containers of one or more such components. In another aspect, one or more reagents are included on the assay support surface provided with the kit.

### L. Incorporation by Reference

All references cited herein, including patents, patent applications, papers, text books and the like, and the references cited therein, to the extent that they are not already, are hereby incorporated herein by reference in their entirety for all purposes.

### WORKING EXAMPLES

### Example 1. RNase Protection Assay (RPA) Lower Limit of Detection (LLOD) using Full-length probes

The detection limits for the sense (SS) and antisense (AS) strands of a model 16-mer heteroduplex antisense oligonucleotide (ASO) (one DNA strand and one RNA strand; both are modified) were determined in the absence of the opposite strand using an RNase Protection Assay (RPA).

A chimeric full-length antisense probe containing an RNA strand with a sequence complementary to a nucleic acid-based therapeutic molecule and a DNA strand with a sequence complementary to the capture oligonucleotide was generated and included a single stranded oligonucleotide tag, a full-length (16-mer) antisense binding portion and a biotin label. A chimeric full-length sense probe was generated containing an RNA strand with a sequence complementary to a nucleic acid-based therapeutic molecule and a DNA strand with a sequence complementary to the capture oligonucleotide was generated and included a single stranded oligonucleotide tag, a full-length (16-mer) sense binding portion, and a biotin label.

Briefly, individual antisense (AS) or sense (SS) strands (10 mg/mL) were diluted to 200 µg/mL and a 10-point calibration curve was generated for the individual antisense or sense strands by spiking the ASO into Diluent 54 with a highest calibrator concentration of 40,000 pg/mL using 4-fold serial dilutions (plus 2 blank wells) as shown in Table 1.

**Table 1. Calibration Curve**

| **Calibrator** | **Concentration (pg/mL**) |
|---|---|
| Cal-1 | 40000 |
| Cal-2 | 10000 |
| Cal-3 | 2500 |
| Cal-4 | 625 |
| Cal-5 | 156 |
| Cal-6 | 39.1 |
| Cal-7 | 9.77 |
| Cal-8 | 2.44 |
| Cal-9 | 0.610 |
| Cal-10 | 0.153 |
| Blank | 0 |
| Blank | 0 |

Two concentrations of the full-length probe were used to assess the impact on positive signal and backgrounds: 50 pM or 200 pM (4X). The chimeric probes were hybridized to the antisense or sense strands in Diluent 54 (Meso Scale Diagnostics, Rockville, MD, U.S.A.) using the hybridization protocol shown in **Table 2:**

**Table 2. Hybridization Protocol**

| **Step** | **Temp.** | **Time** | |
|---|---|---|---|
| 1 | 80°C | 2 min. | |
| 2 | 65°C | 5 min. | then decrease 1°C down to 50°C, each for 5 min. |
| 3 | 37°C | Hold | 30 min @ 37°C, shaking |

A 96-well N-PLEX^{®} plate (Meso Scale Discovery, Rockville, MD, USA ("MSD")), on which single stranded capture oligonucleotides (with sequences complementary to the oligonucleotide tag sequences of the chimeric sense and antisense probes) were immobilized was blocked with N-PLEX^{™} blocking buffer (MSD) and washed 3x with a minimum of 150 µL/well Dulbecco's phosphate-buffered saline (DPBS). After the probes were hybridized to their target strands, the probe/analyte complexes were diluted in buffer, added to the plate and allowed to hybridize for 1 hour @ 37°C, while shaking. Two different buffers were used to determine impact on hybridization to the plate: Diluent 54, or a blend of NPLEX^{™} Hybridization Buffers 1 and 2 (MSD) at a 2:3 ratio.

The plates were washed again (3x with a minimum of 150 µL/well DPBS) and an RNase cocktail (RNase A, RNase I, and RNase T1) in Diluent 54 was added to the plate and incubated for 30 min @ 37°C, while shaking.

The plates were washed again and SULFO-TAG^{™} streptavidin (MSD) was added to the plate in Diluent 54 + 1% Blocker A (MSD) and incubated for 30 min @ room temperature, while shaking. The plate was washed, MSD GOLD^{™} Read Buffer A (MSD) was added and the plates were read using SECTOR^{®} S6000 or MESO^{®} SECTOR^{®} S600 imager (MSD).

The ECL signals for the antisense strand and probe are shown in **Table 3** and for the sense strand and probe are shown in **Table 4**. The probes against the antisense (DNA) strand had a higher ECL reading than the probes against the sense (RNA) strand in Diluent 54 and in the Hybridization Buffer blend. Increasing the amount of probe increased the positive and negative signal, with a signal saturation of around 10,000 pg/mL (10 ng/mL).

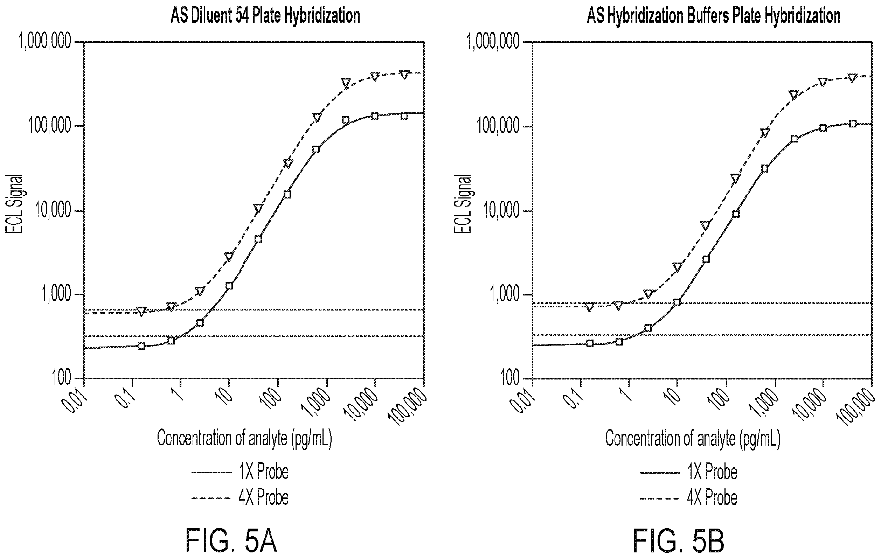

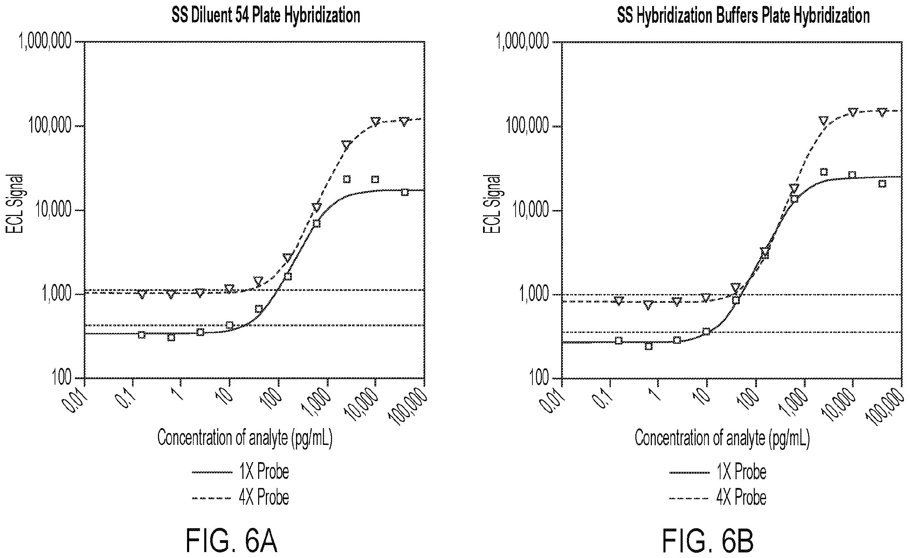

FIG. 5A and 5B show the ECL signal curves for the antisense (AS) strand Diluent 54 plate hybridization (FIG. 5A) and blended hybridization buffer plate hybridization (FIG. 5B) and for sense (SS) Diluent 54 plate hybridization (FIG. 6A) and blended hybridization buffer plate hybridization (FIG. 6B).

**Tables 5** and **Table 6** show the lower limit of detection (LLOD) for the antisense (AS) probe and strand and the sense (SS) probe and strand, respectively.

The results demonstrate that both assays give good results, although the antisense probe has a higher ECL signal and a lower LLOD than the sense probe, and the backgrounds tend to be higher with the sense probe. The results for Diluent 54 and the Hybridization Buffer blend were similar.

**Table 3. ECL Signal - antisense (AS) probe and strand**

| **Calibrator** | **Concentration (pg/mL)** | **1x probe / Diluent 54** | **1x probe / Hyb. Buffer** | **4x probe / Diluent 54** | **4x probe / Hyb. Buffer** |
|---|---|---|---|---|---|
| Cal-1 | 40000 | 131701 | 105904 | 396886 | 370824 |
| Cal-2 | 10000 | 130806 | 93823 | 384167 | 332165 |
| Cal-3 | 2500 | 118400 | 70690 | 329414 | 232325 |
| Cal-4 | 625 | 53291 | 31619 | 122887 | 82766 |
| Cal-5 | 156 | 15306 | 9263 | 35245 | 23374 |
| Cal-6 | 39.1 | 4548 | 2652 | 10220 | 6558 |
| Cal-7 | 9.77 | 1259 | 817 | 2781 | 2054 |
| Cal-8 | 2.44 | 456 | 395 | 1072 | 1006 |
| Cal-9 | 0.610 | 282 | 278 | 693 | 749 |
| Cal-10 | 0.153 | 240 | 260 | 613 | 733 |
| Blank | 0 | 247 | 261 | 600 | 720 |

**Table 4. ECL Signal - sense (SS) probe and strand**

| **Calibrator** | **Concentration (pg/mL)** | **1x probe** / **Diluent 54** | **1x probe / Hvb. Buffer** | **4x probe / Diluent 54** | **4x probe / Hvb. Buffer** |
|---|---|---|---|---|---|
| Cal-1 | 40000 | 16178 | 20896 | 111291 | 143937 |
| Cal-2 | 10000 | 23008 | 26874 | 108890 | 145697 |
| Cal-3 | 2500 | 23328 | 28933 | 57900 | 114503 |
| Cal-4 | 625 | 6981 | 14050 | 10419 | 17989 |
| Cal-5 | 156 | 1651 | 3011 | 2664 | 3127 |
| Cal-6 | 39.1 | 661 | 866 | 1405 | 1199 |
| Cal-7 | 9.77 | 425 | 371 | 1137 | 903 |
| Cal-8 | 2.44 | 358 | 294 | 1010 | 811 |
| Cal-9 | 0.610 | 306 | 247 | 989 | 750 |
| Cal-10 | 0.153 | 329 | 288 | 955 | 838 |
| Blank | 0 | 362 | 297 | 1015 | 813 |

**Table 5. Antisense (AS) probe and strand lower limit of detection (LLOD)**

| | **1x Probe** / **Diluent 54** | **1x Probe** / **Hvb Buffers** | **4x Probe** / **Diluent 54** | **4x Probe** / **Hyb Buffers** |
|---|---|---|---|---|
| Hill Slope | 1.06 | 1.04 | 1.06 | 1.06 |
| 2 r value | 0.990 | 1.00 | 0.989 | 0.998 |
| LLODₑₛₜ (pg/mL) | 0.88 | 1.41 | 0.42 | 0.65 |

**Table 6. Sense (SS) probe and strand lower limit of detection (LLOD)**

| | **1x Probe / Diluent 54** | **1x Probe / Hyb Buffers** | **4x Probe / Diluent 54** | **4x Probe / Hyb Buffers** |
|---|---|---|---|---|
| Hill Slope | 1.40 | 1.42 | 1.46 | 1.67 |
| 2 r value | 0.921 | 0.940 | 0.993 | 0.984 |
| LLODₑₛₜ (pg/mL) | 17.9 | 10.3 | 19.6 | 26.6 |

### Example 2. Hybridization of shortened probes

The ability of shortened probes (12-mer, 11-mer, and 10-mer) to hybridize to the sense (SS) and antisense (AS) strand of the model 16 nucleotide heteroduplex antisense oligonucleotide (ASO) from Example 1 in the absence of the opposite strand was evaluated in an RNase protection assay (RPA). The probes were shortened on the 3' end.

Briefly, 10-point calibration curves (plus 2 blank wells) were generated using individual antisense (AS) or sense (SS) strands from the ASO with a 4-fold serial dilution and a highest calibrator of 40,000 pg/mL.

Four different probe lengths were assessed for detection following the method described in Example 1, above: Full-length (FL), 12-mer, 11-mer, and 10-mer probes using a 200 pM probe concentration. The probes were hybridized to the antisense or sense strand in Diluent 54 and hybridized to the plate in either Diluent 54 or Hybridization Buffer 1. The calibration curve was split between Diluent 54 or Hybridization Buffer 1 in singlicate, rather than in duplicate wells.

Four chimeric antisense (AS) probes were generated that included a single stranded oligonucleotide tag, an antisense binding portion (full-length 16-mer (FL), 12-mer, 11-mer or 10-mer), and a biotin label.

Four chimeric sense (SS) probes were generated that included a single stranded oligonucleotide tag, a sense binding portion (full length 16-mer (FL), 12-mer, 11-mer or 10-mer), and a biotin label.

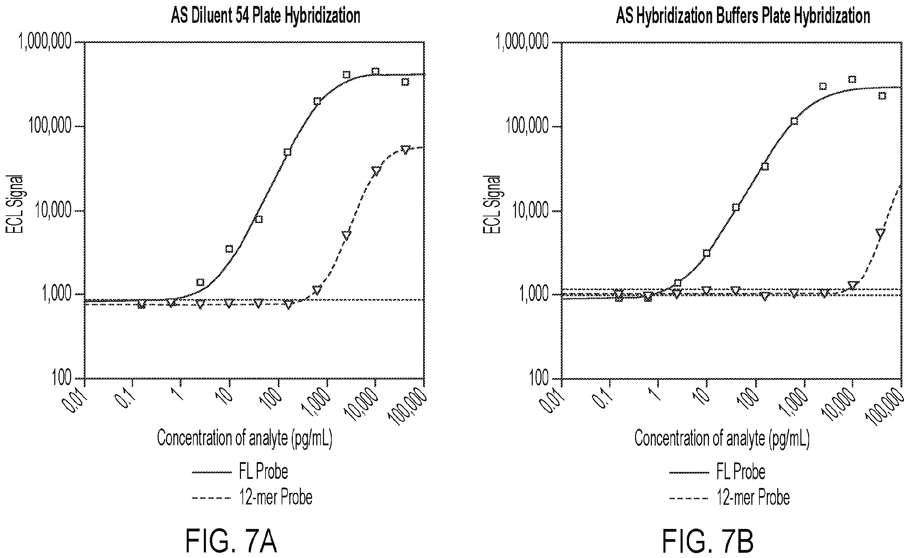

**Table 7** and **Table 8** show the ECL signals for the antisense probe and strand hybridized in Diluent 54 or in Hybridization Buffers, respectively. **Table 9** and **Table 10** show the ECL signals for the sense probe and strand hybridized in Diluent 54 or in Hybridization Buffers, respectively. The data from **Tables 7-10** is shown graphically in FIG. 7A-7B.

**Table 11** show the lower limit of detection (LLOD) for the antisense (AS) probe and strand for the 16-mer (FL) probe and 12-mer probe in Diluent 54 and Hybridization Buffers. **Table 12** and **Table 13** show the LLOD for the sense (SS) probe and strand for the 16-mer (FL), 12-mer, 11-mer and 10-mer probes in Diluent 54 and Hybridization Buffers, respectively.

The data indicate that hybridization with Hybridization Buffers results in a loss of signal in the 12-mer probe for the antisense (AS) strand as compared with Diluent 54. For the antisense strand, shortening the probes has a negative impact on ECL signal and LLODₑₛₜ. For the sense strand, shortening the probes has minimal impact on ECL signal and LLODₑₛₜ. The type of hybridization buffers did not have an impact on the assays.

**Table 7: Diluent 54 Hybridization - Antisense (AS) probe and strand**

| **Calibrator** | **Concentration (pg/mL**) | **FL probe** | **12-mer probe** | **11-mer probe** | **10-mer probe** |
|---|---|---|---|---|---|
| Cal-1 | 40000 | 345329 | 54562 | 1016 | 844 |
| Cal-2 | 10000 | 477705 | 29524 | 905 | 764 |
| Cal-3 | 2500 | 414430 | 5049 | 870 | 809 |
| Cal-4 | 625 | 203586 | 1103 | 861 | 770 |
| Cal-5 | 156 | 50989 | 749 | 866 | 781 |
| Cal-6 | 39.1 | 8054 | 787 | 904 | 775 |
| Cal-7 | 9.77 | 3574 | 786 | 864 | 822 |
| Cal-8 | 2.44 | 1411 | 757 | 887 | 798 |
| Cal-9 | 0.610 | 827 | 777 | 915 | 869 |
| Cal-10 | 0.153 | 755 | 759 | 882 | 811 |
| Blank | 0 | 705 | 784 | 862 | 894 |

**Table 8: Hybridization Buffers- Antisense (AS) probe and strand**

| **Calibrator** | **Concentration (pg/mL**) | **FL probe** | **12-mer probe** | **11-mer probe** | **10-mer probe** |
|---|---|---|---|---|---|
| Cal-1 | 40000 | 230756 | 5368 | 1164 | 1031 |
| Cal-2 | 10000 | 360035 | 1206 | 1114 | 764 |
| Cal-3 | 2500 | 297142 | 1004 | 1112 | 997 |
| Cal-4 | 625 | 115269 | 1018 | 902 | 921 |
| Cal-5 | 156 | 33152 | 908 | 1128 | 1031 |
| Cal-6 | 39.1 | 11240 | 1058 | 1214 | 986 |
| Cal-7 | 9.77 | 3077 | 1063 | 1144 | 1014 |
| Cal-8 | 2.44 | 1365 | 994 | 1087 | 848 |
| Cal-9 | 0.610 | 906 | 927 | 1125 | 987 |
| Cal-10 | 0.153 | 915 | 980 | 1073 | 965 |
| Blank | 0 | 976 | 1131 | 1164 | 1080 |

**Table 9: Diluent 54 Hybridization - Sense (SS) probe and strand**

| **Calibrator** | **Concentration (pg/mL)** | **FL probe** | **12-mer probe** | **11-mer probe** | **10-mer probe** |
|---|---|---|---|---|---|
| Cal-1 | 40000 | 104668 | 96844 | 79642 | 128029 |
| Cal-2 | 10000 | 98109 | 103383 | 82916 | 133309 |
| Cal-3 | 2500 | 76763 | 58215 | 47668 | 59987 |
| Cal-4 | 625 | 15942 | 14514 | 11904 | 14247 |
| Cal-5 | 156 | 3513 | 4357 | 4374 | 4180 |
| Cal-6 | 39.1 | 1440 | 2283 | 2474 | 1472 |
| Cal-7 | 9.77 | 1150 | 1782 | 2305 | 996 |
| Cal-8 | 2.44 | 1083 | 1787 | 2044 | 994 |
| Cal-9 | 0.610 | 1029 | 1563 | 2078 | 893 |
| Cal-10 | 0.153 | 1025 | 1567 | 2066 | 926 |
| Blank | 0 | 1043 | 1643 | 2105 | 911 |

**Table 10: Hybridization Buffers- Sense (SS) probe and strand**

| **Calibrator** | **Concentration (pg/mL)** | **FL probe** | **12-mer probe** | **11-mer probe** | **10-mer probe** |
|---|---|---|---|---|---|
| Cal-1 | 40000 | 138021 | 229349 | 129664 | 88682 |
| Cal-2 | 10000 | 120157 | 188366 | 89150 | 30794 |
| Cal-3 | 2500 | 137402 | 113197 | 52769 | 16766 |
| Cal-4 | 625 | 26606 | 29127 | 12014 | 2849 |
| Cal-5 | 156 | 4034 | 7469 | 3986 | 1169 |
| Cal-6 | 39.1 | 1386 | 2670 | 2540 | 1031 |
| Cal-7 | 9.77 | 1030 | 1863 | 1786 | 787 |
| Cal-8 | 2.44 | 969 | 1603 | 1556 | 685 |
| Cal-9 | 0.610 | 779 | 1285 | 1388 | 754 |
| Cal-10 | 0.153 | 897 | 1615 | 1715 | 817 |
| Blank | 0 | 921 | 1545 | 1851 | 789 |

**Table 11. Antisense probe and strand**

| | **Diluent 54** | | **Hybridization Buffer** | |
|---|---|---|---|---|
| | **FL probe** | **12-mer probe** | **FL probe** | **12-mer probe** |
| Hill Slope | 1.25 | 1.86 | 1.092 | 2.83 |
| r² value | 0.957 | 1.00 | 0.923 | 0.998 |
| LLODₑₛₜ (pg/mL) | 1.07 | 273.8 | 0.47 | 9077 |

**Table 12. Sense probe and strand (Diluent 54)**

| | **FL probe** | **12-mer probe** | **11-mer probe** | **10-mer probe** |
|---|---|---|---|---|
| Hill Slope | 1.51 | 1.32 | 1.36 | 1.24 |
| r² value | 0.991 | 0.987 | 0.991 | 0.982 |
| LLODₑₛₜ (pg/mL) | 23.1 | 23.6 | 32.5 | 8.02 |

**Table 13. Sense probe and strand (Hybridization Buffers)**

| | **FL probe** | **12-mer probe** | **11-mer probe** | **10-mer probe** |
|---|---|---|---|---|
| Hill Slope | 1.70 | 1.22 | 1.16 | 1.09 |
| r² value | 0.960 | 0.998 | 0.993 | 0.989 |
| LLODₑₛₜ (pg/mL) | 19.2 | 11.8 | 18.3 | 31.7 |

### Example 3. Heteroduplex detection of antisense and sense strands

The detection limits of the antisense (AS) and sense (SS) strands of the model ASO from Example 1 were determined for an RNase Protection Assay (RPA), using the full-length (16-mer) or 12-mer shortened chimeric probes from Example 2.

A 10-point calibration curve was generated using individual AS, SS, or heteroduplex strands with a highest calibrator concentration of 40,000 pg/mL using 4-fold serial dilutions (plus 2 blank wells).

Two different probe lengths were assessed: a full-length 16-mer (FL) and 12-mer at a concentration of 200 pM.

The probes were hybridized to the antisense or sense strands in Diluent 54 and added to a 96-well plate in Diluent 54 in duplicate wells, essentially as described in Example 2.

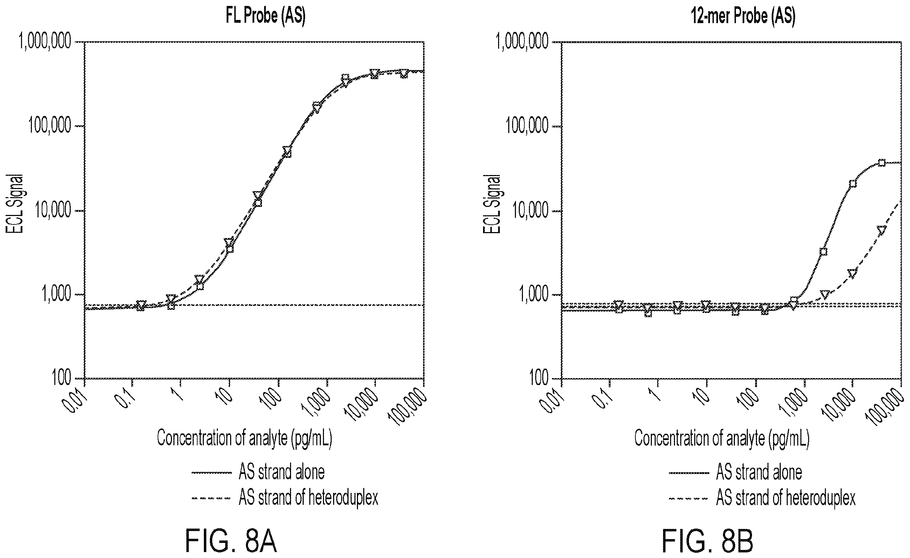

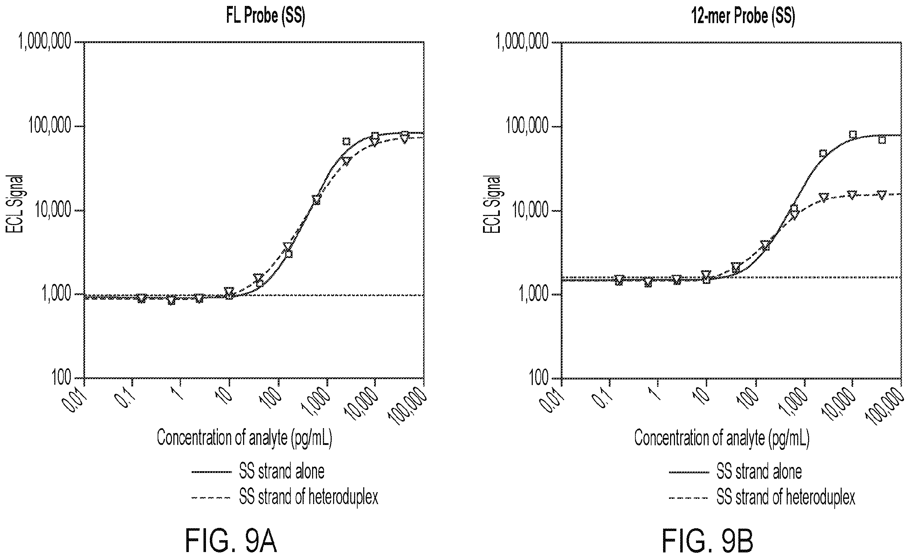

The ECL signals for the antisense strand detection and the sense strand detection are shown in **Table 14** and **Table 15,** respectively. The data from **Table 14** is shown graphically in FIG. 8A-8B and the data from **Table 15** is shown graphically in FIG. 9A-9B.

**Table 16** shows the lower limit of detection (LLOD) for the antisense (AS) strand using the 16-mer (FL) probe and 12-mer probe alone or in a heteroduplex. **Table 17** shows the LLOD for the sense (SS) strand using the 16-mer (FL) probe and 12-mer probe alone or in a heteroduplex.

The results show that the 12-mer probe decreased the ECL signal in the antisense strand, but not in the sense strand. The full-length probe had similar ECL signals for both strands, whether from individual strands or in a heteroduplex. The 12-mer probes decreased ECL signals for both the antisense and sense strands in the heteroduplex. The heteroduplex did not impact the ECL signal or LLODₑₛₜ when using full-length probes for both the antisense and sense strands. The 12-mer probes decreased ECL signal and increased LLODₑₛₜ for both the antisense and sense strands in the heteroduplex.

**Table 14. Antisense detection**

| | | AS Strand Alone | | Heteroduplex | |
|---|---|---|---|---|---|
| **Calibrator** | **Concentration (pg/mL)** | **FL Probe** | **12-mer Probe** | **FL Probe** | **12-mer Probe** |
| Cal-1 | 40000 | 436522 | 36684 | 430618 | 5626 |
| Cal-2 | 10000 | 413134 | 21066 | 421128 | 1716 |
| Cal-3 | 2500 | 385432 | 3213 | 327599 | 980 |
| Cal-4 | 625 | 180946 | 826 | 161471 | 726 |
| Cal-5 | 156 | 48504 | 630 | 51140 | 668 |
| Cal-6 | 39.1 | 12506 | 628 | 14699 | 689 |
| Cal-7 | 9.77 | 3533 | 670 | 4164 | 738 |
| Cal-8 | 2.44 | 1271 | 646 | 1476 | 728 |
| Cal-9 | 0.610 | 741 | 607 | 884 | 673 |
| Cal-10 | 0.153 | 731 | 665 | 748 | 750 |
| Blank | 0 | 731 | 697 | 678 | 693 |

**Table 15. Sense detection**

| | | AS Strand Alone | | Heteroduplex | |
|---|---|---|---|---|---|
| **Calibrator** | **Concentration (pg/mL**) | **FL Probe** | **12-mer Probe** | **FL Probe** | **12-mer Probe** |
| Cal-1 | 40000 | 79174 | 68893 | 69891 | 14864 |
| Cal-2 | 10000 | 75868 | 80815 | 62486 | 15216 |
| Cal-3 | 2500 | 65654 | 47818 | 37580 | 13855 |
| Cal-4 | 625 | 13041 | 10549 | 13124 | 8512 |
| Cal-5 | 156 | 3031 | 3727 | 3620 | 3839 |
| Cal-6 | 39.1 | 1359 | 2001 | 1543 | 2153 |
| Cal-7 | 9.77 | 962 | 1546 | 1056 | 1698 |
| Cal-8 | 2.44 | 877 | 1471 | 894 | 1497 |
| Cal-9 | 0.610 | 830 | 1400 | 834 | 1408 |
| Cal-10 | 0.153 | 882 | 1413 | 888 | 1483 |
| Blank | 0 | 906 | 1508 | 914 | 1471 |

**Table 16: Antisense strand detection alone or in heteroduplex**

| | **AS Strand alone** | | **Heteroduplex** | |
|---|---|---|---|---|
| | **FL probe** | **12-mer probe** | **FL Probe** | **12-mer probe** |
| Hill Slope | 1.12 | 2.00 | 1.03 | 1.11 |
| r² value | 0.993 | 1.000 | 0.999 | 0.999 |
| LLODₑₛₜ (pg/mL) | 0.42 | 411 | 0.26 | 945 |

**Table 17: Sense strand detection alone or in heteroduplex**

| | **AS Strand alone** | | **Heteroduplex** | |
|---|---|---|---|---|
| | **FL probe** | **12-mer probe** | **FL Probe** | **12-mer probe** |
| Hill Slope | 1.46 | 1.34 | 1.14 | 1.11 |
| r² value | 0.978 | 0.973 | 1.000 | 0.997 |
| LLODₑₛₜ (pg/mL) | 12.1 | 23.4 | 6.99 | 12.8 |

### Example 4. Lower Limit of Detection (LLOD) using Full-length (16-mer) probes

The detection limits for the individual antisense (AS) or sense (SS) strands or the full model heteroduplex ASO from Example 1 were determined for the RNase Protection Assay (RPA) from Example 1 using 16-mer full-length (FL) probes.

Briefly, a 10-point calibration curve was generated from individual antisense, sense or heteroduplex strands with a highest calibrator concentration of 40,000 pg/mL using 4-fold serial dilutions (plus 2 blank wells). Individual strands have 2 wells were used for each calibrator or individual strands and 4 wells were used for each calibrator for the heteroduplex with 24 individual blank wells for the full LLOD determination. Two probe lengths were assessed: a full-length 16-mer (FL) and a 12-mer at a concentration of 200 pM. The probes were hybridized to the antisense (AS) or sense (SS) strands and hybridized to the plate in Diluent 54.

As shown in **Table 18**, the ECL signals are similar between the individual antisense or sense strands and the heteroduplex. The results shown in **Table 19** indicate that the LLOD of the singleplex assay for the antisense strand was below 0.5 pg/mL for both the individual strand and the heteroduplex - the LLOD of the singleplex assay for the sense strand was 25 pg/mL and 14 pg/mL for the individual strand and the heteroduplex, respectively. The coefficients of variation (CV) for Cal-3 were all below 15%, with CVs for the antisense strand much lower than those of the sense strand.

**Table 18: ECL Signals - 16-mer Full-Length (FL) antisense or sense probes**

| | | **FL Antisense Probe** | | **FL Sense Probe** | |
|---|---|---|---|---|---|
| **Calibrator** | **Concentration (pg/mL**) | **Antisense strand** | **Heteroduplex** | **Sense strand** | **Heteroduplex** |
| Cal-1 | 40000 | 411846 | 381867 | 100429 | 92338 |
| Cal-2 | 10000 | 402206 | 345787 | 114521 | 74490 |
| Cal-3 | 2500 | 362468 | 255147 | 89301 | 40525 |
| Cal-4 | 625 | 173784 | 120117 | 17994 | 10785 |
| Cal-5 | 156 | 56156 | 41165 | 4384 | 3590 |
| Cal-6 | 39.1 | 15325 | 11862 | 1264 | 1637 |
| Cal-7 | 9.77 | 4263 | 3357 | 1227 | 1142 |
| Cal-8 | 2.44 | 1546 | 1163 | 1060 | 969 |
| Cal-9 | 0.610 | 835 | 784 | 1016 | 881 |
| Cal-10 | 0.153 | 696 | 650 | 928 | 939 |
| Blank | 0 | 616 | 605 | 979 | 960 |

**Table 19: LLOD determination for individual strands or heteroduplex**

| | **FL AS Probe** | | **FL SS Probe** | |
|---|---|---|---|---|
| | AS Individual | Heteroduplex | SS Individual | Heteroduplex |
| Hill Slope | 1.04 | 1.02 | 1.53 | 1.08 |
| r² value | 0.994 | 1.000 | 0.977 | 0.978 |
| LLOD (pg/mL) | 0.37 | 0.47 | 25.3 | 14.3 |
| Blank CV (%) | 7.71 | 8.07 | 7.11 | 8.40 |
| Cal-3 CV (%) | 1.71 | 5.75 | 11.3 | 12.0 |

### Example 5. Multiplex detection of a heteroduplex

This experiment was designed to determine whether antisense (AS) and sense (SS) strands of the model heteroduplex antisense oligonucleotide (ASO) from Example 1 can be detected using the RNase Protection Assay (RPA) from Example 1 in a multiplex format.

Briefly, a 10-point calibration curves were generated from individual antisense (AS), sense (SS) or heteroduplex strands with a highest calibrator concentration of 40,000 pg/mL using 4-fold serial dilutions (plus 2 blank wells). Wells with only probe were included to determine whether probe-probe interactions would occur with the antisense and sense probes and whether probe-probe interactions could be eliminated with the shortened 12-mer probes.

Two probe lengths were assessed: Full-length 16- mer (FL) and a 12-mer (probes sequences are shown in Example 2), at a concentration of 200 pM.

The probes were hybridized to the antisense or sense strands and to a 96-well plate in Diluent 54 in duplicate wells.

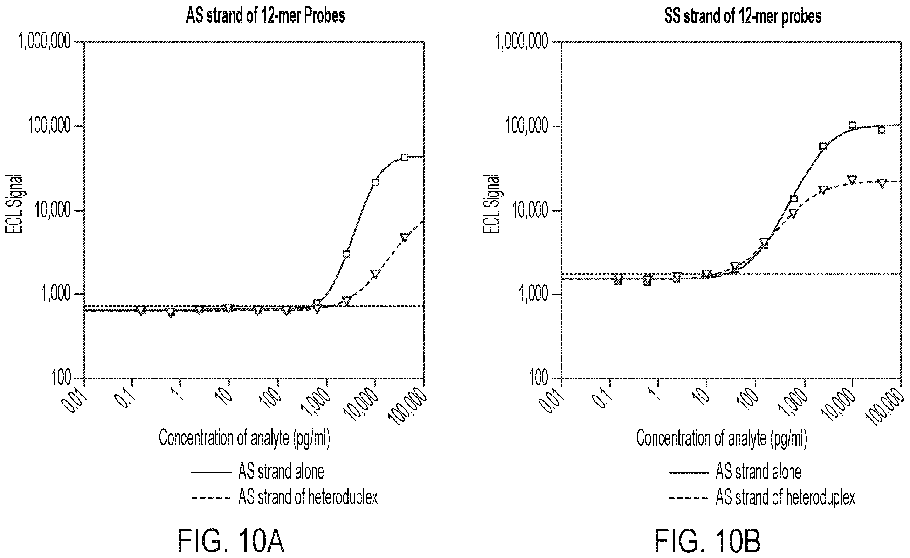

The ECL signals for the antisense strand detection using the 16-mer (FL) and 12-mer probes are shown in **Table 20** and **Table 21,** respectively. The ECL signals for the sense strand detection using the 16-mer (FL) and 12-mer probes are shown in **Table 22** and **Table 23,** respectively. The ECL data is shown graphically in FIG. 10A and 10B. **Table 24** shows the lower limit of detection (LLOD) for multiplexed detection using the 12-mer probe for the antisense (AS) and sense (SS) strands alone or in a heteroduplex.

The results show that the antisense and sense probes cross-react when full-length probes were used in multiplex assay, resulting in highly elevated background signals. At high concentrations, there is an additive effect for the antisense strand (individual or heteroduplex), while there is an inverse correlation with the sense strand on the antisense spot signal and vice versa (likely due to loss of a probe-probe interactions from target binding to probe).

Use of the 12-mer probes in singleplex (Example 2) or multiplex (current Example) resulted in similar ECL levels, indicating no deleterious impact of multiplexing the probes.

**Table 20: ECL Signals - Antisense**

| | | **FL Probe** | | | |
|---|---|---|---|---|---|
| **Calibrator** | **Conc. (pg/mL)** | **Antisense Strand** | **Sense Strand** | **Heteroduplex** | **Blank** |
| Cal-1 | 40000 | 445564 | 38268 | 428453 | 253738 |
| Cal-2 | 10000 | 417321 | 106092 | 417266 | 265647 |
| Cal-3 | 2500 | 410957 | 188466 | 367584 | 255043 |
| Cal-4 | 625 | 319217 | 235504 | 301552 | 255697 |
| Cal-5 | 156 | 264762 | 250846 | 259345 | 238399 |
| Cal-6 | 39.1 | 267705 | 258659 | 253933 | 247750 |
| Cal-7 | 9.77 | 264975 | 259514 | 254619 | 246520 |
| Cal-8 | 2.44 | 259803 | 260489 | 250511 | 256856 |
| Cal-9 | 0.610 | 249089 | 247304 | 231383 | 240383 |
| Cal-10 | 0.153 | 266104 | 262336 | 253636 | 252661 |
| Blank | 0 | 269205 | 269493 | 260503 | 263732 |

**Table 21: ECL Signals - Antisense**

| | | **12-mer Probe** | | | |
|---|---|---|---|---|---|
| **Calibrator** | **Conc. (pg/mL)** | **Antisense Strand** | **Sense Strand** | **Heteroduplex** | **Blank** |
| Cal-1 | 40000 | 43389 | 674 | 4775 | 624 |
| Cal-2 | 10000 | 21803 | 716 | 1736 | 627 |
| Cal-3 | 2500 | 3088 | 660 | 867 | 642 |
| Cal-4 | 625 | 806 | 662 | 686 | 621 |
| Cal-5 | 156 | 670 | 619 | 663 | 623 |
| Cal-6 | 39.1 | 667 | 641 | 646 | 618 |
| Cal-7 | 9.77 | 683 | 666 | 697 | 679 |
| Cal-8 | 2.44 | 673 | 664 | 675 | 647 |
| Cal-9 | 0.610 | 609 | 625 | 622 | 630 |
| Cal-10 | 0.153 | 675 | 654 | 634 | 652 |
| Blank | 0 | 703 | 684 | 664 | 685 |

**Table 22: ECL Signals - sense**

| | | **FL Probe** | | | |
|---|---|---|---|---|---|
| **Calibrator** | **Conc. (pg/mL)** | **Antisense Strand** | **Sense Strand** | **Heteroduplex** | **Blank** |
| Cal-1 | 40000 | 70169 | 138232 | 126624 | 265355 |
| Cal-2 | 10000 | 124831 | 193101 | 166476 | 273025 |
| Cal-3 | 2500 | 211483 | 251805 | 214333 | 266967 |
| Cal-4 | 625 | 250605 | 246399 | 245508 | 260377 |
| Cal-5 | 156 | 263716 | 266637 | 252830 | 246284 |
| Cal-6 | 39.1 | 278373 | 274500 | 263248 | 259176 |
| Cal-7 | 9.77 | 290189 | 282544 | 266020 | 262873 |
| Cal-8 | 2.44 | 275351 | 263998 | 263776 | 256705 |
| Cal-9 | 0.610 | 275655 | 255402 | 244191 | 242755 |
| Cal-10 | 0.153 | 283794 | 277184 | 257911 | 267708 |
| Blank | 0 | 288126 | 282058 | 276128 | 285388 |

**Table 23: ECL Signals - sense**

| | | **12-mer Probe** | | | |
|---|---|---|---|---|---|
| **Calibrator** | **Conc. (pg/mL**) | **Antisense Strand** | **Sense Strand** | **Heteroduplex** | **Blank** |
| Cal-1 | 40000 | 1485 | 93077 | 21051 | 1373 |
| Cal-2 | 10000 | 1467 | 104926 | 22860 | 1800 |
| Cal-3 | 2500 | 1570 | 58004 | 17875 | 1516 |
| Cal-4 | 625 | 1491 | 13992 | 9372 | 1484 |
| Cal-5 | 156 | 1593 | 4042 | 4201 | 1457 |
| Cal-6 | 39.1 | 1583 | 2252 | 2185 | 2009 |
| Cal-7 | 9.77 | 1641 | 1751 | 1733 | 1589 |
| Cal-8 | 2.44 | 1584 | 1599 | 1668 | 1486 |
| Cal-9 | 0.610 | 1504 | 1479 | 1517 | 1435 |
| Cal-10 | 0.153 | 1544 | 1496 | 1551 | 1527 |
| Blank | 0 | 1630 | 1539 | 1608 | 1567 |

**Table 24: Multiplexed Detection LLOD with 12-mer probes**

| | **Antisense Spot** | | **Sense Spot** | |
|---|---|---|---|---|
| | **Antisense Individual** | **Heteroduplex** | **Sense Individual** | **Heteroduplex** |
| Hill Slope | 1.99 | 1.25 | 1.34 | 1.10 |
| r² value | 1.000 | 1.000 | 0.981 | 0.994 |
| LLODₑₛₜ (pg/mL) | 429 | 1083 | 27.0 | 7.71 |

### Example 6. Effect of hybridization time on detection of Heteroduplex

The effect of a reduced hybridization time on the detection limit of the antisense (AS) and sense (SS) strands of the model ASO from Example 1 was determined.

Briefly, a 10-point calibration curve was generated using the heteroduplex with a highest calibrator concentration of 40,000 pg/mL using 4-fold serial dilutions plus 2 blank wells). Two probe lengths were assess: a full-length 16-mer (FL) and a 12-mer at a concentration of 200 pM. The probes were hybridized to the antisense or sense strands in Diluent 54 using a "long hybridization" protocol (**Table 25**) and a "short hybridization" protocol (**Table 26**). The probes were hybridized in duplicate wells of a 96-well plate in Diluent 54.

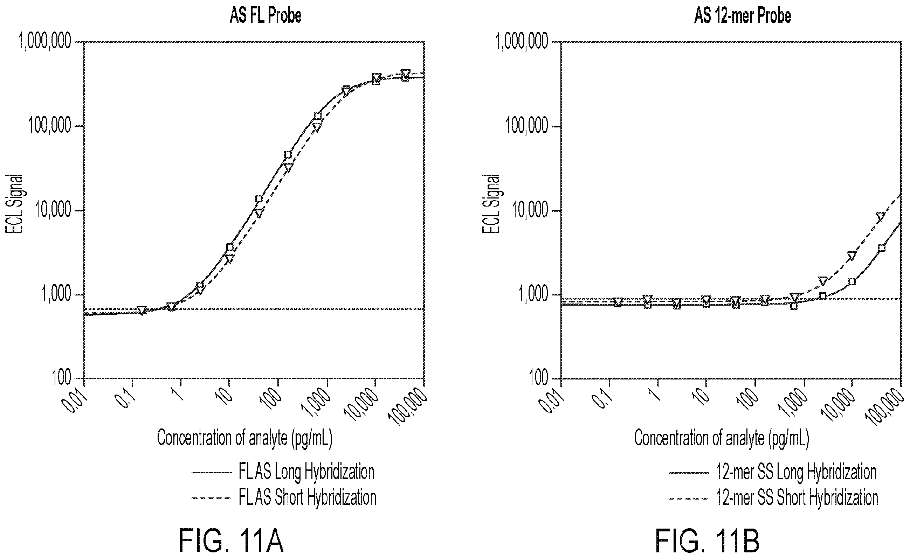

The full-length (FL) probes had better signals than the 12-mer probes for detection of both the antisense (AS) and sense (SS) strands in the heteroduplex. The results are shown graphically in FIG. 11A and FIG. 11B. The change in hybridization time did not have a large impact on ECL signal generation.

The sensitivities of detection for the ASO or SO strands of the heteroduplex are not impacted by reducing the amount of time for hybridizing the probe to its corresponding strand. In order to shorten the assay time, we recommend performing the probe/strand hybridization under the shortened hybridization conditions.

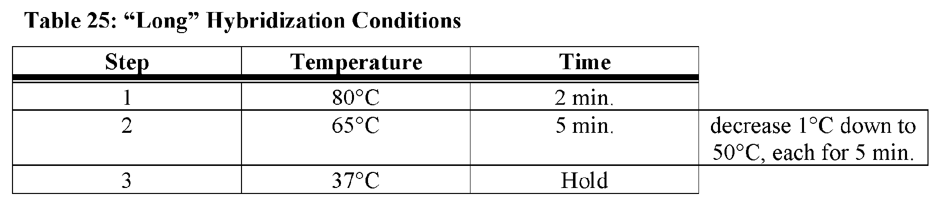

**Table 26: "Short" Hybridization Conditions**

| **Step** | **Temperature** | **Time** |
|---|---|---|
| 1 | 95°C | 2 min. |
| 2 | 65°C | 1 min. (ramp down 50%) |
| 3 | 4°C | Hold (ramp down 3%) |

### Example 7. Detection of antisense and sense strands in plasma

The detection limits for the antisense (AS) or sense (SS) strands of the model ASO from Example 1 were determined when spiked in mouse plasma.

Briefly, a 10-point calibration curve was generated using individual antisense or sense strands or the heteroduplex with a highest calibrator concentration of 40,000 pg/mL using 4-fold serial dilutions (plus 2 blank wells). The mouse plasma and diluent calibration curve were both pretreated with RNAsecure^{™} RNase inactivation reagent at 60°C for 10 min with a 4°C hold.

RNAsecure^{™} reagent was added in Diluent 54 at 1:1 ratio (20 µL calibrator or blank : 20 µL RNAsecure^{™} reagent). Full-length 16-mer (FL) probes were used at a final concentration of 200 pM. 5x probe was added in a 10 µL volume prepared with Diluent 54 for a 50 µL total volume. The probe was hybridized to the antisense or sense strands in Diluent 54 or in plasma in duplicate wells using the shortened hybridization protocol shown in **Table 26**.

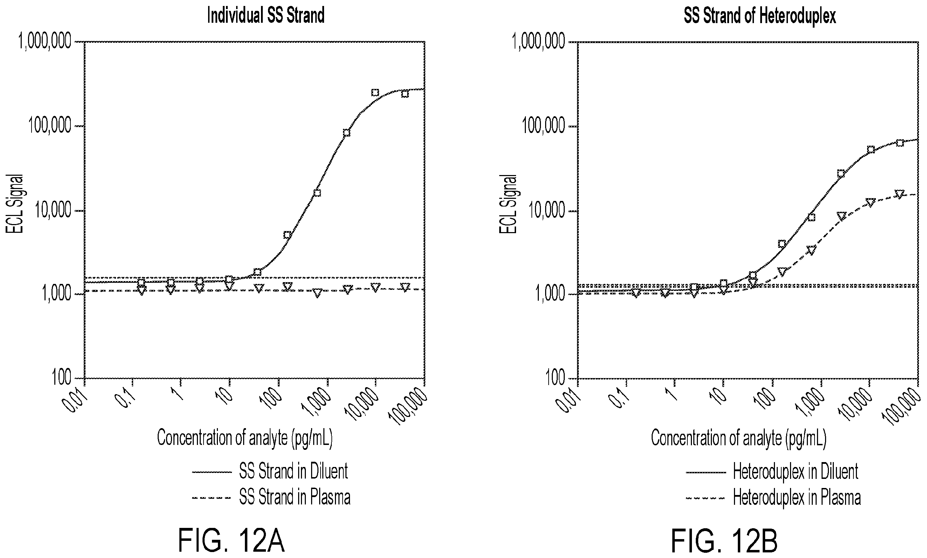

The data (not shown) demonstrate that ECL signals generated from antisense strand detection do not change much with plasma as the matrix, either with the individual strand or as part of the heteroduplex. As shown in FIG. 12A and 12B, the ECL signals were lost when the individual sense strand (RNA) was added to mouse plasma and were reduced when the sense strand of the heteroduplex was added to mouse plasma. This is likely due to endogenous RNases in the plasma that degrade the ssRNA. **Table 27** shows the lower limit of detection (LLOD) for the antisense strand in Diluent 54 or plasma. **Table 28** shows the lower limit of detection (LLOD) for the sense strand in Diluent 54 or plasma.

The results demonstrate that the ability to detect the antisense strand (DNA) at high sensitivity was not altered when measured in plasma. For antisense (DNA) strand, both the individual strand and the antisense strand of the heteroduplex have similar LLODₑₛₜ in diluent and plasma. However, the ability to detect the sense strand (RNA) was altered when measured in plasma: the individual sense strand was undetectable; and the sense strand of the heteroduplex was detectable, but at a reduced sensitivity and with a reduced dynamic range.

**Table 27: Antisense strand lower limit of detection (LLOD)**

| | **Antisense strand alone** | | **Heteroduplex** | |
|---|---|---|---|---|
| | **Diluent 54** | **Plasma** | **Diluent 54** | **Plasma** |
| Hill Slope | 1.02 | 0.99 | 1.01 | 0.95 |
| r² value | 0.996 | 0.997 | 1.000 | 1.000 |
| LLODₑₛₜ (pg/mL) | 0.40 | 0.53 | 0.44 | 0.19 |

**Table 28: Sense strand detection lower limit of detection (LLOD)**

| | **Antisense strand alone** | | **Heteroduplex** | |
|---|---|---|---|---|
| | **Diluent 54** | **Plasma** | **Diluent 54** | **Plasma** |
| Hill Slope | 1.32 | N/A | 0.96 | 0.93 |
| r² value | 0.975 | N/A | 0.994 | 0.997 |
| LLODₑₛₜ (pg/mL) | 19.9 | N/A | 9.92 | 37.1 |

### Example 8. Singleplex detection of antisense and sense strands in mouse plasma

The detection limits for the antisense and sense strands or the full model heteroduplex ASO from Example 1 were determined using the RNase Protection Assay (RPA) essentially described in Example 1 (with the modifications provided below), using full-length (16-mer) probes in mouse plasma.

Briefly, a 10-point calibration curve was generated using the heteroduplex strands with a highest calibrator concentration of 40,000 pg/mL using 4-fold serial dilutions (plus 2 blank wells) in mouse plasma. 4 wells were used for each calibrator with 24 blank wells for a full lower limit of detection (LLOD) determination. The calibrator samples and blanks were pretreated with RNAsecure^{™} as described in Example 7.

The probes were hybridized to the antisense or sense strands with the shortened hybridization protocol described in Example 6, with one modification: the denaturation temperature was reduced to 80°C because heating the plasma to 95°C for denaturation renders the plasma too viscous to pipette easily. The revised protocol is shown in **Table 29**.

**Table 29: Hybridization Conditions**

| **Step** | **Temperature** | **Time** |
|---|---|---|
| 1 | 80°C | 2 min. |
| 2 | 65°C | 1 min. (ramp down 50%) |
| 3 | 4°C | Hold (ramp down 3%) |

The results (not shown) indicate that the ECL signals are consistent with the plasma experiment in Example 7. The top of curve (TOC) signal for the antisense strand (DNA) is much higher compared to the sense strand (RNA). The LLOD for the antisense strand (DNA) of the heteroduplex was about 1 pg/mL and the LLOD for the sense (RNA) strand was about 31 pg/mL in the singleplex assay. The CVs for Cal-3 and blanks were all below 10%, showing low signal variability.

### Example 9. Impact of Blocker S1 on Background

The effect of adding Blocker S1 to the N-PLEX^{™} Blocking Buffer was evaluated for the impact on backgrounds associated with the antisense (AS) and sense (SS) singleplex assays.

Briefly, a 10-point calibration curve was generated from heteroduplex strands with a highest calibrator concentration of 40,000 pg/mL using 4-fold serial dilutions (plus 2 blank wells). Full-length (16-mer) probes were used at a concentration of 200 pM essentially following the method for singleplex antisense and sense assays described in Example 8. Blocker S1 (MSD) was added to N-PLEX^{™} Blocker (MSD). The probes were hybridized to the sense or antisense strands with Diluent 54 (MSD).

The results indicate that including Blocker S1 in the N-PLEX^{™} Blocker had no impact on positive or background signal generation for either the AS or SS strands in singleplex.

### Example 10. Impact of Double RNase Digestion on Background

The effect of performing two RNase digestion steps was evaluated for the impact on backgrounds associated with the antisense (AS) and sense (SS) singleplex assay described in Example 1.

Briefly, a 10-point calibration curve was generated from heteroduplex strands with a highest calibrator concentration of 40,000 pg/mL using 4-fold serial dilutions (plus 2 blank wells). Full-length (16-mer) probes were used at a concentration of 200 pM essentially following the method for singleplex antisense and sense assays described in Example 8. The RNase digestion step was performed once or twice. The wells in which the RNase digestion step was performed once were incubated with Diluent 54 during the second RNase digestion step. The probes were hybridized to the antisense or sense strands with Diluent 54.

The data (not shown) indicate that adding an extra RNase digestion step had little impact on positive or background ECL signals or LLODₑₛₜ measurement.

### Example 11. Detection of antisense and sense strands in brain lysates

The detection limits for the antisense and sense strands or the full model heteroduplex ASO from Example 1 were determined using the RNase Protection Assay (RPA) essentially described in Example 8 (with the modifications provided below), using full-length (16-mer) probes in brain lysates.

Briefly, a 10-point calibration curve was generated using the antisense, sense and heteroduplex strands spiked into diluted brain lysate with a highest calibrator concentration of 40,000 pg/mL using 4-fold serial dilutions (plus 2 blank wells). Full-length 16-mer (FL) probes were used at a concentration of 200 pM.

The probes were hybridized to the antisense or sense strands following the protocol described in Example 8. Brains (BioIVT, Westbury, NY, U.S.A.) were homogenized and lysed in Diluent 54. The homogenate was centrifuged and the lysate was collected and diluted 1:4 in Diluent 54.

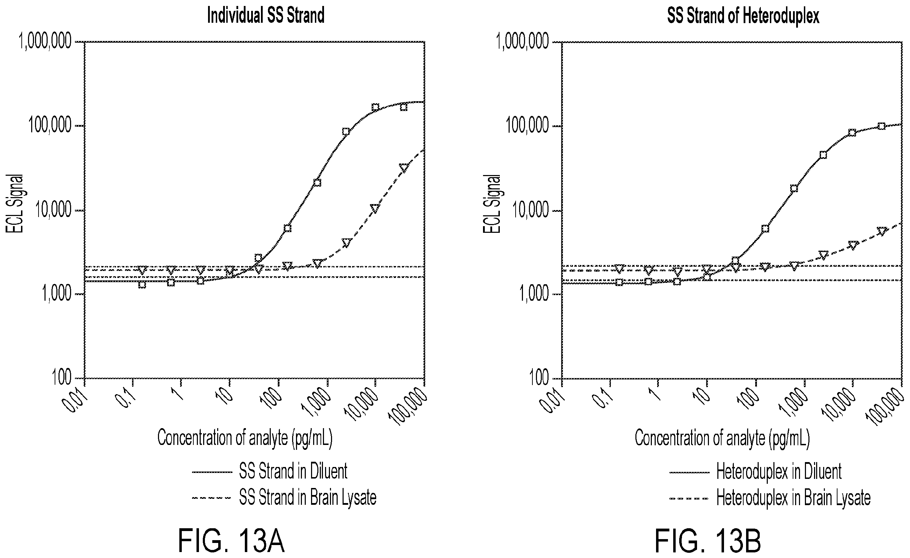

The data (not shown) indicate that ECL signal generation from the AS strand (individual or as part of heteroduplex) is very similar between Diluent 54 or 1:4 diluted brain lysates. As shown in FIG. 13A and 13B, the ECL signals were much lower from the sense (SS) strand (individual or as part of the heteroduplex) in brain lysates, compared with Diluent 54. In contrast to the results in Example 8 showing the results in plasma, the individual sense (SS) strand generates higher ECL signals than the sense (SS) strand of the heteroduplex in brain lysates. This could be due to the release of RNase H upon lysis of brain cells.

The LLOD for the antisense (AS) and sense (SS) strands alone or in a heteroduplex in Diluent 54 or brain lysate are shown in **Table 30** and **Table 31**. These results show that the antisense (DNA) strand can readily be measured in diluted brain lysates.

**Table 30: Antisense strand detection (LLOD) in Diluent 54 or brain lysate**

| | **Antisense strand alone** | | **Heteroduplex** | |
|---|---|---|---|---|
| | **Diluent 54** | **Brain Lysate** | **Diluent 54** | **Brain Lysate** |
| Hill Slope | 1.05 | 1.07 | 1.01 | 0.99 |
| r² value | 0.996 | 0.996 | 0.999 | 1.000 |
| LLODₑₛₜ (pg/mL) | 0.26 | 0.42 | 0.25 | 0.16 |

**Table 31: Sense strand detection (LLOD) in Diluent 54 or brain lysate**

| | **Sense strand alone** | | **Heteroduplex** | |
|---|---|---|---|---|
| | **Diluent 54** | **Brain Lysate** | **Diluent 54** | **Brain Lysate** |
| Hill Slope | 1.18 | 1.10 | 1.02 | 0.70 |
| r² value | 0.985 | 1.000 | 1.000 | 0.995 |
| LLODₑₛₜ (pg/mL) | 11.3 | 226 | 2.70 | 341 |

### Example 12. Addition of LNAs to probes

The impact of including locked nucleic acids (LNAs) in the 12-mer RNase protection probes was evaluated for the impact on detection of the antisense or sense strands of the model ASO from Example 1 was determined in singleplex assays. The method was performed essentially as described in Example 8.

Briefly, a 10-point calibration curve was generated using the antisense, sense and heteroduplex strands spiked with a highest calibrator concentration of 40,000 pg/mL using 4-fold serial dilutions (plus 2 blank wells). Full-length 16-mer (FL) or 12-mer probes with LNAs were used at a concentration of 200 pM. The probes were hybridized to the antisense or sense strands of the model ASO with Diluent 54.

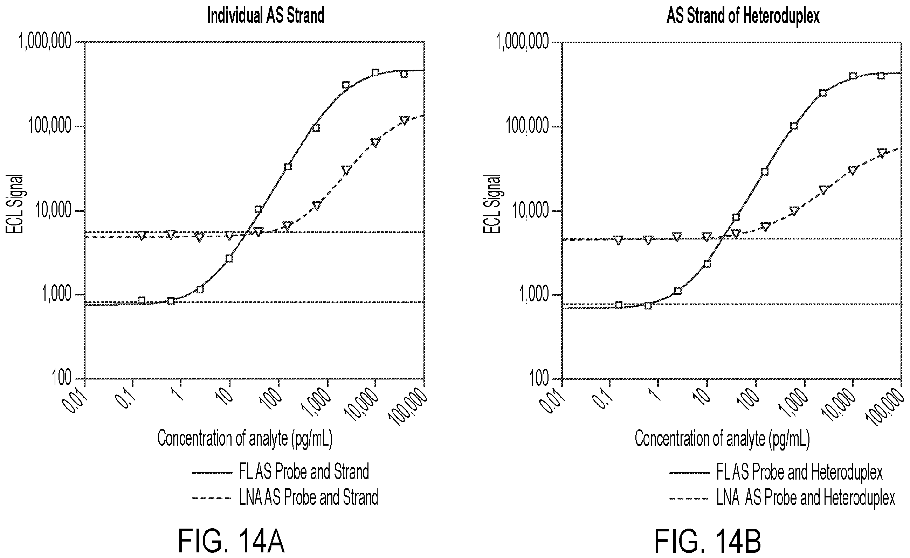

FIG. 14A and 14B show the impact of LNAs in the 16-mer (FL) or 12-mer RNase probes for detecting the antisense (AS) strand alone or in a heteroduplex. **Table 32** shows the impact of LNAs in the 16-mer (FL) or 12-mer RNase probes for detecting the sense (SS) strand alone or in a heteroduplex. **Table 33** shows the LLOD for a FL or 12-mer antisense probe with and without LNA. **Table 34** shows the LLOD for a FL or 12-mer sense probe with and without LNA.

The results show that the full length 16-mer (FL) probes generated higher positive and lower background signals for both the AS and SS singleplex assays. The higher backgrounds observed with the LNA probes were likely due to the resistance of LNA oligos to nuclease degradation. Although the target of degradation in the probes are the RNA residues, LNAs helped protect the entire oligos from RNases.

**Table 32: ECL Signal - sense detection with FL or LNA 12-mer probe**

| | | **Sense strand alone** | | **Heteroduplex** | |
|---|---|---|---|---|---|
| **Calibrator** | **Conc. (pg/mL)** | **FL Probe** | **LNA 12-mer** | **FL Probe** | **LNA 12-mer** |
| Cal-1 | 40000 | 129219 | 92954 | 85283 | 81086 |
| Cal-2 | 10000 | 117400 | 75898 | 66956 | 61790 |
| Cal-3 | 2500 | 47186 | 37943 | 36465 | 31961 |
| Cal-4 | 625 | 10107 | 10023 | 12307 | 11572 |
| Cal-5 | 156 | 3179 | 4572 | 4335 | 5453 |
| Cal-6 | 39.1 | 1559 | 3561 | 1717 | 3660 |
| Cal-7 | 9.77 | 1137 | 3252 | 1138 | 3098 |
| Cal-8 | 2.44 | 1043 | 2986 | 1079 | 2966 |
| Cal-9 | 0.610 | 1027 | 3042 | 927 | 2916 |
| Cal-10 | 0.153 | 1026 | 3135 | 917 | 3029 |
| Blank | 0 | 1061 | 3220 | 943 | 3084 |

**Table 33: Antisense strand detection (LLOD) - LNA probe**

| | **Antisense strand alone** | | **Heteroduplex** | |
|---|---|---|---|---|
| | **FL Probe** | **LNA 12-mer** | **FL Probe** | **LNA 12-mer** |
| Hill Slope | 1.03 | 0.99 | 1.04 | 0.73 |
| r² value | 0.984 | 0.999 | 0.995 | 1.000 |
| LLODₑₛₜ (pg/mL) | 0.42 | 57.8 | 0.48 | 7.17 |

**Table 34: Sense strand detection (LLOD) - LNA probe**

| | **Sense strand alone** | | **Heteroduplex** | |
|---|---|---|---|---|
| | **FL Probe** | **LNA 12-mer** | **FL Probe** | **LNA 12-mer** |
| Hill Slope | 1.24 | 1.35 | 1.01 | 1.03 |
| r² value | 0.989 | 1.000 | 1.000 | 1.000 |
| LLODₑₛₜ (pg/mL) | 12.7 | 84.4 | 3.63 | 31.1 |

### Example 13. Concentration Testing of LNA probes

The impact of a decreased concentration of LNA probe on background levels and sensitivity of the singleplex AS and SS assays was evaluated for the model ASO from Example 1.

Briefly, a 10-point calibration curve was generated using the heteroduplex strands with a highest calibrator concentration of 40,000 pg/mL using 4-fold serial dilutions (plus 2 blank wells). The AS and SS assays were run in singleplex and 12-mer probes with LNAs were used for both AS and SS assays at 4 concentrations: 200 pM (0.2 nM); 100 pM (0.1 nM); 50 pM (0.05 nM); and 20 pM (0.02 nM). The probes were hybridized to the antisense (AS) or sense (SS) strands with Diluent 54.

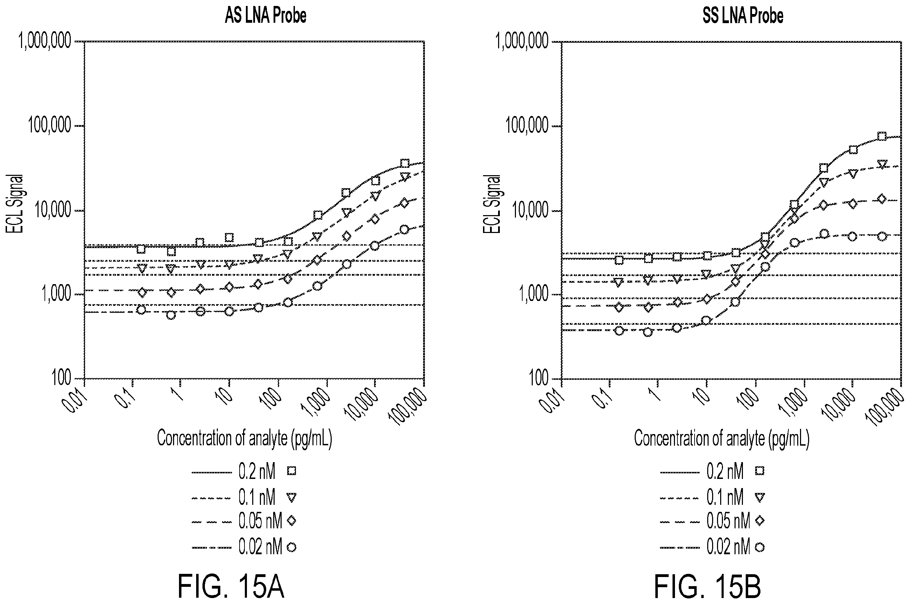

FIG. 15A and 15B show that decreasing the concentration of LNA probes for either the AS or SS strand of the heteroduplex decreases both positive and background ECL signals, with a probe concentration of 0.02 mM and 0.1 nM, respectively, providing improved positive and background signals.

**Table 35** and **Table 36** show the LLOD for varying LNA probe concentrations for detecting the antisense or sense strand in a heteroduplex, respectively.

**Table 35: Antisense strand heteroduplex detection (LLOD)**

| | **0.2 nM** | **0.1 nM** | **0.05 nM** | **0.02 nM** |
|---|---|---|---|---|
| Hill Slope | 0.92 | 0.71 | 0.81 | 0.79 |
| r² value | 0.976 | 0.998 | 0.997 | 0.998 |
| LLODₑₛₜ (pg/mL) | 15.9 | 50.9 | 191 | 81.7 |

**Table 36: Sense strand heteroduplex detection (LLOD)**

| | **0.2 nM** | **0.1 nM** | **0.05 nM** | **0.02 nM** |
|---|---|---|---|---|
| Hill Slope | 1.07 | 0.99 | 1.10 | 1.21 |
| r² value | 0.995 | 0.992 | 0.995 | 0.996 |
| LLODest (pg/mL) | 29.6 | 17.7 | 10.7 | 7.76 |

### Example 14. LNA probes in multiplexed detection of antisense and sense strands

Multiplexed detection of AS and SS strands of the model heteroduplex from Example 1 was evaluated using LNA probes.

Briefly, a 10-point calibration curve was generated using the individual antisense (AS), sense (SS) or heteroduplex strands with a highest calibrator concentration of 40,000 pg/mL using 4-fold serial dilutions (plus 2 blank wells). The assay included wells with only probe to determine whether probe-probe interactions occurred between the antisense and sense probes.

Two different probe types were assessed: an unmodified 12-mer probe or an LNA 12-mer probe. 200 pM probe concentration for unmodified 12-mer probes. The 12-mer AS probe was used at a concentration of 20 pM and the 12-mer SS probe was used at a concentration of 100 pM for AS. The SS LNA probe was also used at a concentration of 100 pM. The concentration of LNA probes was changed to account for higher backgrounds. The probes were hybridized to the antisense or sense strands with Diluent 54.

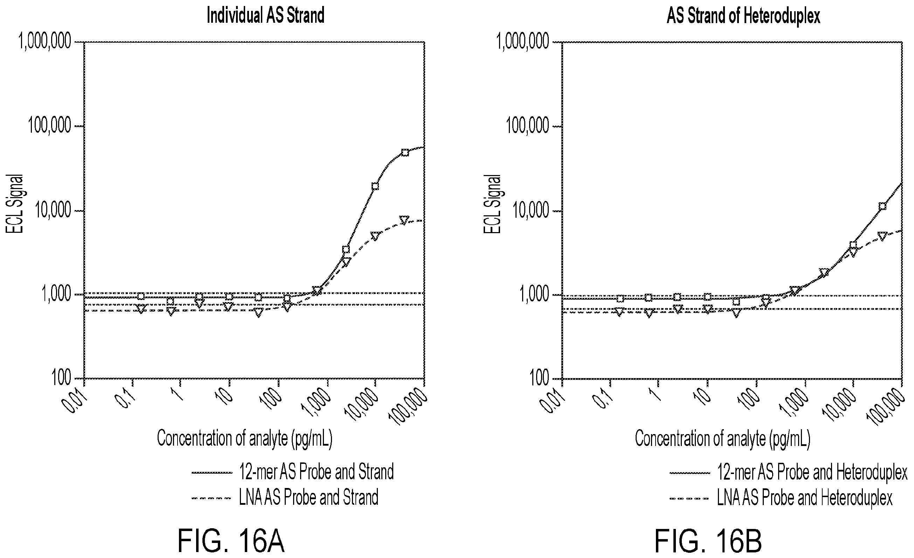

FIG. 16A and 16B show there was no off-target signal from either the non-modified 12-mer probes or the LNA 12-mer probes, indicating that probe-probe interactions did not occur in either case, although the LNA probes gave a lower positive ECL signal and similar background to the non-modified 12-mer probes for the AS strand.

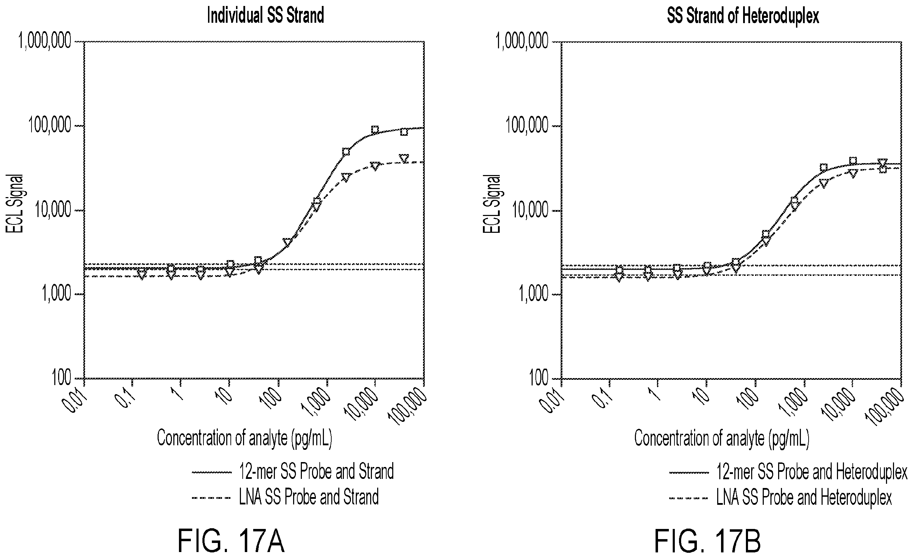

FIG. 17A and 17B show there was no off-target signal from either the non-modified 12-mer probes or the LNA 12-mer probes, indicating that probe-probe interactions did not occur in either case for the SS probes.

**Table 37** shows the LLOD for the multiplex detection of individual antisense (AS) strand or antisense strands in a heteroduplex with unmodified and LNA modified 12-mer probes. **Table 38** shows the LLOD for the multiplex detection of individual sense (SS) strand or sense strands in a heteroduplex with unmodified and LNA modified 12-mer probes.

The LNA probes were able to detect both the AS and SS strands as individual strands or as part of the heteroduplex. There was no advantage of using the 12-mer LNA probes over the full-length probes in the singleplex assays. The LNA probes have higher backgrounds for both assays and must be diluted to get backgrounds to acceptable levels. Specific signal is also sacrificed. The unmodified 12-mer probes were superior to the 12-mer LNA probes for multiplexed detection of the AS and SS strands.

**Table 37: Antisense multiplexed detection (LLOD)**

| | **12- mer antisense probe** | | **12-mer LNA antisense probe** | |
|---|---|---|---|---|
| | **Antisense Individual** | **Heteroduplex** | **Antisense Individual** | **Heteroduplex** |
| Hill Slope | 1.73 | 0.95 | 1.12 | 0.80 |
| r² value | 1.000 | 1.000 | 0.997 | 0.999 |
| LLODₑₛₜ (pg/mL) | 432 | 186 | 180 | 61.6 |

**Table 38: Sense multiplexed detection (LLOD)**

| | **12- mer sense probe** | | **12-mer LNA sense probe** | |
|---|---|---|---|---|
| | **Sense Individual** | **Heteroduplex** | **Sense Individual** | **Heteroduplex** |
| Hill Slope | 1.36 | 1.33 | 1.18 | 1.10 |
| r² value | 0.986 | 0.977 | 0.998 | 0.987 |
| LLODₑₛₜ (pg/mL) | 30.0 | 19.6 | 32.5 | 9.30 |

### Example 15. Singleplex detection of antisense and sense strands using 13- and 14-mer probes

Detection of the individual antisense (AS) and sense (SS) strands of the model heteroduplex from Example 1 was evaluated using 13-mer and 14-mer probes following the method described in Example 8.

Briefly, a 10-point calibration curve was generated using heteroduplex strands with a highest calibrator concentration of 40,000 pg/mL using 4-fold serial dilutions (plus 2 blank wells). Four different probe types were assessed: an unmodified full-length (FL) probe, a 14-mer, a 13-mer, and a 12-mer probe at a concentration of 200 pM. The probes were hybridized to the antisense or sense strands with Diluent 54.

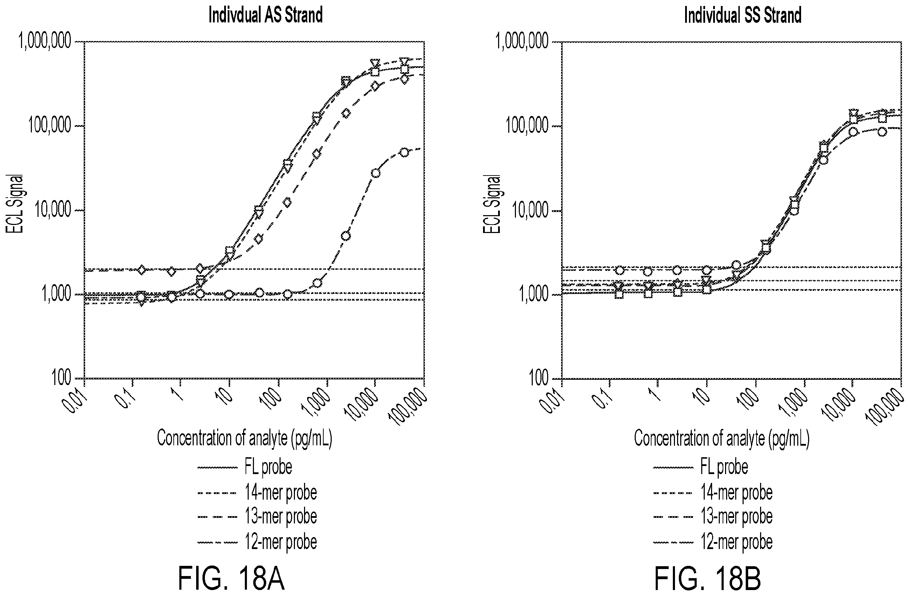

FIG. 18A and 18B show that the ECL signal for the 14-mer probe was similar to the FL probes for antisense and sense strand detection, while slight signal loss was observed with the 13-mer probe. A more marked loss was observed with the 12-mer probe as compared with the FL probe. Background increased with the 12-mer and 13-mer probes.

**Table 39** and **Table 40** show the LLOD for the detection of antisense (AS) or sense (SS) strands with the full-length (FL) probe, a 14-mer, a 13-mer, or a 12-mer probe.

**Table 39: Antisense detection (LLOD)**

| | **FL Probe** | **14-mer** | **13-mer** | **12-mer** |
|---|---|---|---|---|
| Hill Slope | 1.05 | 1.02 | 1.06 | 1.81 |
| r² value | 0.994 | 0.994 | 0.998 | 1.000 |
| LLODₑₛₜ (pg/mL) | 0.69 | 0.41 | 2.23 | 267 |

**Table 40: Sense detection (LLOD)**

| | **FL Probe** | **14-mer** | **13-mer** | **12-mer** |
|---|---|---|---|---|
| Hill Slope | 1.31 | 1.27 | 1.32 | 1.36 |
| r² value | 0.987 | 0.978 | 0.985 | 0.989 |
| LLODₑₛₜ (pg/mL) | 13.6 | 19.9 | 24.6 | 31.20 |

### Example 16. Heteroduplex detection of antisense and sense strands using 13- and 14-mer probes

Detection of the antisense (AS) and sense (SS) strands of the model heteroduplex from Example 1 was evaluated using 13-mer and 14-mer probes essentially described in Example 8.

Briefly, a 10-point calibration curve was generated using heteroduplex strands with a highest calibrator concentration of 40,000 pg/mL using 4-fold serial dilutions (plus 2 blank wells). Four different probe types were assessed: an unmodified full-length (FL) probe, a 14-mer, a 13-mer, and a 12-mer probe at a concentration of 200 pM. The probes were hybridized to the antisense or sense strands with Diluent 54.

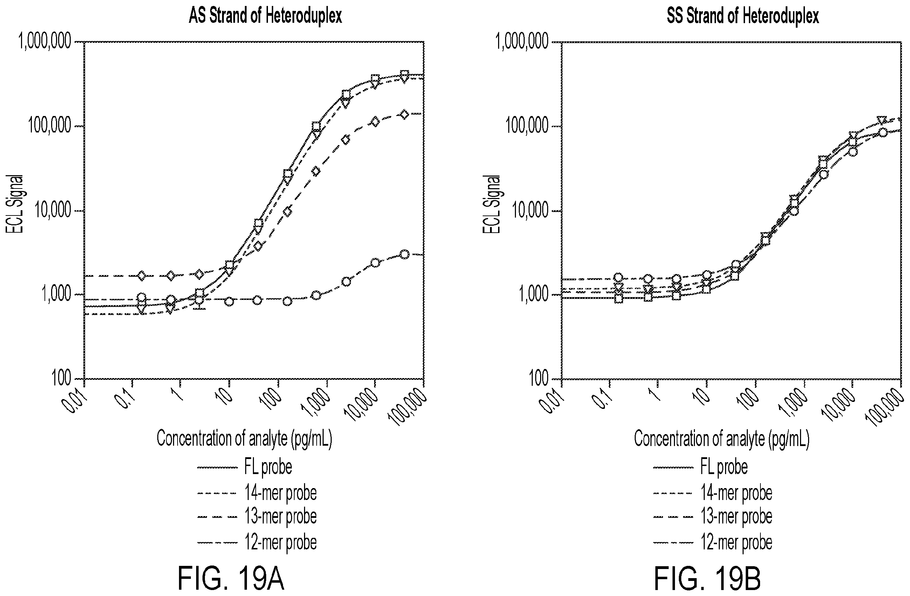

As shown in FIG. 19A and 19B, the FL and 14-mer probes gave similar signals when measuring the AS strand of the heteroduplex. The 13-mer probes showed a significant loss in signal and the 12-mer probes were unable to produce sufficient signal when measuring the AS strand of the heteroduplex. Backgrounds increased with the 13-mer probes. For the SS probes and strand detection, the 14-mer and 13-mer probes were similar to the FL probes, whereas there was some signal loss with the 12-mer probe. Backgrounds increased with the 12-mer probe.

**Tables 41** and **Table 42** show the LLOD for the heteroduplex detection of antisense (AS) or sense (SS) strands with a full-length (FL) probe, a 14-mer, a 13-mer, or a 12-mer probe.

**Table 41: Antisense strand detection (LLOD)**

| | **FL Probe** | **14-mer** | **13-mer** | **12-mer** |
|---|---|---|---|---|
| Hill Slope | 1.09 | 1.06 | 1.00 | 0.78 |
| r² value | 1.000 | 0.996 | 0.999 | 0.714 |
| LLODₑₛₜ (pg/mL) | 0.94 | 0.91 | 1.98 | 223 |

**Table 42: Sense detection (LLOD)**

| | **FL Probe** | **14-mer** | **13-mer** | **12-mer** |
|---|---|---|---|---|
| Hill Slope | 1.02 | 0.94 | 0.98 | 0.90 |
| r² value | 1.000 | 0.998 | 0.992 | 0.995 |
| LLODₑₛₜ (pg/mL) | 3.94 | 4.87 | 3.17 | 4.31 |

### Example 17. Multiplex detection of antisense and sense strands using 13- and 14-mer probes

This experiment was designed to determine whether the 13-mer or 14-mer probes allows for multiplex detection of the antisense (AS) and sense (SS) strands or the model heteroduplex from Example 1.

Briefly, a 10-point calibration curve was generated using heteroduplex strands with a highest calibrator concentration of 40,000 pg/mL using 4-fold serial dilutions (plus 2 blank wells). Probe-only wells were included to evaluate probe-probe interactions.

Two different probe types were assessed: a 14-mer and a 13-mer probe at a concentration of 200 pM. The probes were hybridized to the antisense or sense strands in Diluent 54.

As shown in **Table 43** and **Table 44,** the 14-mer probe allowed for detection of both the individual AS strand and the AS strand of the heteroduplex, although the 14-mer probe showed a slight decrease in signal when measuring the heteroduplex compared with the individual strand. The 13-mer probes also allowed for detection of the individual AS strand and the AS strand of the heteroduplex, but a signal drop was observed. Importantly, both the 14-mer and 13-mer probes were compatible with multiplexed detection of the AS strand of the heteroduplex and did not cause probe-probe interactions.

As shown in **Table 45** and **Table 46,** both the 14-mer and 13-mer probes allowed for detection of the SS strand of the heteroduplex, although the 14-mer probe gave slightly higher signals than the 13-mer probe. Importantly, both the 14-mer and 13-mer probes were compatible with multiplexed detection of the SS strand of the heteroduplex and did not cause probe-probe interactions.

**Table 47** and **Table 48** show the LLOD for the detection of antisense (AS) or sense (SS) strands alone or as part of a heteroduplex with a 14-mer and 13-mer probe.

Therefore, the 14-mer probe is the optimal probe for the antisense strand, while either the 13-mer or 14-mer probe can be used for detection of the sense strand.

**Table 43: ECL Signal -Detection of Antisense (AS) strand with 14-mer probe**

| **Calibrator** | **Conc. (pg/mL)** | **AS strand** | **SS strand** | **Heteroduplex** | **Blank (probe only)** |
|---|---|---|---|---|---|
| Cal-1 | 40000 | 491963 | 670 | 409536 | 674 |
| Cal-2 | 10000 | 370227 | 639 | 307180 | 705 |
| Cal-3 | 2500 | 270123 | 674 | 185649 | 697 |
| Cal-4 | 625 | 97312 | 704 | 75888 | 726 |
| Cal-5 | 156 | 25190 | 678 | 24355 | 696 |
| Cal-6 | 39.1 | 6479 | 681 | 6257 | 743 |
| Cal-7 | 9.77 | 1932 | 638 | 2295 | 725 |
| Cal-8 | 2.44 | 931 | 655 | 1114 | 721 |
| Cal-9 | 0.610 | 718 | 607 | 775 | 731 |
| Cal-10 | 0.153 | 714 | 650 | 747 | 728 |
| Blank | 0 | 691 | 688 | 704 | 747 |

**Table 44: ECL Signal -Detection of Antisense (AS) strand with 13-mer probe**

| **Calibrator** | **Conc. (pg/mL**) | **AS strand** | **SS strand** | **Heteroduplex** | **Blank (probe only)** |
|---|---|---|---|---|---|
| Cal-1 | 40000 | 280311 | 1628 | 182534 | 1577 |
| Cal-2 | 10000 | 236813 | 1464 | 116301 | 1486 |
| Cal-3 | 2500 | 125262 | 1559 | 65630 | 1448 |
| Cal-4 | 625 | 37176 | 1466 | 26445 | 1379 |
| Cal-5 | 156 | 10311 | 1492 | 7678 | 1434 |
| Cal-6 | 39.1 | 3950 | 1458 | 3703 | 1462 |
| Cal-7 | 9.77 | 2151 | 1471 | 1782 | 1384 |
| Cal-8 | 2.44 | 1705 | 1486 | 1506 | 1425 |
| Cal-9 | 0.610 | 1160 | 1393 | 1401 | 1343 |
| Cal-10 | 0.153 | 1490 | 1450 | 1464 | 1379 |
| Blank | 0 | 1547 | 1508 | 1572 | 1498 |

**Table 45: ECL Signal - Detection of Sense (SS) strand with 14-mer probe**

| **Calibrator** | **Conc. (pg/mL)** | **AS strand** | **SS strand** | **Heteroduplex** | **Blank (probe only)** |
|---|---|---|---|---|---|
| Cal-1 | 40000 | 1211 | 123111 | 65819 | 1454 |
| Cal-2 | 10000 | 1165 | 121003 | 63842 | 1543 |
| Cal-3 | 2500 | 1238 | 61737 | 41795 | 1478 |
| Cal-4 | 625 | 1198 | 14349 | 15469 | 1473 |
| Cal-5 | 156 | 1221 | 5029 | 5811 | 1478 |
| Cal-6 | 39.1 | 1222 | 2355 | 2222 | 1622 |
| Cal-7 | 9.77 | 1419 | 1863 | 1713 | 1535 |
| Cal-8 | 2.44 | 1298 | 1403 | 1527 | 1519 |
| Cal-9 | 0.610 | 1308 | 1276 | 1477 | 1476 |
| Cal-10 | 0.153 | 1243 | 1401 | 1433 | 1515 |
| Blank | 0 | 1270 | 1301 | 1407 | 1518 |

**Table 46: ECL Signal - Detection of Sense (SS) strand with 13-mer probe**

| **Calibrator** | **Conc. (pg/mL)** | AS **strand** | **SS strand** | **Heteroduplex** | **Blank (probe only)** |
|---|---|---|---|---|---|
| Cal-1 | 40000 | 967 | 80842 | 54354 | 1037 |
| Cal-2 | 10000 | 1039 | 106108 | 52571 | 1028 |
| Cal-3 | 2500 | 1097 | 59334 | 32456 | 1011 |
| Cal-4 | 625 | 1036 | 12920 | 12531 | 992 |
| Cal-5 | 156 | 1036 | 4100 | 4668 | 983 |
| Cal-6 | 39.1 | 1036 | 1643 | 1967 | 1003 |
| Cal-7 | 9.77 | 1118 | 1306 | 1250 | 1028 |
| Cal-8 | 2.44 | 1114 | 1103 | 1113 | 986 |
| Cal-9 | 0.610 | 961 | 1050 | 1038 | 983 |
| Cal-10 | 0.153 | 1061 | 1070 | 1042 | 977 |
| Blank | 0 | 1100 | 1082 | 1082 | 1083 |

**Table 47: Antisense (AS) strand detection (LLOD)**

| | **14-mer probe** | | **13-mer probe** | |
|---|---|---|---|---|
| | **AS alone** | **AS of heteroduplex** | **AS alone** | **AS of heteroduplex** |
| Hill Slope | 1.10 | 0.98 | 1.04 | 0.94 |
| r² value | 0.993 | 0.997 | 0.997 | 0.993 |
| LLODₑₛₜ (pg/mL) | 0.77 | 0.43 | 3.85 | 2.32 |

**Table 48: Sense (SS) strand detection (LLOD)**

| | **14-mer probe** | | **13-mer probe** | |
|---|---|---|---|---|
| | **SS alone** | **SS of heteroduplex** | **SS alone** | **SS of heteroduplex** |
| Hill Slope | 1.12 | 1.10 | 1.29 | 1.02 |
| r² value | 0.981 | 0.997 | 0.951 | 0.996 |
| LLODₑₛₜ (pg/mL) | 14.5 | 9.78 | 15.30 | 3.44 |

### Example 18. Probe cross-check for multiplex detection

This experiment was designed to evaluate different combination(s) of AS and SS probes for multiplexed detection of both strands of the heteroduplex.

Briefly, a 10-point calibration curve was generated using heteroduplex strands with a highest calibrator concentration of 40,000 pg/mL using 4-fold serial dilutions (plus 2 blank wells). Three different probe types were assessed: a 14-mer, a 13-mer and a 12-mer probe at a concentration of 200 pM. The probes were hybridized to the antisense or sense strands with Diluent 54.

Each probe was crossed with the other two probes as follows:
- 14-mer AS vs 14-mer SS
- 14-mer AS vs 13-mer SS
- 14-mer AS v. 12-mer SS
- 13-mer AS vs 14-mer SS
- 13-mer AS vs 13-mer SS
- 13-mer AS v. 12-mer SS
- 12-mer AS vs 14-mer SS
- 12-mer AS vs 13-mer SS

12-mer AS vs 12-mer SS was not evaluated.

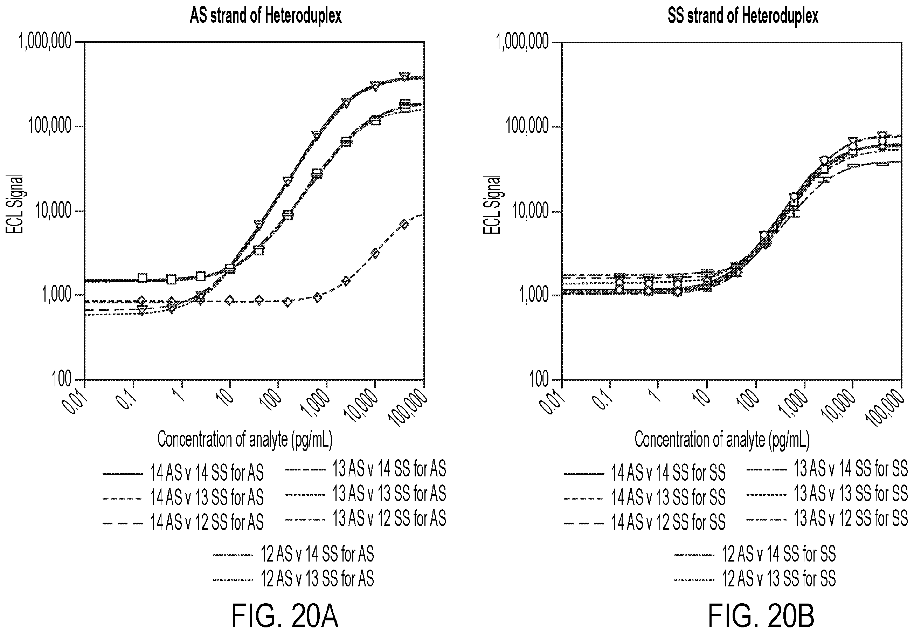

As shown in FIG. 20A, the ECL signals were consistent for a given AS probe length, regardless of the length of the SS probe. The 14-mer AS probe showed less signal loss than the 13-mer and 12-mer AS probes. As shown in FIG. 20B, changing the length of the SS probe had a less dramatic impact on the ECL signal, as compared with changes to the AS probe length. The SS signal decreased as the AS probe decreases, especially with the 12-mer AS probe. Importantly, either the 14-mer or 13-mer SS probes worked well for multiplex detection.

**Table 49** provides LLOD and hill slope measurements for the probe combinations. These data indicate that it is possible to measure both the sense and antisense strand of a heteroduplex in the same well with high sensitivity by using shortened probes.

**Table 49: Curve Fit and Limits**

| AS Probe | **14-mer** | | | **13-mer** | | | **12-mer** | |
|---|---|---|---|---|---|---|---|---|
| SS Probe | **14-mer** | **13-mer** | **12-mer** | **14-mer** | **13-mer** | **12-mer** | **14-mer** | **13-mer** |
| AS Hill Slope | 1.01 | **0.99** | 1.00 | 0.95 | 0.94 | 0.91 | 1.13 | 1.16 |
| AS r² value | 0.995 | **0.997** | 0.999 | 0.997 | 0.990 | 0.995 | 1.000 | 1.000 |
| AS LLODₑₛₜ (pg/mL) | 0.88 | **0.54** | 0.46 | 1.92 | 2.03 | 4.07 | 377 | 408 |
| SS Hill Slope | 1.03 | **1.00** | 1.00 | 1.04 | 1.09 | 1.07 | 1.05 | 1.04 |
| SS r² value | 0.999 | **1.000** | 0.999 | 0.999 | 1.000 | 0.996 | 1.000 | 1.000 |
| SS LLODₑₛₜ (pg/mL) | 7.38 | **3.60** | 6.50 | 3.58 | 4.88 | 10.94 | 4.13 | 8.06 |

### ASPECTS

1. A method of detecting or quantifying a sense and an antisense strand of an oligonucleotide duplex in a sample, the method comprising:
   (a) contacting the sample with a composition comprising a set of probes, wherein the set of probes comprises:
      (i) a sense probe comprising a first single stranded oligonucleotide tag that is complementary to at least a portion of a first capture oligonucleotide immobilized on a support surface, a sense binding portion capable of hybridizing to a nucleotide sequence of the sense strand of the oligonucleotide duplex, and a first label; and
      (ii) an antisense probe comprising a second single stranded oligonucleotide tag that is complementary to at least a portion of a second capture oligonucleotide immobilized on the support surface, an antisense binding portion capable of hybridizing to a nucleotide sequence of the antisense strand of the oligonucleotide duplex, and a second label,
         wherein the sense binding portion of the sense probe has a sense binding length that is shorter than a sense strand length of the sense strand, and
         wherein the antisense binding portion of the antisense strand has an antisense binding length that is shorter than an antisense strand length of the antisense strand; and
   (b) incubating the probes with the sample to form a hybridization mixture comprising hybridization complexes comprising:
      (i) a sense complex comprising the sense probe hybridized with the sense strand of the oligonucleotide duplex; and
      (ii) an antisense complex comprising the antisense probe hybridized with the antisense strand of the oligonucleotide duplex; and
   (c) contacting the support surface with the hybridization mixture in which the first and second oligonucleotide tags of the sense and antisense probes hybridize to the first and second capture oligonucleotides immobilized on the support surface and contacting the hybridization complexes in the hybridization mixture with a single-strand specific nuclease; and
   (d) detecting or quantifying the sense and antisense strands of the oligonucleotide duplex based on the presence of the label on the support surface.
2. The method of aspect 1, wherein (c) comprises:
   (i) contacting the support surface with the hybridization mixture under conditions in which the first and second oligonucleotide tags of the hybridization complexes hybridize to the first and second capture oligonucleotides on the support surface to immobilize the hybridization complexes on the support surface; and
   (ii) contacting the immobilized hybridization complexes with a single-strand specific nuclease.
3. The method of aspect 1, wherein (c) comprises:
   (i) contacting the hybridization mixture with a single-strand specific nuclease to form a reaction mixture; and
   (ii) contacting the support surface with the reaction mixture of (i) under conditions in which the first and second oligonucleotide tags of the hybridization complexes hybridize to the first and second capture oligonucleotides immobilized on the support surface.
4. The method of aspect 1, wherein the sense and antisense strands of the oligonucleotide duplex each comprise, individually, from about 8 to about 50 nucleotides.
5. The method of any of aspects 1 to 4, wherein the sense and antisense strands of the oligonucleotide duplex each comprise, individually, from about 16 to about 30 nucleotides.
6. The method of any of aspects 1 to 5, wherein the sense strand of the oligonucleotide duplex comprises DNA.
7. The method of any of aspects 1 to 5, wherein the sense strand of the oligonucleotide duplex comprises RNA.
8. The method of any of aspects 1 to 5, wherein the antisense strand of the oligonucleotide duplex comprises DNA.
9. The method of any of aspects 1 to 5, wherein the antisense strand of the oligonucleotide duplex comprises RNA.
10. The method of any of aspects 1 to 5, wherein the oligonucleotide duplex comprises a DNA/DNA duplex.
11. The method of any of aspects 1 to 5, wherein the oligonucleotide duplex comprises a RNA/RNA duplex.
12. The method of any of aspects 1 to 5, wherein the oligonucleotide duplex comprises a DNA/RNA heteroduplex.
13. The method of any of aspects 1 to 12, wherein the sense strand, antisense strand or both the sense and antisense strands of the oligonucleotide duplex individually comprise one or more modified nucleic acids.
14. The method of any of aspects 1 to 13, wherein the sense strand, the antisense strand or both the sense and antisense strands of the oligonucleotide duplex individually comprise a 5'- or 3'- conjugate.
15. The method of aspect 14, wherein the conjugate comprises polyethylene glycol (PEG), N-acetylgalactosamine (GalNAc), a cell penetrating peptide (CPP), *α*-tocopherol, an aptamer, an antibody, cholesterol, squalene, a fatty acid, or a nucleolipid.
16. The method of any of aspects 1 to 15, wherein the sense binding length of the sense probe is shorter than the sense strand length of the sense strand by at least 1 nucleotide.
17. The method of any of aspects 1 to 16, wherein the sense binding length is about 10 to about 16 nucleotides in length.
18. The method of any of aspect 1 to 17, wherein the sense binding portion of the sense probe has a 5' end that aligns with a 3' end of the sense strand of the oligonucleotide duplex.
19. The method of any of aspects 1 to 18, wherein the antisense binding length of the antisense probe is shorter than the antisense strand length of the antisense strand by at least 1 nucleotide.
20. The method of any of aspects 1 to 19, wherein the antisense binding length is about 10 to about 16 nucleotides in length.
21. The method of any of aspects 1 to 20, wherein the antisense binding portion of the antisense probe has a 5' end that aligns with a 3' end of the antisense strand of the oligonucleotide duplex.
22. The method of any of aspects 1 to 21, wherein the first oligonucleotide tag has a first oligonucleotide tag length and the first capture oligonucleotide has a first capture oligonucleotide length, and the first oligonucleotide tag length is the same as the first capture oligonucleotide length.
23. The method of any of aspects 1 to 21, wherein the first oligonucleotide tag has a first oligonucleotide tag length and the first capture oligonucleotide has a first capture oligonucleotide length, and the first oligonucleotide tag length is the shorter than the first capture oligonucleotide length.
24. The method of any of aspects 1 to 21, wherein the second oligonucleotide tag has a second oligonucleotide tag length and the second capture oligonucleotide has a second capture oligonucleotide length, and the second oligonucleotide tag length is the same as the second capture oligonucleotide length.
25. The method of any of aspects 1 to 21, wherein the second oligonucleotide tag has a second oligonucleotide tag length and the second capture oligonucleotide has a second capture oligonucleotide length, and the second oligonucleotide tag length is the shorter than the second capture oligonucleotide length.
26. The method of any of aspects 1 to 25, wherein the sense probe comprises DNA.
27. The method of aspect 26, wherein the sense binding portion of the sense probe comprises DNA.
28. The method of aspect 26, wherein the first oligonucleotide tag of the sense probe comprises DNA.
29. The method of aspect 26, wherein the sense binding portion and the oligonucleotide tag of the sense probe comprise DNA.
30. The method of aspect 29, wherein the sense binding portion of the sense probe comprises DNA and the oligonucleotide tag of the sense probe comprises RNA.
31. The method of any of aspects 1 to 25, wherein the antisense probe comprises DNA.
32. The method of aspect 31, wherein the antisense binding portion of the antisense probe comprises DNA.
33. The method of aspect 31, wherein the first oligonucleotide tag of the antisense probe comprises DNA.
34. The method of aspect 31, wherein the antisense binding portion and the oligonucleotide tag of the antisense probe comprise DNA.
35. The method of aspect 31, wherein the antisense binding portion of the antisense probe comprises DNA and the oligonucleotide tag of the antisense probe comprises RNA.
36. The method of any of aspects 1 to 25, wherein the sense probe comprises RNA.
37. The method of aspect 36, wherein the sense binding portion of the sense probe comprises RNA.
38. The method of aspect 36, wherein the first oligonucleotide tag of the sense probe comprises RNA.
39. The method of aspect 36, wherein the sense binding portion and the oligonucleotide tag of the sense probe comprise RNA.
40. The method of aspect 36, wherein the sense binding portion of the sense probe comprises RNA and the oligonucleotide tag of the sense probe comprises DNA.
41. The method of any of aspects 1 to 25, wherein the antisense probe comprises RNA.
42. The method of aspect 41, wherein the antisense binding portion of the antisense probe comprises RNA.
43. The method of aspect 41, wherein the first oligonucleotide tag of the antisense probe comprises RNA.
44. The method of aspect 41, wherein the antisense binding portion and the oligonucleotide tag of the antisense probe comprise RNA.
45. The method of aspect 41, wherein the antisense binding portion of the antisense probe comprises RNA and the oligonucleotide tag of the antisense probe comprises DNA.
46. The method of any of aspects 1 to 45, wherein the sense probe, the antisense probe or both comprise one or more modified nucleic acids.
47. The method of aspect 46, wherein one or more modified nucleotides comprise a locked nucleic acid (LNA).
48. The method of aspect 46, wherein one or more modified nucleotides are selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof.
49. The method of any of aspects 26 to 35, wherein the single-strand specific nuclease comprises a single-strand specific DNase.
50. The method of aspect 49, wherein the single-strand specific DNase is S1 nuclease, P1 nuclease or Mung Bean nuclease.
51. The method of any of aspects 36 to 45, wherein the single-strand specific nuclease comprises a single-strand specific RNase.
52. The method of aspect 51, wherein the single-strand specific RNase is selected from RNase A, RNase H, RNase I, RNase III, RNase L, RNase P, RNase PhyM, RNase T1, RNase T2, RNase U2, RNase V, PNPase, RNase PH, RNase R, RNase D, RNase T, RNaseONE, oligoribonuclease, exoribonuclease I, and exoribonuclease II.
53. The method of aspect 1, wherein (a)-(c) are performed concurrently.
54. The method of aspect, wherein (a)-(c) are performed sequentially.
55. The method of any of aspects 1 to 54, wherein the hybridization conditions in (b) comprise:
   (i) incubating the probes with the sample a first temperature to denature the sense and antisense strands of the oligonucleotide duplex; and
   (ii) incubating the probes with the denatured sense and antisense strands of the oligonucleotide duplex at a second temperature to allow the sense and antisense probes to hybridize to the sense and antisense strands.
56. The method of aspect 55, further comprising incubating the sense and antisense complexes at a hold temperature of about 2°C to about 8°C.
57. The method of any of aspects 1 to 56, wherein the hybridization conditions in (b) comprise:
   (i) incubating the probes with the sample a first temperature from about 60°C to about 95°C for about 1 minute to about 15 minutes;
   (ii) incubating the probes with the sample at a second temperature from about 10°C to about 65°C for about 30 seconds to about 5 minutes; and
   (iii) incubating the probes with the sample at a hold temperature from about 2°C to about 8°C.
58. The method of aspect 57, wherein the hybridization conditions in (b) comprise:
   (i) incubating the probes with the sample a first temperature of about 95°C for about 2 minutes;
   (ii) incubating the probes with the sample at a second temperature of about 65°C for about 1 min; and
   (iii) incubating the probes with the sample at a hold temperature of about 4°C.
59. The method of aspect 57 or 58, comprising a first temperature transition rate between steps (i) and (ii) of about 1 °C/s to about 2 °C/s.
60. The method of aspect 57 or 58, comprising a first temperature transition rate between steps (i) and (ii) of about 1.8°C/s.
61. The method of aspect 57 or 58, comprising a second temperature transition rate between steps (ii) and (iii) about 0.05°C/s to about 1 C/s.
62. The method of aspect 57 or 58, comprising a second temperature transition rate between steps (ii) and (iii) of about 0.1°C/s.
63. The method of any of aspects 1 to 62, wherein the probes are incubated with the sample in a buffer comprising diluent 54 or N- PLEX hybridization Buffer 1 or 2.
64. The method according to any of aspects 1 to 63, wherein the sample comprises a plurality of oligonucleotide duplexes and the composition in (a) comprises a plurality of sets of probes, wherein each set of probes hybridizes with a unique sense or antisense strand of a unique oligonucleotide duplex.
65. The method of any of aspects 1 to 64, wherein (c) comprises incubating the support surface with the sense and antisense complexes for about 15 minutes to about 12 hours at a temperature of about 20 °C to about 40 °C.
66. The method of any of aspects 1 to 65, wherein (c) comprises incubating the support surface with the sense and antisense complexes for about 1 hour to about 2 hours at a temperature of about 20 °C to about 40 °C.
67. The method of aspect 65 or 66, wherein the support surface is incubated with the sense and antisense complexes while shaking.
68. The method of any of aspects 1 to 67, wherein (c) comprises incubating the support surface with the sense and antisense complexes for about 1 hour at a temperature of about 37°C, while shaking at about 705 rpm.
69. The method of any of aspects 1 to 68, wherein the composition in (a) comprises about 20 pM to about 10 nM sense probe.
70. The method of any of aspects 1 to 69, wherein the composition in (a) comprises about 20 pM to about 10 nM antisense probe.
71. The method of any of aspects 1 to 70, wherein the sample comprises a biological sample.
72. The method of aspect 71, wherein the sample comprises an untreated biological sample.
73. The method of aspect 71, wherein the sample comprises a pretreated biological sample.
74. The method of aspect 71, wherein the sample comprises a purified sample.
75. The method of aspect 74, wherein the sample is purified by precipitation, centrifugation, or column chromatography.
76. The method of aspect 74, wherein the sample comprises an extracted sample.
77. The method of aspect 71, wherein the sample comprises a naturally occurring RNase.
78. The method of aspect 77, wherein the method comprises combining the sample with an RNase inhibitor before (a).
79. The method of aspect 71, wherein the sample comprises cell-free DNA.
80. The method of aspect 71, wherein the biological sample comprises a fluid obtained from an organism.
81. The method of aspect 80, wherein the biological sample comprises whole blood, plasma, serum, urine, feces, breast milk, saliva, or amniotic fluid.
82. The method of any of aspects 1 to 70, wherein the sample comprises an environmental sample.
83. The method of any of aspects 1 to 70, wherein the sample comprises a manufacturing process sample.
84. The method of any of aspects 1 to 83, wherein the method has a limit of detection of less than about 200 pg/mL.
85. The method of any of aspects 1 to 84, wherein the support surface comprises one or more electrodes.
86. The method of aspect 85, wherein one or more electrodes comprise carbon electrodes.
87. The method of aspect 85 or 86, wherein one or more electrodes comprise carbon ink electrodes.
88. The method of any of aspects 85 to 87, wherein one or more electrodes are included in a multi-well plate.
89. The method of aspect 88, wherein each well of the multi-well plate includes an electrode.
90. The method of any of aspects 1 to 89, wherein the label comprises a member of a binding pair.
91. The method of aspect 90, wherein the label comprises biotin.
92. The method of any of aspects 1 to 89, wherein the label comprises an electrochemiluminescent label.
93. The method of aspect 92, comprising a step of generating an assay signal by contacting the electrodes with an electrochemiluminescence read buffer comprising an electrochemiluminescence co-reactant, and applying an electrical potential to the electrodes.
94. The method of aspect 93, wherein the co-reactant is selected from a tertiary amine, tripropylamine, N-butyldiethanolamine, and combinations thereof.
95. A composition comprising a set of probes, wherein the set of probes comprises:
   (a) a sense probe comprising a first single stranded oligonucleotide tag that is complementary to at least a portion of a first capture oligonucleotide, a sense binding portion capable of hybridizing to a nucleotide sequence of a sense strand of an oligonucleotide duplex, and a first label; and
   (b) an antisense probe comprising a second single stranded oligonucleotide tag that is complementary to at least a portion of a second capture oligonucleotide, an antisense binding portion capable of hybridizing to a nucleotide sequence of an antisense strand of the oligonucleotide duplex, and a second label,
      wherein the sense binding portion of the sense probe has a sense binding length that is shorter than a sense strand length of the sense strand, and
      wherein the antisense binding portion of the antisense strand has an antisense binding length that is shorter than an antisense strand length of the antisense strand.
96. A composition comprising:
   (a) an oligonucleotide duplex comprising a sense strand and an antisense strand; and
   (b) a set of probes comprising:
      (i) a sense probe comprising a first single stranded oligonucleotide tag that is complementary to at least a portion of a first capture oligonucleotide, a sense binding portion capable of hybridizing to a nucleotide sequence of a sense strand of an oligonucleotide duplex, and a first label; and
      (ii) an antisense probe comprising a second single stranded oligonucleotide tag that is complementary to at least a portion of a second capture oligonucleotide, an antisense binding portion capable of hybridizing to a nucleotide sequence of an antisense strand of the oligonucleotide duplex, and a second label,

      wherein the sense binding portion of the sense probe has a sense binding length that is shorter than a sense strand length of the sense strand, and
      wherein the antisense binding portion of the antisense strand has an antisense binding length that is shorter than an antisense strand length of the antisense strand.
97. A composition comprising one or more hybridization complexes, the hybridization complexes comprising:
   (a) a sense complex comprising a sense probe hybridized to a sense strand of an oligonucleotide duplex, wherein the sense probe comprises a first single stranded oligonucleotide tag that is complementary to at least a portion of a first capture oligonucleotide, a sense binding portion capable of hybridizing to a nucleotide sequence of the sense strand of the oligonucleotide duplex, and a first label, wherein the sense binding portion of the sense probe has a sense binding length that is shorter than a sense strand length of the sense strand;
   (b) an antisense complex comprising an antisense probe hybridized to an antisense strand of the oligonucleotide duplex, wherein the antisense probe comprises a second single stranded oligonucleotide tag that is complementary to at least a portion of a second capture oligonucleotide, an antisense binding portion capable of hybridizing to a nucleotide sequence of the antisense strand of the oligonucleotide duplex, and a second label, wherein the antisense binding portion of the antisense strand has an antisense binding length that is shorter than an antisense strand length of the antisense strand,
   and combinations thereof.
98. The composition of any of aspects 95 to 97, wherein the sense and antisense strands of the oligonucleotide duplex each comprise, individually, from about 8 to about 50 nucleotides.
99. The composition of any of aspects 95 to 98, wherein the sense and antisense strands of the oligonucleotide duplex each comprise, individually, from about 16 to about 30 nucleotides.
100. The composition of any of aspects 95 to 99, wherein the sense strand of the oligonucleotide duplex comprises DNA.
101. The composition of any of aspects 95 to 99, wherein the sense strand of the oligonucleotide duplex comprises RNA.
102. The composition of any of aspects 95 to 99, wherein the antisense strand of the oligonucleotide duplex comprises DNA.
103. The composition of any of aspects 95 to 99, wherein the antisense strand of the oligonucleotide duplex comprises RNA.
104. The composition of any of aspects 95 to 99, wherein the oligonucleotide duplex comprises a DNA/DNA duplex.
105. The composition of any of aspects 95 to 99, wherein the oligonucleotide duplex comprises a RNA/RNA duplex.
106. The composition of any of aspects 95 to 99, wherein the oligonucleotide duplex comprises a DNA/RNA heteroduplex.
107. The composition of any of aspects 95 to 106, wherein the sense strand, antisense strand or both the sense and antisense strands of the oligonucleotide duplex individually comprise one or more modified nucleic acids.
108. The composition of any of aspects 95 to 107, wherein the sense strand, the antisense strand or both the sense and antisense strands of the oligonucleotide duplex individually comprise a 5'- or 3'- conjugate.
109. The composition of aspect 108, wherein the conjugate comprises polyethylene glycol (PEG), N-acetylgalactosamine (GalNAc), a cell penetrating peptide (CPP), *α*-tocopherol, an aptamer, an antibody, cholesterol, squalene, a fatty acid, or a nucleolipid.
110. The composition of any of aspects 95 to 109, wherein the sense binding length of the sense probe is shorter than the sense strand length of the sense strand by at least 1 nucleotide.
111. The composition of any of aspects 95 to 110, wherein the sense binding length is about 10 to about 16 nucleotides in length.
112. The composition of any of aspect 95 to 111, wherein the sense binding portion of the sense probe has a 5' end that aligns with a 3' end of the sense strand of the oligonucleotide duplex.
113. The composition of any of aspects 95 to 112, wherein the antisense binding length of the antisense probe is shorter than the antisense strand length of the antisense strand by at least 1 nucleotide.
114. The composition of any of aspects 95 to 113, wherein the antisense binding length is about 10 to about 16 nucleotides in length.
115. The composition of any of aspects 95 to 114, wherein the antisense binding portion of the antisense probe has a 5' end that aligns with a 3' end of the antisense strand of the oligonucleotide duplex.
116. The composition of any of aspects 95 to 115, wherein the sense probe comprises DNA.
117. The composition of aspect 116, wherein the sense binding portion of the sense probe comprises DNA.
118. The composition of aspect 116, wherein the first oligonucleotide tag of the sense probe comprises DNA.
119. The composition of aspect 116, wherein the sense binding portion and the oligonucleotide tag of the sense probe comprise DNA.
120. The composition of aspect 116, wherein the sense binding portion of the sense probe comprises DNA and the oligonucleotide tag of the sense probe comprises RNA.
121. The composition of any of aspects 95 to 115, wherein the antisense probe comprises DNA.
122. The composition of aspect 121, wherein the antisense binding portion of the antisense probe comprises DNA.
123. The composition of aspect 121, wherein the first oligonucleotide tag of the antisense probe comprises DNA.
124. The composition of aspect 121, wherein the antisense binding portion and the oligonucleotide tag of the antisense probe comprise DNA.
125. The composition of aspect 121, wherein the antisense binding portion of the antisense probe comprises DNA and the oligonucleotide tag of the antisense probe comprises RNA.
126. The composition of any of aspects 95 to 115, wherein the sense probe comprises RNA.
127. The composition of aspect 126, wherein the sense binding portion of the sense probe comprises RNA.
128. The composition of aspect 126, wherein the first oligonucleotide tag of the sense probe comprises RNA.
129. The composition of aspect 126, wherein the sense binding portion and the oligonucleotide tag of the sense probe comprise RNA.
130. The composition of aspect 126, wherein the sense binding portion of the sense probe comprises RNA and the oligonucleotide tag of the sense probe comprises DNA.
131. The composition of any of aspects 95 to 115, wherein the antisense probe comprises RNA.
132. The composition of aspect 131, wherein the antisense binding portion of the antisense probe comprises RNA.
133. The composition of aspect 131, wherein the first oligonucleotide tag of the antisense probe comprises RNA.
134. The composition of aspect 131, wherein the antisense binding portion and the oligonucleotide tag of the antisense probe comprise RNA.
135. The composition of aspect 131, wherein the antisense binding portion of the antisense probe comprises RNA and the oligonucleotide tag of the antisense probe comprises DNA.
136. The composition of any of aspects 95 to 135, wherein the sense probe, the antisense probe or both comprise one or more modified nucleic acids.
137. The composition of aspect 136, wherein one or more modified nucleotides comprise a locked nucleic acid (LNA).
138. The composition of aspect 136, wherein one or more modified nucleotides are selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof.
139. The composition of any of aspects 95 to 138, wherein the label comprises a member of a binding pair.
140. The composition of aspect 139, wherein the label comprises biotin.
141. The composition of any of aspects 95 to 138, wherein the label comprises an electrochemiluminescent label.
142. A kit for carrying out the method of any of aspects 1 to 94.
143. The method of any of aspects 1 to 94, wherein the sense and antisense strand of the oligonucleotide duplex comprises a nucleic acid sequence of a microorganism.
144. The method of aspect 143, wherein the microorganism is a component of the human microbiome.
145. The method of aspect 143 or 144, wherein the microorganism is a bacteria, fungi, protozoa or a virus.
146. The method of aspect 145, wherein the microorganism is a bacteria.
147. The method of aspect 146, wherein the sense strand and the antisense strand of the oligonucleotide duplex comprise 16S rRNA or rDNA from bacteria.
148. The method of aspect 147, wherein the bacteria is Achromobacter, Acidaminococcus, Acinetobacter, Actinomycetales, Aerococcus, Anaerococcus, Aggregatibacter, Aeromonas, Alcaligenes, Anaerobiospirillum, Atopobium, Bacillus, Bacillota, Bacteroides, Bacterionema, Bartonella, Bifidobacterium, Bordetella, Borrelia, Brucella, Burkholderia, Buchnera, Butyriviberio, Campylobacter, Capnocytophaga, Cardiobacterium, Chlamydia, Chlamydophila, Collinsella, Citrobacter, Clostridium, Corynebacterium, Cutibacterium, Dialister, Demodex, Eggerthella, Eikenella, Enterococcus, Enterobacter, Escherichia, Eubacterium, Faecalibacterium, Finegoldia, Firmicutes, Flavobacterium, Francisella, Fusobacterium, Gardnerella, Gordonia, Haemophilus, Helicobacter, Kingella, Klebsiella, Lactobacillus, Legionella, Leptospira, Leptotrichia, Listeria, Megasphaera, Methanobrevibacter, Microbacterium, Micrococcus, Mobiluncus, Morganella, Moraxella, Mycobacterium, Mycoplasma, Neisseria, Peptococcus, Peptoniphilus, Peptostreptococcus, Plesiomonas, Porphyromonas, Prevotella, Propionibacterium, Proteus, Providencia, Pseudomonas, Pseudomonadota, Rickettsia, Roseburia, Rothia, Ruminococcus, Sarcina, Salmonella, Selenomonas, Shigella, Slackia, Sneathia, Spirochaeta, Staphylococcus, Streptobacillus, Streptococcus, Streptomyces, Tannerella, Treponema, Trichophyton, Ureaplasma, Veillonella, Vibrio, Wolinella or Yersinia bacteria.
149. The method of any of aspects 143 to 148, wherein the sample comprises a biological sample.
150. The method of aspect 149, wherein the biological sample comprises a sample isolated from a portion of a human body.
151. The method of aspect 150, wherein the portion of the body is a nasal passage, oral cavity, skin, ear, mucus membrane, gastrointestinal tract, urogenital tract, respiratory tract, eye or a combination thereof.

## Claims

1. A method of detecting or quantifying a sense and an antisense strand of an oligonucleotide duplex in a sample, the method comprising:
(a) contacting the sample with a composition comprising a set of probes, wherein the set of probes comprises:
(i) a sense probe comprising a first single stranded oligonucleotide tag that is complementary to at least a portion of a first capture oligonucleotide immobilized on a support surface, a sense binding portion capable of hybridizing to a nucleotide sequence of the sense strand of the oligonucleotide duplex, and a first label; and
(ii) an antisense probe comprising a second single stranded oligonucleotide tag that is complementary to at least a portion of a second capture oligonucleotide immobilized on the support surface, an antisense binding portion capable of hybridizing to a nucleotide sequence of the antisense strand of the oligonucleotide duplex, and a second label,
wherein the sense binding portion of the sense probe has a sense binding length that is shorter than a sense strand length of the sense strand, and
wherein the antisense binding portion of the antisense probe has an antisense binding length that is shorter than an antisense strand length of the antisense strand; and
(b) incubating the probes with the sample to form a hybridization mixture comprising hybridization complexes comprising:
(i) a sense complex comprising the sense probe hybridized with the sense strand of the oligonucleotide duplex; and
(ii) an antisense complex comprising the antisense probe hybridized with the antisense strand of the oligonucleotide duplex; and
(c) contacting the support surface with the hybridization mixture in which the first and second oligonucleotide tags of the sense and antisense probes hybridize to the first and second capture oligonucleotides immobilized on the support surface and contacting the hybridization complexes in the hybridization mixture with a single-strand specific nuclease; and
(d) detecting or quantifying the sense and antisense strands of the oligonucleotide duplex based on the presence of the label on the support surface,
wherein the sense and antisense strands of the oligonucleotide duplex each comprise, individually, from about 8 to about 50 nucleotides or from about 16 to about 30 nucleotides.

2. The method of claim 1, wherein (c) comprises:
(i) contacting the support surface with the hybridization mixture under conditions in which the first and second oligonucleotide tags of the hybridization complexes hybridize to the first and second capture oligonucleotides on the support surface to immobilize the hybridization complexes on the support surface; and contacting the immobilized hybridization complexes with a single-strand specific nuclease; or
(ii) contacting the hybridization mixture with a single-strand specific nuclease to form a reaction mixture; and contacting the support surface with the reaction mixture under conditions in which the first and second oligonucleotide tags of the hybridization complexes hybridize to the first and second capture oligonucleotides immobilized on the support surface.

3. The method of any of claims 1 to 2, wherein:
(a) the sense strand of the oligonucleotide duplex comprises DNA or RNA; the antisense strand of the oligonucleotide duplex comprises DNA or RNA; the oligonucleotide duplex comprises a DNA/DNA or an RNA/RNA duplex; or the oligonucleotide duplex comprises a DNA/RNA heteroduplex; and/or
(b) the sense strand, the antisense strand or both the sense and antisense strands of the oligonucleotide duplex individually comprise a 5'- or 3'- conjugate, optionally wherein the conjugate comprises polyethylene glycol (PEG), N-acetylgalactosamine (GalNAc), a cell penetrating peptide (CPP), *α*-tocopherol, an aptamer, an antibody, cholesterol, squalene, a fatty acid, or a nucleolipid.

4. The method of any of claims 1 to 3, wherein the sense probe, the antisense probe or both comprise one or more modified nucleic acids.

5. The method of claim 4, wherein:
(a) one or more modified nucleotides comprise a locked nucleic acid (LNA); or
(b) one or more modified nucleotides are selected from phosphodiester (PO); phosphorothioate (PS); 2'O-methyl (2'OMe); 2'O-methoxyethyl (MOE); peptide nucleic acid (PNA); phosphoroamidate morpholino (PMO); locked nucleic acid (LNA); 2'-deoxy-2'-fluoro (2'-F); or a combination thereof.

6. The method according to any of claims 1 to 5, wherein the sample comprises a plurality of oligonucleotide duplexes and the composition in (a) comprises a plurality of sets of probes, wherein each set of probes hybridizes with a unique sense or antisense strand of a unique oligonucleotide duplex.

7. The method of any of claims 1 to 6, wherein the sample comprises a biological sample, an environmental sample, or a manufacturing process sample.

8. The method of any of claims 1 to 7, wherein the label comprises an electrochemiluminescent label.

9. The method of claim 8, comprising a step of generating an assay signal by contacting the electrodes with an electrochemiluminescence read buffer comprising an electrochemiluminescence co-reactant, and applying an electrical potential to the electrodes, optionally wherein the electrochemiluminescence co-reactant is selected from a tertiary amine, tripropylamine, N-butyldiethanolamine, and combinations thereof.

10. A composition comprising a set of probes, wherein the set of probes comprises:
(a) a sense probe comprising a first single stranded oligonucleotide tag that is complementary to at least a portion of a first capture oligonucleotide, a sense binding portion capable of hybridizing to a nucleotide sequence of a sense strand of an oligonucleotide duplex, and a first label; and
(b) an antisense probe comprising a second single stranded oligonucleotide tag that is complementary to at least a portion of a second capture oligonucleotide, an antisense binding portion capable of hybridizing to a nucleotide sequence of an antisense strand of the oligonucleotide duplex, and a second label,
wherein the sense binding portion of the sense probe has a sense binding length that is shorter than a sense strand length of the sense strand, and
wherein the antisense binding portion of the antisense probe has an antisense binding length that is shorter than an antisense strand length of the antisense strand,
wherein the sense and antisense strands of the oligonucleotide duplex each comprise, individually, from about 8 to about 50 nucleotides, or from about 16 to about 30 nucleotides.

11. A composition comprising:
(a) an oligonucleotide duplex comprising a sense strand and an antisense strand; and
(b) a set of probes comprising:
(i) a sense probe comprising a first single stranded oligonucleotide tag that is complementary to at least a portion of a first capture oligonucleotide, a sense binding portion capable of hybridizing to a nucleotide sequence of a sense strand of an oligonucleotide duplex, and a first label; and
(ii) an antisense probe comprising a second single stranded oligonucleotide tag that is complementary to at least a portion of a second capture oligonucleotide, an antisense binding portion capable of hybridizing to a nucleotide sequence of an antisense strand of the oligonucleotide duplex, and a second label,
wherein the sense binding portion of the sense probe has a sense binding length that is shorter than a sense strand length of the sense strand, and
wherein the antisense binding portion of the antisense probe has an antisense binding length that is shorter than an antisense strand length of the antisense strand, wherein the sense and antisense strands of the oligonucleotide duplex each comprise, individually, from about 8 to about 50 nucleotides, or from about 16 to about 30 nucleotides.

12. A composition comprising one or more hybridization complexes, the hybridization complexes comprising:
(a) a sense complex comprising a sense probe hybridized to a sense strand of an oligonucleotide duplex, wherein the sense probe comprises a first single stranded oligonucleotide tag that is complementary to at least a portion of a first capture oligonucleotide, a sense binding portion capable of hybridizing to a nucleotide sequence of the sense strand of the oligonucleotide duplex, and a first label, wherein the sense binding portion of the sense probe has a sense binding length that is shorter than a sense strand length of the sense strand;
(b) an antisense complex comprising an antisense probe hybridized to an antisense strand of the oligonucleotide duplex, wherein the antisense probe comprises a second single stranded oligonucleotide tag that is complementary to at least a portion of a second capture oligonucleotide, an antisense binding portion capable of hybridizing to a nucleotide sequence of the antisense strand of the oligonucleotide duplex, and a second label, wherein the antisense binding portion of the antisense strand has an antisense binding length that is shorter than an antisense strand length of the antisense probe,
and combinations thereof,
wherein the sense and antisense strands of the oligonucleotide duplex each comprise, individually, from about 8 to about 50 nucleotides, or from about 16 to about 30 nucleotides.

13. The composition of any of claims 10-12, wherein the sense strand of the oligonucleotide duplex comprises DNA or RNA; the antisense strand of the oligonucleotide duplex comprises DNA or RNA; the oligonucleotide duplex comprises a DNA/DNA or an RNA/RNA duplex; or the oligonucleotide duplex comprises a DNA/RNA heteroduplex.

14. The composition of any of claims 10-13, wherein:
(a) the sense strand, antisense strand or both the sense and antisense strands of the oligonucleotide duplex individually comprise one or more modified nucleic acids; and/or
(b) the sense strand, the antisense strand or both the sense and antisense strands of the oligonucleotide duplex individually comprise a 5'- or 3'- conjugate, optionally wherein the conjugate comprises polyethylene glycol (PEG), N-acetylgalactosamine (GalNAc), a cell penetrating peptide (CPP), *α*-tocopherol, an aptamer, an antibody, cholesterol, squalene, a fatty acid, or a nucleolipid.

15. A kit for carrying out the method of any of claims 1 to 9, comprising:
(i) one or more capture oligonucleotides;
(ii) a sense probe comprising a first single stranded oligonucleotide tag that is complementary to at least a portion of a first capture oligonucleotide, a sense binding portion capable of hybridizing to a nucleotide sequence of a sense strand of an oligonucleotide duplex, and a first label;
(iii) an antisense probe comprising a second single stranded oligonucleotide tag that is complementary to at least a portion of a second capture oligonucleotide, an antisense binding portion capable of hybridizing to a nucleotide sequence of an antisense strand of the oligonucleotide duplex, and a second label;
wherein the sense binding portion of the sense probe has a sense binding length that is shorter than a sense strand length of the sense strand, and
wherein the antisense binding portion of the antisense probe has an antisense binding length that is shorter than an antisense strand length of the antisense strand; and
(iv) a single-stranded nuclease,
wherein the sense and antisense strands of the oligonucleotide duplex each comprise, individually, from about 8 to about 50 nucleotides or from about 16 to about 30 nucleotides.
